(19)

Europäisches
Patentamt
European
Patent Office
Office européen
des brevets

(11)  EP 1 852 703 A1

(12)  **EUROPEAN PATENT APPLICATION**
published in accordance with Art. 158(3) EPC

(43) Date of publication:
**07.11.2007 Bulletin 2007/45**

(21) Application number: **06702656.7**

(22) Date of filing: **10.01.2006**

(51) Int Cl.:
*G01N 35/08* (2006.01)    *G01N 27/49* (2006.01)
*G01N 33/24* (2006.01)    *G01N 37/00* (2006.01)

(86) International application number:
**PCT/JP2006/300136**

(87) International publication number:
**WO 2006/080186 (03.08.2006 Gazette 2006/31)**

(84) Designated Contracting States:
**DE FR GB NL**

(30) Priority:  **07.01.2005   JP  2005002540**

(71) Applicant: **SEKISUI CHEMICAL CO., LTD.**
**Osaka-shi,**
**Osaka 530-8565 (JP)**

(72) Inventors:
• **IWASA, Koichiro**
**o, Kamitoba, Minami-ku, Kyoto-shi, Kyoto (JP)**
• **TAMAKI, Satoshi**
**o, Kamitoba, Minami-ku, Kyoto-shi, Kyoto (JP)**

(74) Representative: **HOFFMANN EITLE**
**Patent- und Rechtsanwälte**
**Arabellastrasse 4**
**81925 München (DE)**

(54)  **DETECTION DEVICE USING CARTRIDGE**

(57)     Discloses is a cartridge-type detection apparatus which comprises a detection cartridge having a passage for passing a sample liquid containing an target substance, and a processing unit adapted to be loaded with the detection cartridge so as to produce information about the target substance contained in the sample liquid passed through the detection cartridge. The detection cartridge includes an storing section for temporarily storing the target substance, a liquid passage routed through the storing section, and a plurality of ports in liquid communicate with the liquid passage. The detection cartridge is provided with a part or entirety of a detection mechanism on a downstream side relative to the storing section. The processing unit includes a liquid feed pump and a line switching valve mechanism adapted to switchingly provide liquid communication between the liquid feed pump and a selected one of the plurality of ports of the detection cartridge. The valve mechanism is operable to switch between a first passage connection mode for allowing the sample liquid supplied into the detection cartridge to be passed through the storing section and then discharged out of the detection cartridge, and a second passage connection mode for allowing a reagent to be supplied from one of the plurality of ports to the storing section of the detection cartridge by an action of the liquid feed pump, and allowing the reagent passed across the storing section to be discharged out of the detection cartridge from one of the remaining ports.

FIG.4

**Description**

TECHNICAL FIELD

The present invention relates to a detection apparatus for use in detecting a detection-target substance target substance contained in a liquid sample. In particular, the present invention relates to a detection apparatus comprising a cartridge and a processing unit adapted to be combined with the cartridge. More specifically, the present invention relates to a cartridge-type detection apparatus for generating information on existence, concentration, and/or composition, or the like in a liquid sample containing a detection-target substance, which comprises a detection cartridge formed with a passage or passages for passing a liquid sample, and a processing unit adapted to be loaded with the cartridge so as to produce information about the detection-target substance contained in the liquid sample being passed through the cartridge.

BACKGROUND ART

Japanese Patent Laid-Open Publication No. 10-311829 (JP 10-311829A) discloses a disposable card-type portable analysis system. This analysis system comprises a testing tool which includes a sensor for detecting at least one test parameter value about a human or animal body fluid and generating an output signal indicative of the detected value, and a portable analysis unit which includes a processing section for receiving the signal from the testing tool and processing the received signal, and a display section.

The card-type disposable testing tool comprises two base plates designed to be liquid-tightly superimposed on each other while interposing a thin partition plate therebetween. A first one of the base plates has an inner surface formed with a passage for passing a human or animal body fluid serving as a test sample, and a first body-fluid reservoir portion in liquid communication with one end of the passage. The first base plate is formed with a body-fluid inlet port penetrating therethrough in a thickness direction thereof. The sensor is mounted on an inner surface of a second one of the base plates. The inner surface of the second base plate is formed with a concave portion for receiving therein a reagent container which contains a reagent for calibrating the sensor, and a second body-fluid reservoir portion in liquid communication with the first body-fluid reservoir portion of the first base plate via an opening of the partition plate.

The portable analysis unit has an insertion slot for inserting the testing tool therethrough. When the testing tool is inserted into the portable analysis unit, the reagent container placed in the reagent-container-receiving concave portion is broken, and the reagent is led to the sensor to calibrate the sensor in advance of an actual analysis. Then, a human or animal body fluid is injected from the inlet port. The body fluid is supplied to the sensor through the passage formed in the inner surface of the first base plate, and subjected to a measurement. An electrical signal generated during the measurement is sent to the analysis unit, and processed by the processing section. An obtained analysis result will be indicated on the display section.

This analysis system has advantages of being able to perform an on-site test on its portability, and to allow a body-fluid sample to be directly injected therein using an injector, such as a syringe, so as to prevent the body-fluid sample from contacting ambient atmosphere. However, the analysis system is intended to analyze a high concentration of liquid, such as a human or animal body fluid, and is therefore unusable for a concentration measurement or chromatography analysis of an extremely small amount of detection-target substance, such as a hazardous heavy metal contained in soil.

U.S. Patent No. 6,110,354 discloses an analyzer equipped with a microband electrode arrangement and adapted for use in an analysis of drinking water, waste water, a biological fluid such as blood or urine, etc. An analytical principle of this analyzer is to detect a faradaic component generated when an electrolyte liquid as a test sample comes into contact with the electrodes. In one illustrated embodiment, the analyzer has a plate-shaped sensor comprising a flat substrate. It would be understood that the microband electrode arrangement makes it possible to suppress the generation of a non-faradaic component so as to provide enhanced sensitivity allowing a detection of a hazardous metal contained in an aqueous solution in a small amount. However, even with such detection sensitivity, this analyzer is hardly used for the concentration measurement of an extremely small amount of target substance, such as a hazardous heavy metal contained in soil.

DISCLOSURE OF THE INVENTION

It is a major object of the present to provide a detection apparatus comprising a detection cartridge a processing unit adapted to be used in combination with the cartridge, usable in a simplified manner with less restriction on the type of targets to be detected and the site where it is used.

It is another object of the present invention to provide a detection apparatus capable of being used in concentration detecting devices, liquid chromatography analysis, and immunoassay processes, and other various detection methodologies for a detection-target substance contained in a liquid sample.

In a specific aspect, it is yet another object of the present invention to provide a cartridge-type simplified detection apparatus which is conveniently used as a portable device.

It is still another object of the present invention to provide a cartridge-type conveniently carried detection apparatus having a structure which allows a passage line for liquid communication essential to detections, to be arranged in a significantly compact manner.

It is yet still another object of the present invention to provide a concentration detection apparatus capable of detecting a concentration of a detection-target substance contained in a sample even if the concentration is extremely low.

It is another further object of the present invention to provide a cartridge-type portable simplified detection apparatus capable of performing an analysis, such as chromatography analysis, of a liquid sample, in a simplified manner even at a site where a sampling is carried out.

It is still a further object of the present invention to provide a readily-portable analysis apparatus capable of readily performing concentration detection, liquid chromatography analysis, immunoassay analysis, or other detection, at any location without restriction on test locations

It is an additional object of the present invention to provide a detection cartridge and/or a processing unit for use in the above cartridge-type detection apparatus and/or analysis apparatus.

In order to achieve the above objects, according to the broadest aspect of the present invention, there is provided a cartridge-type detection apparatus which comprises a detection cartridge having a passage for passing a sample liquid containing an detection-target substance, and a processing unit adapted to be loaded with the detection cartridge so as to produce information about the target substance contained in the sample liquid passed through the detection cartridge. The detection cartridge includes an storing section for temporarily storing the target substance, at least a part of a detection mechanism, a liquid passage adapted to be selectively routed through either one or both of the storing section and the at least a part of the detection mechanism, and a plurality of ports in liquid communicate with the liquid passage. As used in connection with the present invention, the term "detection" is intended to mean "generating information about whether an target substance is present or absent, or a property of an target substance, such as a concentration or composition of the target substance", and includes assay or analysis, such as quantitative analysis and/or qualitative analysis.

The processing unit includes a reagent tank and a liquid feed pump. In this aspect of the present invention, the detection cartridge is associated with the processing unit in a manner that the liquid passage is switched between a first route for allowing the sample liquid supplied into the detection cartridge to be passed through the storing section and then discharged out of the detection cartridge, and a second route for allowing a reagent to be supplied from one of the plurality of ports to the storing section of the detection cartridge by means of the liquid feed pump, and successively passed through the storing section and the at least a part of the detection mechanism.

In one embodiment of the present invention, the sample liquid can be supplied to the detection cartridge independently from the processing unit. When the sample liquid supplied into the detection cartridge reaches the storing section, the target substance contained in the sample liquid is temporarily stored in the storing section. The storing section may be made of a material having a relatively large surface area, such as a porous material including a porous ceramic material, a fibrous material or a fine particle material, and may have a surface with a structure modified by a functional group which exhibits a chemical reaction with or an adsorption action on the target substance. In this case, the target substance will be absorbed and held to/by the material of the storing section. The remaining sample liquid passed across the storing section is discharged out of the detection cartridge from one of the ports of the detection cartridge. Alternatively, a waste liquid reservoir may be formed inside the detection cartridge, and the sample liquid from the storing section may be led to the waste liquid reservoir.

Then, a reagent is supplied from the liquid feed pump of the processing unit into the detection cartridge, and passed through the storing section. This reagent has a function of eluting the target substance which is held by the storing section through a chemical reaction or an absorption action. By having the reagent passed through the storing section, the target substance held by the storing section is eluted from the storing section, and passed through the detection cartridge in a downstream direction together with the reagent in a manner dissolved therein. The reagent containing the target substance is temporarily sent to a passage outside of the detection cartridge and then returned to the detection cartridge, or sent toward the detection mechanism disposed on the downstream side through the internal passing the detection cartridge without being sent out of the detection cartridge.

The reagent tank disposed in the processing unit may be connected to the liquid feed pump. In this case, the processing unit may be provided with a plurality of the reagent tanks, and a tank switching valve mechanism may be provided for alternately provide liquid communication between a desired one of the plurality of reagent tanks and the liquid feed pump. The processing unit may also be provided with a waste liquid tank, and the sample liquid discharged from the detection cartridge may be led to the waste liquid tank.

In another embodiment of the present invention, the cartridge-type detection apparatus is designed, when the detection cartridge is unloaded from the processing unit, to define the first route of liquid passage for allowing the sample liquid supplied into the detection cartridge to be passed through the storing section and then discharged out of the detection cartridge, and, when the detection cartridge is loaded into the processing unit, to define the second route of liquid passage for allowing a reagent to be supplied from one of the plurality of ports to the storing section of the detection cartridge by the action of the liquid feed pump, and successively passed through the storing section and at least a part of the detection mechanism. In this case, the cartridge-type detection apparatus may have a valve mechanism for allowing the liquid passage to be switched between these routes, and this valve mechanism may be disposed in the processing unit.

The detection apparatus of the present invention can be applied to detections of various different substances. In one aspect of the present invention, the detection apparatus is designed as a concentration detection apparatus for providing information about a concentration of an target substance contained in a sample liquid. In this case, the detection cartridge is designed to generate an electric signal indicative of a concentration of the target substance. As one example, the sample liquid is prepared by dissolving a sample, such as soil or mud containing a small amount of target substance, in liquid, such as water.

In one embodiment where the present invention is applied to a concentration detection apparatus, the detection cartridge includes a sample-liquid inlet portion for feeding therethrough a sample liquid having a sample dissolved therein, and a liquid passage extending from the sample-liquid inlet portion. The storing section is disposed in the liquid passage. In this embodiment, the storing section is formed as an enrichment section for enriching an target substance contained in the sample liquid. This enrichment section may be provided in the form of a filter having an ability to absorb the target substance. The detection mechanism provided in the detection cartridge is formed as a detection electrode arrangement. The target substance absorbed to the filter is eluted into an eluent liquid supplied as a reagent, and sent to the detection electrode arrangement serving as the detection mechanism. When the eluent liquid containing the eluted target substance reaches the detection electrode arrangement, a detection electric signal is generated by the electrode.

In the detection apparatus according to this embodiment of the present invention, the processing unit includes a read section for reading the electric signal from the detection cartridge, and produce information about a concentration of the target substance. The detection cartridge in this embodiment may include a waste liquid reservoir. In this case, the liquid passage is formed to extend from the sample-liquid inlet portion to the waste liquid reservoir.

The read section includes processing means which is operable, in response to receiving the electric signal from the detection cartridge, to process the received electric signal so as to produce information about a concentration of the target substance in the sample. The processing unit may be optionally provided with a display section for indicating a detection result.

The cartridge-type concentration detection apparatus according to this embodiment of the present invention may be used for detecting a heavy metal contained in soil or mud. In this case, the electrode arrangement in the detection cartridge is configured to generate an electric signal indicative of a concentration of a heavy metal contained in a sample liquid, and the read section is designed to read the electric signal from the detection cartridge so as to produce information about a concentration of the heavy metal.

In the cartridge-type concentration detection apparatus for detecting a concentration of a heavy metal, the detection cartridge includes a sample-liquid inlet portion for feeding the sample liquid therethrough, a liquid passage in liquid communication with the sample-liquid inlet portion, a storing section serving as an enrichment section disposed in the liquid passage extending from the sample-liquid inlet portion and adapted to enrich the sample liquid, and a detection electrode arrangement. The storing section or the enrichment section includes an absorptive element disposed in the liquid passage and adapted to absorb the heavy metal. The storing section includes an eluent-liquid supply section adapted to be associated with the enrichment element and supply an eluent liquid for eluting the heavy metal absorbed to the absorptive element, through the absorptive element and toward the detection electrode arrangement. Thus, the heavy metal absorbed by the absorptive element is eluted by a predetermined volume of the eluent liquid from the eluent-liquid supply section, and the eluted heavy metal is brought into contact with the detection electrode arrangement to allow the detection electrode arrangement to generate an electric signal indicative of a concentration of the heavy metal.

In a cartridge-type concentration detection apparatus according to another embodiment of the present invention, the storing section or enrichment section includes an absorptive element adapted to receive the sample liquid from the sample-liquid inlet portion and absorb the target substance contained in the sample liquid, and the enrichment section is associated with an eluent-liquid supply section whereby an eluent liquid which functions to elute the target substance absorbed by the absorptive element is directed through the absorptive element and toward the detection electrode arrangement, so that the target substance absorbed by the absorptive element is eluted by a predetermined volume of the eluent liquid from the eluent-liquid supply section, and the eluted target substance is brought into contact with the detection electrode arrangement to allow the detection electrode arrangement to generate an electric signal indicative of a concentration of the target substance

In a cartridge-type concentration detection apparatus according to yet another embodiment of the present invention, the detection cartridge includes a sample-liquid inlet portion for feeding therethrough a sample liquid having the sample dissolved therein, an enrichment section for enriching the sample liquid fed in the sample-liquid inlet portion, a detection electrode arrangement, and a passage for providing liquid communication between respective ones of the sample-liquid inlet portion, the enrichment section and the detection electrode arrangement. The enrichment section includes an absorptive element adapted to receive the sample liquid from the sample-liquid inlet portion and absorb the target substance contained in the sample liquid. The concentration detection apparatus further includes an eluent-liquid supply section adapted to be associated with the enrichment section and supply an eluent liquid for eluting the target substance absorbed to the absorptive element, through the absorptive element and toward the detection electrode arrangement, and a passage switching valve mechanism. This valve mechanism is adapted to be selectively shifted between a sample-liquid feed position for opening a sample-liquid feed passage extending from the sample-liquid inlet portion to the enrichment section and closing an eluent-liquid supply passage extending from the eluent-liquid supply section to the enrichment section, and an eluent-liquid supply position for closing the sample-liquid feed passage extending from the sample-liquid inlet portion to the enrichment section and opening the eluent-liquid supply passage extending from the eluent-liquid supply section to the enrichment section. Further, pumping means is provided for supplying an eluent liquid from the eluent-liquid supply section to the enrichment section when the passage switching valve mechanism is at the eluent-liquid supply position.

In this embodiment, the target substance absorbed to the absorptive element is eluted by a predetermined volume of the eluent liquid from the eluent-liquid supply section, and the eluted target substance is brought into contact with the detection electrode arrangement to allow the detection electrode arrangement to generate an electric signal indicative of a concentration of the target substance. Each of the eluent-liquid supply section, the valve mechanism and the pumping means may be housed in a casing of the processing unit.

The detection cartridge may be formed with a discharge passage for discharging the sample liquid after being passed across the absorptive element, outside the detection cartridge, and a waste liquid reservoir for storing the eluent liquid after being passed across the electrode arrangement. In this case, the discharge passage and a passage between the electrode arrangement and the waste liquid reservoir may be opened and closed, respectively, in a process of feeding the sample liquid, and closed and opened, respectively, in a process of supplying the eluent liquid. The absorptive element may be formed as any one of a membrane (or film), a fine particle and a porous body.

The absorptive element may be a cationic substance-absorptive element. In this case, the absorptive element may be formed of a material having a surface modified with a sulfonic acid group. Alternatively, the absorptive element may be an anionic substance-absorptive element. In this case, the absorptive element may be formed of a material having a surface modified with a quaternary amine group. Alternatively, the absorptive element may be formed of a material having a surface treated by a heavy-metal receptor. This heavy-metal receptor may be any one of a chelating substance, a clathrate, and a heavy-metal absorptive substance. The chelating substance may be either one of iminodiacetic acid and ethylene diamine group. The clathrate may be either one of porphyrin and calixarene. The heavy-metal absorptive substance may be either one of apoenzyme and heavy-metal absorptive antibody.

The detection cartridge may be formed in a card shape. In this case, the processing unit preferably has a casing formed with an insertion portion for allowing insertion of the card-shaped cartridge.

Preferably, the electrode arrangement includes at least one microelectrode element of a size not larger than 10 $\mu$m. In this case, the microelectrode element is preferably prepared by attaching an insulation sheet on an upper surface of an electrode member and forming in the insulation sheet a hole of a size not larger than 10 $\mu$m.

The electrode arrangement may include a plurality of working electrode elements, at least one counter electrode element, and at least one reference electrode element. Each of the plurality of working electrode elements may be formed to have a different area so as to work for a measurement of a different concentration range.

Alternatively, the electrode arrangement may include at least one working electrode element, at least one counter electrode element, and at least one reference electrode element.

The enrichment section serving as the storing section may have a structure where a cation-absorptive element adapted to absorb a cationic substance and an anion-absorptive element adapted to absorb an anionic substance are arranged in parallel relation to each other. Further, the electrode arrangement serving as the detection mechanism may include two electrode pairs each associated with a corresponding one of the cation-absorptive element and the anion-absorptive element.

In a cartridge-type concentration detection apparatus according to still another embodiment of the present invention, the detection cartridge includes therewithin a sample-liquid inlet portion for feeding therethrough a sample liquid as a concentration-detection target, a liquid passage in liquid communication with the sample-liquid inlet portion, and a concentration-detection electrode arrangement disposed in the liquid passage and adapted to generate an electric signal indicative of a concentration of a specific substance contained in the sample liquid which is fed in the liquid passage and passed through the concentration-detection electrode arrangement. The electrode arrangement includes a plurality of working electrode elements, at least one counter electrode element, and at least one reference electrode element. Each of the plurality of working electrode elements is formed to have a different area so as to work for a measurement of a different concentration range.

In a cartridge-type concentration detection apparatus according to yet still another embodiment of the present invention, the detection cartridge has a flat card shape, and the processing unit has a casing formed with an insertion portion for allowing insertion of the card-shaped cartridge. This detection cartridge includes therewithin a sample-liquid inlet portion for feeding therethrough a sample liquid as a concentration-detection target, a liquid passage in liquid communication with the sample-liquid inlet portion, and a concentration-detection electrode arrangement disposed in the liquid passage and adapted to generate an electric signal indicative of a concentration of a specific substance contained in the sample liquid which is fed in the liquid passage and passed through the concentration-detection electrode arrangement.

This detection cartridge includes a first sheet made of a resin material and formed to have one surface with a concave portion defining at least a part of the liquid passage, a second sheet made of a resin material and formed with a through-hole constituting the sample-liquid inlet portion and a concave portion receiving therein the concentration-detection electrode arrangement, and a third sheet made of a resin material and formed with a through-hole constituting the sample-liquid inlet portion. The first, second and third sheets are laminated in turn while interposing an insulation sheet between adjacent ones thereof. The concentration-detection electrode arrangement includes an electrode element disposed in the electrode-receiving concave portion of the second sheet, and the second sheet and the third sheet are laminated to allow the electrode element to face the third sheet. Further, the insulation sheet interposed between the second sheet and the third sheet has a hole formed at a position corresponding to the electrode element to expose a predetermined area of the electrode element, so that a liquid passage for leading the sample liquid to the electrode element is defined on a side of a surface of the third sheet facing the second sheet.

In this case, the first sheet of the cartridge may have a surface located to face the second sheet and formed with a concave portion constituting a waste liquid reservoir for receiving therein a waste liquid from the electrode element.

In the above embodiments of the present invention, the enrichment section for storing the target substance in an enriched manner is provided in the liquid passage. This makes it possible to perform the concentration detection without any problem even if the target substance is contained in the sample liquid in an extremely low concentration. If soil is contaminated by a hazardous substance, such as a heavy metal, it shall be controlled by environmental regulations even if a concentration of the substance is extremely low. While it has been considered that an on-site detection of a substance contained in such an extremely low concentration is impossible, the cartridge-type concentration detection apparatus according to each of the above embodiments of the present invention makes it possible to perform detection of a pollutant in a simplified manner at a location where a soil sample is collected. In this case, a sample liquid may be prepared by dissolving a soil sample. The enrichment section for enriching an target substance is preferably designed to include an absorptive element adapted to absorb the target substance. Alternatively, any other suitable enrichment means may be used. For example, an enrichment technique of heating a sample liquid to evaporate a liquid component, or a technique-type on a reverse osmosis membrane, may be used.

The cartridge-type concentration detection apparatus according to each of the above embodiments of the present invention can be applied to concentration detection of any substance, i.e., target substance, which allows the detection electrode arrangement to generate electric information about a concentration thereof when a liquid containing the target substance comes into contact with the detection electrode arrangement. Typically, the detection electrode arrangement comprises a working electrode, a counter electrode and a reference electrode. The working electrode is operable to absorb an target substance and then release the absorbed target substance into an eluent liquid when it comes into contact with the eluent liquid. A requirement for the working electrode preferably includes a capability to allow a potential to be applied thereto in a relatively wide range, i.e., a relatively wide potential window, and high resistances to corrosion and oxidation. The potential window means a potential range causing no generation/formation of electrochemically undesirable hydrogen ion and oxide film, and this range is varied depending on a material of the electrode and a pH value of a sample liquid.

A material of the working electrode preferably includes platinum, gold, mercury, silver, bismuth and carbon. While the working electrode may be made of one appropriately selected from these materials, it is preferable to select a material having high ability to adsorb an target substance, i.e., a measurement target. When the target substance is cadmium, lead and mercury, an electrode having a carbon surface may be reasonably used as a working electrode. As a working electrode for a measurement of arsenic and mercury, it is reasonable to use an electrode having a gold surface. For detecting hexavalent chrome, an electrode having a carbon surface may be used. The reason is that the electrode with a carbon surface has a property of excellently absorbing an aggregate of hexavalent chrome and diphenylcarbazide.

As the electrode with a carbon surface, a carbon electrode comprising a sintered body of a graphite/carbon mixture having a graphite/glassy carbon ratio of 70/30 is preferable. Generally, in contrast with an advantage of relatively high ability to absorb lead, cadmium and mercury, graphite has a problem about variation in substance-adsorptive capacity due to difficulty in uniforming its crystalline orientation, and swelling due to contact with liquid. As measures against this problem, graphite may be sintered after mixing glassy carbon therein to obtain a densified sintered body capable of suppressing the penetration of liquid. Further, the grassy carbon can randomly orient graphite crystals to minimize the variation in adsorptive capacity. The above sintered body with a graphite/glassy carbon ratio of 70/30 is advantageous in forming a working electrode having high sensitivity and excellent reproducibility.

The electrode with a gold surface is not limited to a specific material. In the present invention, a glass substrate may be coated with gold through a chromium layer to obtain a working electrode having an adequate function. In this case, a chromium film and a gold film may be formed through a sputtering process. A film thickness may be set, but not limited to, at about 40 nm for the chromium layer, and about 400 nm for the gold layer.

The counter electrode is provided as a means to form a current flow in cooperation with the working electrode, and any electrically conductive material may be used for the counter electrode.

The reference electrode is designed to exhibit a known and stable potential usable as a reference potential. A typical reference electrode may include a hydrogen electrode, a saturated calomel electrode (mercury/mercury chloride electrode), and a silver/silver-halide electrode. The silver/silver-halide electrode includes a silver/silver-chloride electrode having a silver surface which forms silver chloride through an equilibrium reaction with a chlorine-containing solution. In this electrode, even when a voltage is being applied thereto, silver and silver chloride are kept in an equilibrium state to allow a resulting potential to be maintained at a constant value so as to serve as a reference electrode. Although a silver/silver-bromide electrode and a silver/silver-iodide electrode may also be used, the silver/silver-chloride electrode is preferable in view of material availability and production costs.

Each of the electrodes is not limited to a specific size. As one example, a sheet, such as a double-faced adhesive tape, formed with a small hole may be attached on a thin plate-shaped electrode having a rectangular planar size of $3 \times 8.4$ mm and a thickness of 0.5 mm, to expose a predetermined area of a surface of the electrode. In view of facilitating a forming process and an attaching operation, a pre-formed thin plate-shaped electrode material is preferably attached onto a base plate of the detection cartridge. Alternatively, the electrode may be directly formed in the base plate of the detection cartridge.

The most typical detection is-type on an electrochemical measurement. In an electrochemical reaction in an aqueous solution, a reaction rate on the electrode is greater than a mass transfer rate in the solution, and therefore a response delay due to the mass transfer rate, so-called "solution resistance", occurs to cause difficulty in clarifying respective peaks. When a micron-size microelectrode is used, the controversial mass transfer will be changed from planar diffusion to point diffusion to reduce a response delay per unit area. Thus, peaks can be discriminated from each other in a smaller scale to provide enhanced sensitivity. In view of expecting this advantage, the electrode is preferably formed to have a minor axis dimension of 10 $\mu$m or less. A large number of such electrodes may be arranged in an array configuration to obtain a large total amount of current. The microelectrode having such a size can be prepared using semiconductor microfabrication techniques. For example, a circular-shaped microelectrode can be obtained by forming an insulation layer on an electrode substrate and then forming in the insulation layer a small hole having a diameter not larger than 10 $\mu$m. A comb-shaped electrode having a configuration where a plurality of working electrodes and counter electrodes each having a diameter not larger than 10 $\mu$m are alternately arranged may also be used to provide the same advantage.

In case of the detection of a heave metal based on the electrochemical measurement, it is desirable to minimize a charge current for increasing a potential of the working electrode up to a predetermined value. In this respect, it is necessary to facilitate electron transfer in a solution containing a heavy metal, and an electrolyte may be added to the solution for this purpose. The electrolyte may be any material capable of forming a salt in the solution. Preferably, the electrolyte includes potassium chloride, sulfuric acid, nitric acid, potassium nitrate and sodium hydroxide, in view of costs. The potential window of the working electrode is characteristically shifted toward a positive or negative side depending on a pH value. Thus, the electrolyte can also be used for adjusting the pH value to prevent the occurrence of a problem about generation of hydrogen from the working electrode and formation of an oxygen film, in a potential range for an intended measurement.

The eluent liquid is typically a solution containing an electrolyte, as described in detail later. In this case, the electrolyte-containing eluent liquid can be sent at a constant flow volume to allow a series of processes from the elution to the detection to be successively performed. This makes it possible to simplify a passage line and an operation and eliminate the need for managing a mixing ratio and a mixing speed between eluent and electrolyte liquids. Specifically, if an eluent liquid and an electrolyte liquid are prepared separately, it is essential to manage respective absolute volumes of the two liquids. Particularly, when a space for arranging the passage and the electrode is extremely small as in the concentration detection apparatus of the present invention, it is difficult to manage of the absolute volumes of the solutions. Thus, it is absolutely critical to allow both the elution process and the electrochemical measurement process to be performed using only a single solution.

As mentioned above, referring to the reference electrode, silver chloride is produced on silver formed by a printing process by having a chlorine-containing solution brought into contact with the printed silver as a reference-electrode activating liquid, and a weak electric current is delivered to the reference electrode. Thus, chlorine may be contained in the eluent liquid to eliminate the need for using the reference-electrode activating liquid separately, and provide a simplified structure. However, in a measurement of selenium, chlorine cannot be contained in the eluent liquid, because chlorine acts as an interfering substance during the electrochemical measurement process. Therefore, it is necessary to use the following reference-electrode activating liquid.

Under the condition that an extremely-weak current is supplied to the reference electrode, a reference-electrode activating liquid is brought into contact with the printed silver to create silver chloride on the printed silver so as to allow the reference electrode to maintain its original function. The reference-electrode activating liquid typically contains an appropriate amount of chlorine. A preferable content of chlorine is in the range of 0.05 to 3 M. An excessively small content of chlorine causes instability in formation of silver chloride, and an excessively large content of chlorine causes poor handling capability due to precipitation of solids. The activating liquid may be prepared by dissolving a predetermined amount of potassium chloride or sodium chloride in water.

The reference electrode is required to have an electrical interaction with the working electrode and the counter electrode, and therefore the reference-electrode activating liquid has to be in contact with the eluent liquid. One purpose of using the reference-electrode activating liquid and the eluent liquid in a separated manner is to prevent chlorine from acting as an interfering substance during the electrochemical measurement process. That is, it is necessary to prevent the reference-electrode activating liquid from flowing into a working electrode section, so that a separate reference electrode chamber is provided to define a liquid passage for providing liquid communication between the reference electrode chamber and the eluent liquid. Preferably, this liquid passage is formed as a micro-passage to prevent the occurrence of undesirable molecular diffusion. Alternatively, the detection cartridge may be designed to separate the reference electrode chamber and the eluent liquid by a porous film. In this case, the reference-electrode activating liquid is obliged to penetrate through the porous film by taking a relatively long time. Thus, in a concentration detection apparatus intended to detect an target substance in a short time of period, as in the present invention, it is more preferable to use the micro-passage. Further, it is desirable to install the reference electrode in the reference electrode chamber, and pre-contain the reference-electrode activating liquid in the reference electrode chamber or supply pre-contain reference-electrode activating liquid into the reference electrode chamber according to need. In case of pre-loading the reference-electrode activating liquid in the detection cartridge, the reference-electrode activating liquid may be advantageously contained in an aluminum pack to prevent precipitation of solids due to vaporization of a liquid component.

Depending on a type of the absorptive element, it is necessary to pass an absorptive element-activating liquid through the absorptive element before passing the sample liquid therethrough. For example, quaternary amine for use in absorbing arsenic, selenium and/or hexavalent chrome, can exhibit its absorption ability only after it comes into contact with an OH ion, because this absorption ability comes from a reaction where an OH ion is substituted with a target anion. In this case, the absorptive element-activating liquid is used. This absorptive element-activating liquid may include sodium hydroxide and potassium hydroxide.

The absorptive element is disposed in the passage on an upstream side relative to the electrode arrangement. As mentioned above, the absorptive element may be formed as any one of a membrane (or film), a fine particle and a porous body, or any combination thereof. The present invention may be applied to chromatography analysis, immunoassay or other detection, and one of these configurations of the absorptive element may be appropriately selected depending on the intended purposes. Each of the configurations will be specifically described below.

[Membrane]

The absorptive element is formed in a filter shape using fibers. Alternatively, a polymer or metal membrane formed with appropriate holes may also be used. For example, the membrane may be designed to have a surface which is formed in a specific configuration or modified with a functional group so as to exhibit an target substance absorption ability, or to have fibers which carry particles having a specific absorption function

[Fine Particle]

The fine particle may be designed to have a surface which is formed in a specific configuration or modified with a functional group so as to exhibit an target substance absorption ability. The passage may be partially filled with such fine particles, for example, over a longitudinal length of about 10 mm or more in a column shape to perform chromatography. The particle-filled portion may have a rectangular parallelepiped shape or a cylindrical shape.

[Porous Body]

This absorptive element consists of a porous body having a large number of continuous pores. For example, the porous body includes a monolithic porous inorganic material such as porous ceramics or porous glass, and a porous polymer material such as porous polyacrylamide gel or porous styrene/divinylbenzene copolymer. The porous body may be designed to have a surface of each continuous pore which is formed in a specific configuration or modified with a functional group so as to exhibit a target substance absorption capability. The substrate or element which has continuous pores and an integral or single-piece structure mayl hereinafter be referred to as "monolithic substrate or element". Further, a monolithic substrate having a length less than that allowing chromatography will hereinafter be referred to as "monolithic disc", and a monolithic substrate having a length allowing chromatography will hereinafter be referred to as "monolithic column". Each of these terms will be used depending on intended purposes. The monolithic column allows a liquid to be passed therethrough at a lower pressure than that in a resin-filled column. Thus, a low-pressure liquid feed pump may be used to facilitate reductions in size and power consumption while maintaining an analytical performance at the same level. For the same reason, the monolithic disc allows a liquid to be passed therethrough at a relatively low pressure, to facilitate a reduction in size of the apparatus. Each of the monolithic column and the monolithic disc having an integral structure can facilitate an operation of installing the absorptive substrate in a cartridge-type microreactor.

The absorptive element may be formed by any of variety of materials including: styrene/divinylbenzene copolymer; polymethacrylate resin; polyhydroxy methacrylate resin; polyvinyl alcohol; polyolefin typified by polyethylene, polypropylene and ethylene/propylene copolymer; olefin/halogenated olefin copolymer typified by ethylene/tetrafluoroethane copolymer and ethylene/chlorotrifluoro- ethylene copolymer; halogenated polyolefin typified by polytetrafluoroethylene, polyvinylidene- fluoride and polychlorotrifluoroethylene; polysulphone; silica; and alumina. The fibrous absorptive element may be made of a fibrous material, such as: various types of natural or regenerated fibers typified by a cellulosic material, a plant fiber including cotton and hemp, and an animal fiber including silk and wool; and various types of synthetic fibers including polyester fiber and polyamide fiber.

As long as a wall surface defining a passage has a function of absorbing an target substance, the absorptive element having any other configuration can also fulfill the same enriching function.

In order to provide the surface of the absorptive element with a property of absorbing a specific target substance, the surface may have a structure complementary to the specific target substance, or by immobilizing onto the surface a functional molecule which exhibits at least one of ion binding, coordinate bonding, chelate bonding, hydrophobic interaction, and interaction due to polarities in molecules.

The functional molecule exhibiting the interaction includes sulfo group, quaternary ammonium group, octadecyl group, octyl group, butyl group, amino group, trimethyl group, cyanopropyl group, aminopropyl group, nitrophenylethyl group, pyrenylethyl group, diethylaminoethyl group, sulfopropyl group, carboxyl group, carboxymethyl group, sulfoxyethyl group, orthophosphate group, diethyl (2-hydroxypropyl) aminoethyl group, phenyl group, iminodiacetate group, and chelate-forming group including ethylenediamine and sulfur atom, for example, functional groups, such as mercapto group, dithiocarbamate group and thiourea group, and atomic groups, such as avidin, biotin, gelatin, heparin, lysine, nicotinamide adenine dinucleotide, protein A, protein G, phenylalanine, castor bean lectin, dextran sulfate, adenosine 5'-phosphate, glutathione, ethylenediamine diacetate, procion red, aminophenylborate, cattle serum albumin, polynucleotide (e.g., DNA), and protein (e.g., antibody). These substances may be used independently or two or more of them may be used in combination.

The eluent liquid has a function of eluting an target substance absorbed to the absorptive element, from the absorptive element. The effectiveness of the eluent liquid depends on absorption mechanisms. Thus, a specific type of eluent liquid is selected in consideration of chemical characteristics of absorption. For example, in case of an eluent liquid containing an ion capable of being easily absorbed to a surface of the absorptive element, when the eluent liquid is passed through the absorptive element, the ion in the eluent liquid is exchanged with an target substance absorbed to the absorptive element in the form of an ion to allow the target substance to be eluted from the absorptive element. In the present invention, an eluent liquid having the following composition may be used depending on target substances.

In the measurement of cadmium, lead and/or mercury, an Empore™ disc cartridge (product name: Cation-SR, available from 3M) is used as the absorptive element, and a liquid (pH = about 4) containing 0.4 M of potassium chloride, 10 mM of citric acid and 3.5 mM of ethylenediamine is used as the elute liquid. This absorptive element is prepared by immobilizing fine particles having a particle size of 50 to 100 $\mu$m to Teflon® fibers, and forming the fibers into a membrane having a thickness of 0.5 to 0.75 mm. The fine particles and the Teflon® fibers are mixed at 10% and 90%, respectively. The absorptive element has a surface modified with a sulfonate group.

In the measurement of arsenic, selenium and/or hexavalent chromium, an Empore™ disc cartridge (product name: Anion-SR, available from 3M) is used as the absorptive element, and 1 M of sulfuric acid (pH = about 2) is used as the elute liquid. This absorptive element is prepared by immobilizing fine particles having a particle size of 50 to 100 $\mu$m to Teflon® fibers, and forming the fibers into a membrane having a thickness of 0.5 to 0.75 mm. The fine particles and the Teflon® fibers are mixed at 10% and 90%, respectively. The absorptive element has a surface modified with quaternary amine.

It should be noted that, depending on the type of the absorptive element, it is necessary to pass an absorptive element-activating liquid through the absorptive element before passing the sample liquid therethrough. For example, quaternary amine for use in absorbing arsenic, selenium and/or hexavalent chrome, can exhibit its absorption ability only after it comes into contact with an OH ion, because this absorption ability comes from a reaction where an OH ion is substituted with a target anion. In this case, the absorptive element-activating liquid is used. This absorptive element-activating liquid may include sodium hydroxide and potassium hydroxide.

A size of the absorptive element may be freely determined to an extent allowing an absorption capacity of the absorptive element to be not saturated during a course of absorbing a target target substance. For example, it may be calculated how much a substance capable of being absorbed to the absorptive element is contained in a liquid, so as to determine the size of the absorptive element. While an absorptive element having a relatively small absorption capacity makes it easy to increase an enrichment rate, saturation adsorption is likely to occur. Thus, it is necessary to select an absorptive element having a size with a desired absorption capacity. If the absorptive element is expected to have a chromatography function, it should be designed to have a column shape with a length of at least 10 mm or more in a direction of the passage.

In the absorptive element, a porosity and a size of a continuous pore are determined to an extent allowing the continuous pores to reliably contact a sample liquid and causing no problem about clogging. Preferably, as used for the absorptive element, a membrane has a fiber mesh size of about 0.3 $\mu$m or more, and a fine particle has a particle size of about 2 to 50$\mu$ m. Further, a monolithic column preferably has a continuous pore with a pore size of about 1 to 50 $\mu$m.

In the aforementioned embodiments of the present invention, an adequate measurement result can be obtained using the above Empore™ disc cartridge available from 3M. This disc cartridge has a significantly small thickness. Thus, a volume of the eluent liquid required for eluting a heavy metal absorbed to the absorptive element can be reduced, for example, to an extremely low value of 9 to 15 $\mu$l, and therefore a concentration of the heavy metal in the eluent liquid becomes higher to achieve analysis with high sensitivity. In an analysis of a small amount of target substance, it is critical to reduce a required volume of the eluent liquid to an extremely low value, irrespective of the configuration of the absorptive element.

In addition, the absorptive element formed as such a thin membrane allows a liquid feed pressure required for passing a sample liquid therethrough to become almost zero. A reduction in size of a pump is essential to downsizing of the apparatus, and a reduction in the liquid feed pressure is effective in this regard. From this point of view, it is preferable to use the absorptive element formed as a thin membrane.

The detection cartridge is formed with a micro-passage for transferring and storing various liquids therewithin. The liquid transfer passage is defined by a groove having a width of about several hundred $\mu$m to several mm, and a depth of several hundred $\mu$m. Preferably, the passage has a sectional area of about 100 $\mu$m$^2$ to 1 mm$^2$. An excessively large sectional area of the passage is likely to cause a problem about clogging of the passage with fine particles residing therein, and/or difficulty in releasing gas bubbles. An inner wall surface of the groove defining the passage may be subjected to a hydrophilic treatment to ensure the liquid transfer. The hydrophilic treatment additionally provides a function of preventing gas bubbles from staying in the passage.

As to a mechanism for loading the detection cartridge into the read section of the processing unit, the detection cartridge is preferably loaded into a casing of the read section in a mechanical manner. In view of allowing the electrodes and the ports of the detection cartridge to be reliably connected to terminals, the valve mechanism and supply ports of various liquids, in their proper positions, the casing of the processing unit preferably has a holder portion for holding the detection cartridge at a predetermined position. The detection cartridge and the casing are formed, respectively, with a depression and a protrusion to be conformably fitted to each other. Thus, the depression and the protrusion will be engaged with each other to allow the detection cartridge to be reliably held by the casing of the processing unit.

The electrochemical detection is performed through a plurality of pin-shaped terminals fixed to the holder portion. Preferably, each of the terminals is arranged at a position of a corresponding one of the electrodes of the detection cartridge after being loaded into the processing unit, and designed to be moved inwardly and outwardly by an action of a spring located on an inward side thereof, so as to ensure a reliable contact therebetween. Thus, when the detection cartridge is loaded into the processing unit, each of the terminals fixed to the holder portion at the predetermined position immediately above a corresponding one of the electrodes of the detection cartridge will be reliably brought into contact with the electrode by a biasing force of the spring. Then, according to a predetermined measurement profile, a voltage is applied to these electrodes to detect a current flowing through the electrodes, and the detection signal is sent to a storage section and/or the display section.

A liquid feed operation section may comprise the liquid feed pump, the valve mechanism for alternately opening/closing the ports during a liquid feed operation, and an electronic board for controlling the pump and the valve mechanism. The valve mechanism is connected to respective containers respectively containing the eluent liquid, the electrolyte liquid, the absorptive element pretreatment liquid, a cleaning liquid, and others.

Preferably, the liquid feed pump is designed to stably feed a small volume of liquid at a constant flow rate without pulsation. More specifically, the liquid feed pump is designed to stably achieve a flow rate of about 5 to 100 $\mu$l/min, and have a liquid feed pressure of 0.01 to 10 MPa. Further, the liquid feed pump preferably has a small and lightweight pump body, and low power consumption. A liquid feed pump meeting these requirements includes a syringe pump. A preferable syringe pump is "Pencil Pump" available from Uniflows Corp of Japan.

In one embodiment of the present invention, it is preferable to perform a measurement while supplying a sample liquid containing an target substance, at a constant flow rate. For this purpose, flow-volume detection means is preferably employed. By having a sample containing a target substance, such as a heavy metals supplied in liquid form, a number of heavy metal ions passed around a surface of the electrode arrangement per unit time can be increased, and thereby an amount of the target substance to be deposited on the electrode is increased to allow the measurement to be performed with higher sensitivity. In addition, a fresh sample liquid can be continuously supplied at a constant flow rate to eliminate the need for taking account of influences of a remaining volume of the absorptive element-activating liquid, the cleaning liquid or the like, and managing a total volume of the sample liquid. This makes it possible to perform a highly accurate analysis only by managing the flow rate. Further, the flow rate can be changed depending on types or concentrations of target substances, so as to perform a measurement under adequate conditions, and measure various types of target substances using a common chip.

In the above embodiment, both analyses of high and low concentration ranges can be simultaneously performed with high measurement accuracy by controlling the flow rate (i.e., linear velocity) of the sample liquid. The control of the flow rate providing this advantage can be achieved only if the flow-volume detection means is employed.

In the above embodiment of the present invention employing the flow rate control, a plurality of working electrodes may be provided, and each of the working electrodes may be formed to have a different surface area, or a portion of the passage receiving therein each of the working electrodes may be formed to have a different dimension, such as width and/or depth, so that an target substance will be deposited onto a surface of each of the working electrodes in a different amount to allow the analyses of high and low concentration ranges to be simultaneously performed. For example, when a first working electrode having a diameter of 1 mm and a second working electrode having a diameter of 2.5 mm are used in combination, two sensitivities having about 19 times disparity therebetween can be utilized. In this case, preferably, the second electrode for the detection of a low concentration range is disposed on a relatively upstream side of the passage, and the first electrode for the detection of a high concentration range is disposed on a relatively downstream side of the passage.

The present invention can be applied to an analysis apparatus for liquid chromatography analysis. That is, in another embodiment of the present invention, the cartridge-type detection apparatus may use a detection cartridge for liquid chromatography analysis. In this case, the detection cartridge may include a sample-liquid adjustment column and an absorbance measurement cell, and the processing unit may include a light source, an incident optical system for directing light from the light source toward the absorbance measurement cell of the detection cartridge, and a spectrometer operable, in response to receiving light transmitted through the absorbance measurement cell, to produce information about an target substance. In this embodiment, it is preferable to perform a measurement while supplying a sample liquid containing an target substance, at a constant flow rate, as with the aforementioned embodiment.

An target substance to be subjected to the liquid chromatography analysis may include protein, nucleic-acid oligomer, DNA, RNA, peptide, agrichemical, synthetic organic molecule oligomer, polymer, additive, monosaccharide, disaccharide, oligosaccharide, polysaccharide, saturated fatty acid, unsaturated fatty acid, glyceride, phospholipid, steroid, anion and cation. A principle of the measurement is commonly known, and a measurement technique-type on the commonly-known principle may be used in the present invention. In the liquid chromatography analysis, the storing section may be designed in the same manner as that of the aforementioned concentration detection apparatus.

In the present invention, the processing unit may have a cartridge loading portion for detachably loading the detection cartridge thereto, and a reagent-tank mounting portion for detachably mounting the reagent tank thereto. Further, the plurality of ports of the detection cartridge may include a waste liquid port, and the processing unit may include therewithin a waste liquid tank for receiving therein a waste liquid from the detection cartridge. In this case, the line switching valve mechanism is designed to selectively provide liquid communication between the waste liquid port of the detection cartridge and the waste liquid tank.

The line switching valve mechanism and the tank switching valve mechanism may be disposed on a line-switching-valve plate and a tank-switching-valve plate, respectively. At least one of the line-switching-valve plate and the tank-switching-valve plate may have a structure prepared by fixedly laminating a plurality of plate elements each formed through an injection molding process using a plastic material or a cutting process using a plate material. In this case, a hole or passage groove necessary for liquid communication is pre-formed in at least one of the plate elements in a desired pattern. Each of the remaining plate members may have a hole or passage groove, or may have no hole or passage groove.

The above structure of the valve plate makes it possible to arrange a required passage line in a compact manner. In addition, this structure is advantageous in view of both production and maintenance, because it can prevent the occurrence of erroneous passage arrangement while reducing the number of components, and allows an operator or user to conveniently find clogging of the passage and liquid leakage, while facilitating a replacement operation. Further, the plate elements may be made of a transparent plastic material to provide an advantage of providing enhanced visibility of an inside of the valve plate. In an actual design, the valve plate can be formed to have a small volume, for example, of several cubic centimeters, and most of the required passage line can be advantageously arranged within this small space.

The plate element may be fixedly laminated by a process using an adhesive or sticker, a thermal bonding (joining) process, an ultrasonic bonding process or a diffusion bonding process. The diffusion bonding process comprises exposing to a high-temperature/high-pressure atmosphere a plurality of target members, so as to induce atomic diffusion in the members to allow respective contact surfaces of the adjacent members to be integrally fused with each other.

This technique is primarily used for metals, and can also be applied to plastic materials. A plastic material suitable for the diffusion bonding process includes acrylic resin, PEEK (polyether ether ketone) resin and PTFE (polytetrafluoroethylene).

In the present invention, the processing unit may have a housing which houses electronic processing means including a power supply and information processing means. The plurality of reagent tanks may include a cleaning liquid tank, an activating liquid tank and an eluent liquid tank. The eluent liquid being passed through the passage within the detection cartridge allows an target substance temporarily stored in the storing section of the detection cartridge to be eluted therefrom and sent to the passage within the detection cartridge so as to be subjected to a desired analysis.

As mentioned above, the storing section may be comprised, for example, of the absorptive element having a property of absorbing an target substance. Further, at least one of the plurality of reagent tanks may be an eluent liquid tank. In this case, an eluent liquid stored in the eluent liquid tank has a function of eluting an target substance stored in the storing section which may be comprised, for example, of the absorptive element having a property of absorbing the target substance. The effectiveness of the eluent liquid depends on absorption mechanisms. Thus, a specific type of eluent liquid is selected in consideration of chemical characteristics of absorption. For example, in case of an eluent liquid containing an ion capable of being easily absorbed to a surface of the absorptive element, when the eluent liquid is passed through the absorptive element, the ion in the eluent liquid is exchanged with an target substance absorbed to the absorptive element in the form of an ion to allow the target substance to be eluted from the absorptive element. In the present invention, an eluent liquid having various composition may be used depending on target substances.

The present invention can also be implemented as an apparatus for detections-type on immunoassay. In this case, an target substance may include various allergens, such as egg yolk, egg white, bovine milk, peanut, shrimp, crab, fish, shellfish, soybean, mango, other food item known as an allergen, dust mite, feather, pollen, fungus, bacillus, cockroach and dog's or cat's fur. The target substance may further include endocrine disrupting chemical, agrichemical, immunoglobulin including IgE and IgG, histamine, gene (RNA), stress marker, antigen or antibody included in various proteins, human or animal blood, blood components, urine and saliva, and component indicative of a specific disease.

The immunoassay is classified into: labeling assay and nonlabeling assay (sandwich assay); or homogeneous assay and inhomogeneous assay, by types of labels to be used or methods for separating an antigen and an antibody after a reaction therebetween. A specific analysis is established by a combination thereof. While various combinations are conceivable, a preferable combination for use in the present invention will be described later.

Variety of detection techniques have been known and include: the labeling assay, such as immunonephelometry, latex nephelometry, and an assay using an immunosensor comprising an antigen electrode and an antibody electrode; and the nonlabeling assay, such as enzyme immunoassay, fluorescence immunoassay, luminescence immunoassay, spin immunoassay, metallo immunoassay, particle immunoassay and viroimmunoassay, and the present invention can be applied to any one of these processes.

In one embodiment, the storing section of the detection cartridge may be formed as a filter, and an target substance, such as an antigen or antibody, is immobilized onto a surface of the filter. The target substance may be directly immobilized onto the surface of the filter, or may be immobilized through an appropriate ligand. For example, this immobilization may be achieved by immersing a plastic material or a carbon fiber in an antigen and/or antibody solution. Depending on antigens or antibodies to be used, it is desirable to perform the immobilization through a metal as in case of immobilizing mercapto to gold. In this case, a metal coating can be readily formed by subjecting a carbon fiber to a plating process, a sputtering process or a plasma treatment.

An antigen and/or an antibody are appropriately selected or combined depending on an target substance. For example, a conceivable combination may include: avidin for biotin; protein A for immunoglobulin; hormone receptor for hormone; DNA receptor for DNA; RNA receptor for RNA; and drug receptor for drug.

A process of the immunoassay is roughly divided into a first stage of inducing a antigen-antibody reaction, and a second stage of detecting a label reacted with the antigen or the antibody. An target substance and other substance are separated from each other in the storing section,-type on the antigen-antibody reaction in the first stage, and the separated target substance is analyzed qualitatively and/or quantitatively in the detection mechanism disposed on a downstream side relative to the storing section. The detection mechanism used in the second stage may be-type on electrochemical analysis or optical analysis. In the electrochemical analysis, the same electrode arrangement as that in the aforementioned concentration detection apparatus may be employed. In the optical analysis, the same optical cell as that in the liquid chromatography analysis may be used.

A labeled substance for the immunoassay may include protein label, chemiluminescent substance label, and metal ion. In the immunoassay using a protein label, after the label is immobilized to the storing section, a substrate is passed through to the storing section, and a resulting reaction product is detected by the downstream detection mechanism. For example, a combination of the label and the substrate may include: glucose for glucose oxidase; xanthine for xanthine oxidase; amino acid for amino acid oxidase; ascorbate for ascorbate oxidase; acyl-CoA for acyl-CoA oxidase; cholesterol for cholesterol oxidase; galactose for galactose oxidase; oxalate for oxalate oxidase; and sarcosine for sarcosine oxidase.

The optical analysis may be performed using an enzyme label, such as peroxidase, β-galactosidase or alkaline phosphatase. A colorimetric method or a fluorescent method may be used in the optical analysis.

The detection apparatus of the present invention can also be applied to an analysis-type on other principle, such as an analysis-type on a specific binding reaction. In this case, the storing section may be made of a substance which exhibits a specific binding reaction, so as to be applicable to IMAC (immobilized metal affinity chromatography), hybridization of complementary DNAs, and other analysis of various proteins.

In any of the above embodiments, the cartridge-type detection apparatus of the present invention allows an element or component necessary for complicated replacement, cleaning and/or refresh operations for each measurement to be mounted to the detection cartridge. For example, the section corresponds to the electrodes for the electrochemical analysis, the chromatography column for the liquid chromatography, or the solid-phase antigen or antibody for the immunoassay, and this component is mounted to the detection cartridge.

The optical cell can be readily incorporated in the detection cartridge, and this arrangement is desirable In view of reducing a load on a measurer or operator. Alternatively, considering that the optical cell can be simply cleaned with water, the optical cell may be mounted to the processing unit.

BRIEF DESCRIPTION OF DRAWINGS

FIG 1 is an exploded perspective view showing a detection cartridge according to one embodiment of the present invention.

FIG 2(a) is a perspective view showing an assembled state of the detection cartridge.

FIG 2(b) is a sectional view taken along the line A-A in FIG 2(a).

FIG 2(c) is a sectional view taken along the line B-B in FIG 2(a).

FIG 3 is a sectional view schematically showing a liquid passing the detection cartridge.

FIG 3(a) is a perspective view showing the flow of a sample liquid in the detection cartridge.

FIG 3(b) is a perspective view showing an attached state of a syringe holder.

FIG 3(c) is a perspective view showing the flow of an eluent liquid in the detection cartridge.

FIG 4 is an exploded perspective view showing the structure of a processing unit.

FIG 4(a) is a perspective view showing a state when the detection cartridge is inserted into a cartridge holder.

FIG 4(b) is a perspective view showing a state after the cartridge holder is closed.

FIG 4 (c) is an exploded perspective view showing an internal structure of the cartridge holder.

FIG 5 is a perspective external view of the processing unit.

FIGS. 6(a) and 6(b) are schematic diagrams showing the processing unit connected with external devices.

FIG 7 is a block diagram of an electric system in the processing unit.

FIG 8(a) is a flowchart showing a part of a measurement process.

FIG 8(b) is a flowchart showing a part of the measurement process following FIG 8(a).

FIG 8(c) is a flowchart showing a part of the measurement process following FIG 8(b).

FIG 8(d) is a system diagram generally showing a detection apparatus according to another embodiment of the present invention.

FIG 9 is a graph showing potential-current curves obtained from a measurement of arsenic and selenium.

FIG 10 is a graph showing potential-current curves obtained from a measurement of cadmium, lead and mercury.

FIG 11 is a schematic sectional view showing one example of modification of an enrichment section in the detection cartridge.

FIG 12 is a schematic sectional view showing another example of modification of the enrichment section.

FIG 13(a) is a perspective view showing an analysis unit for use with a detection cartridge, according to another embodiment of the present invention.

FIG 13(b) is a perspective view showing the analysis unit in FIG 13(a), wherein a cover is detached therefrom.

FIG 13(c) is a perspective view showing a lower body of the analysis unit.

FIG 14(a) is an exploded perspective view showing a detection cartridge for use in electrochemical analysis, according to another embodiment of the present invention.

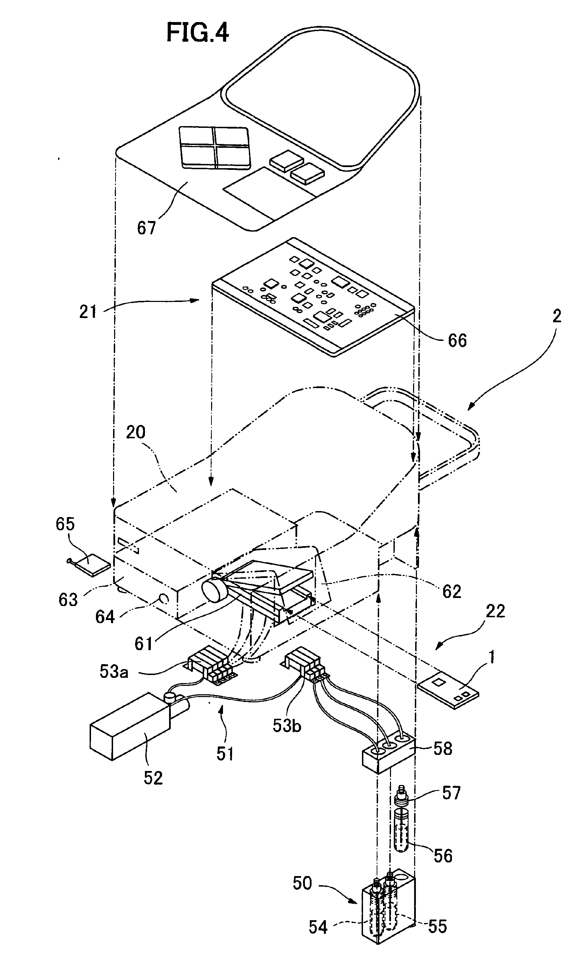

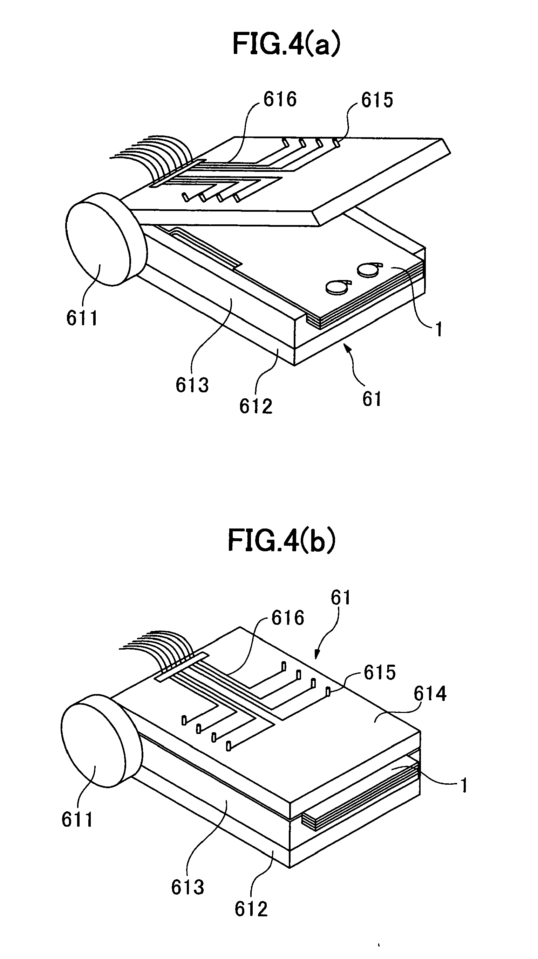

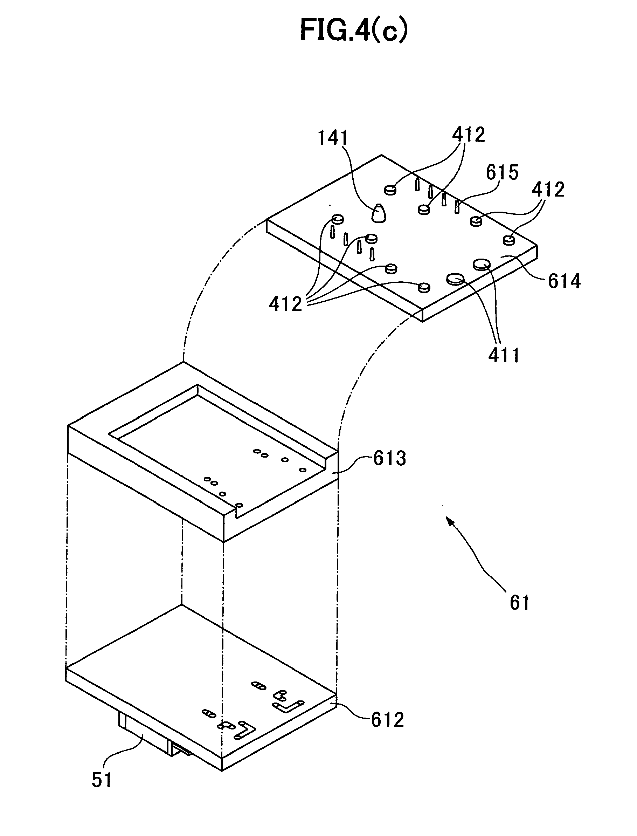

FIGS. 14 (b)-(i) to (b)-(iii) show an assembled state of the detection cartridge, wherein FIGS. 4 (b)-(i), (b)-(ii) and (b)-(iii) are, respectively, a perspective view, a sectional view taken along the line A-A in FIG 4 (b)-(i), and a sectional view taken along the line B-B in FIG 4 (b)-(i).

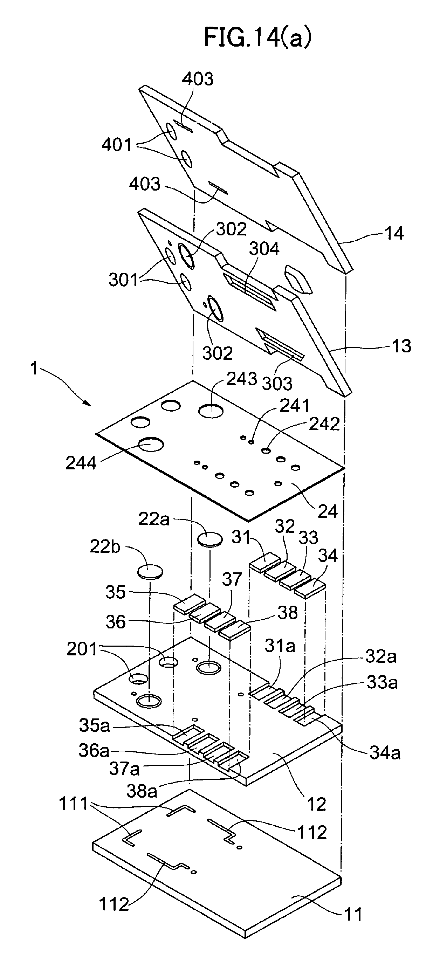

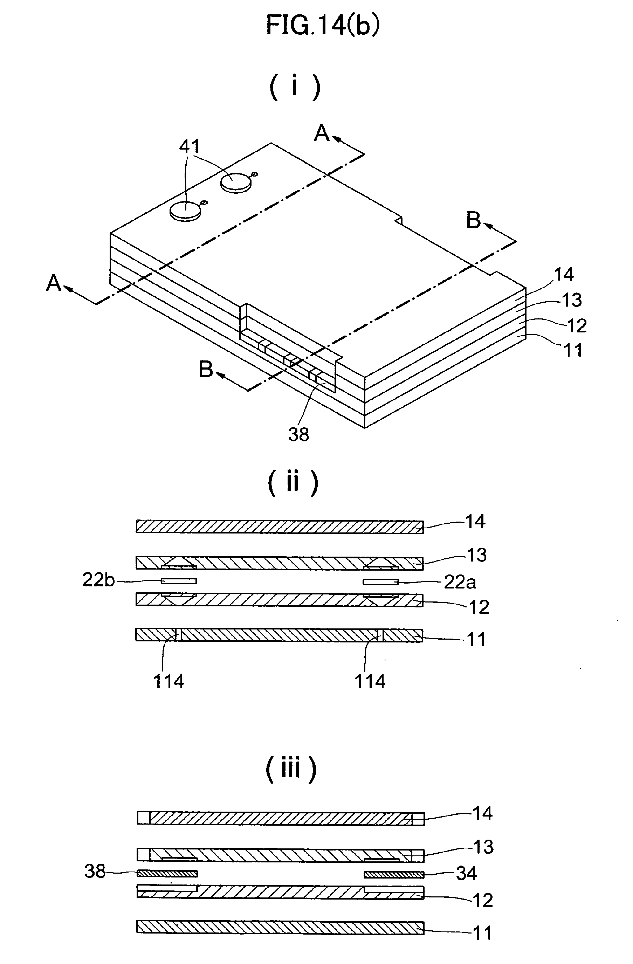

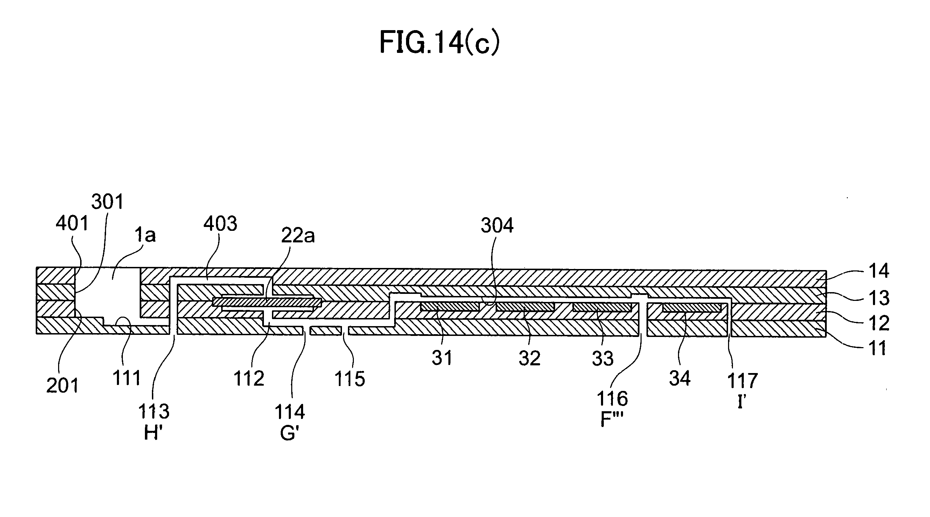

FIG 14(c) is a vertical sectional view of the detection cartridge in FIG 14(a).

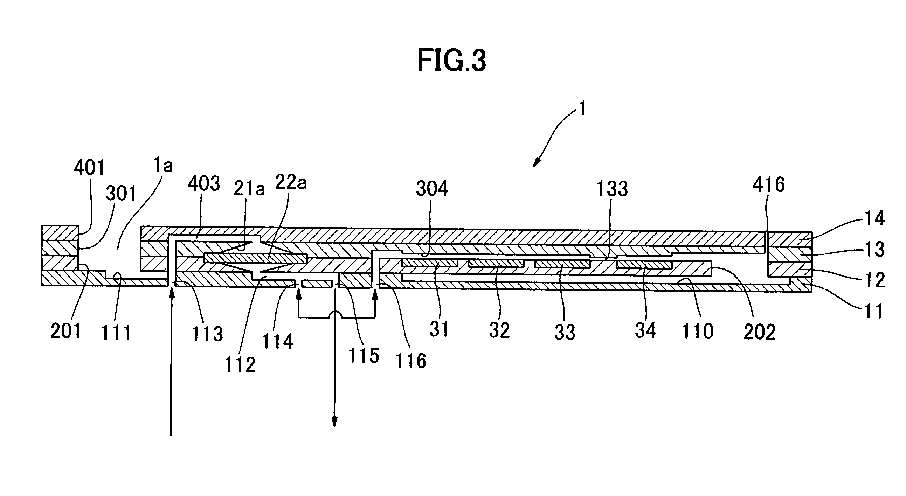

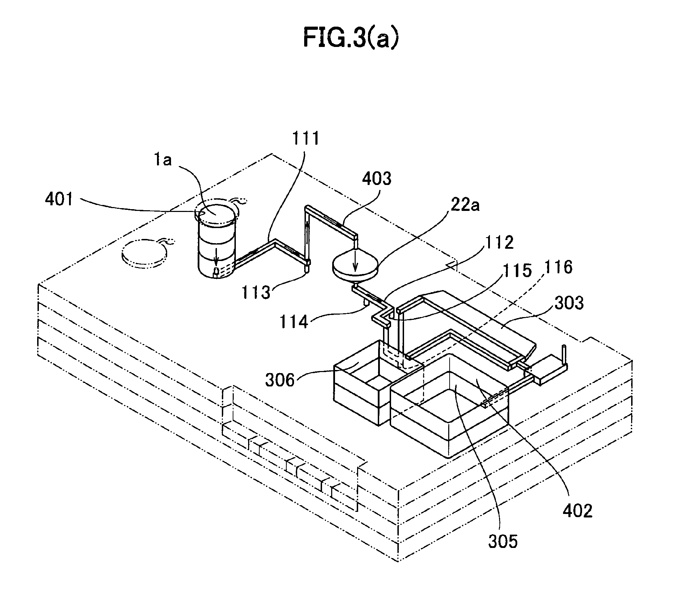

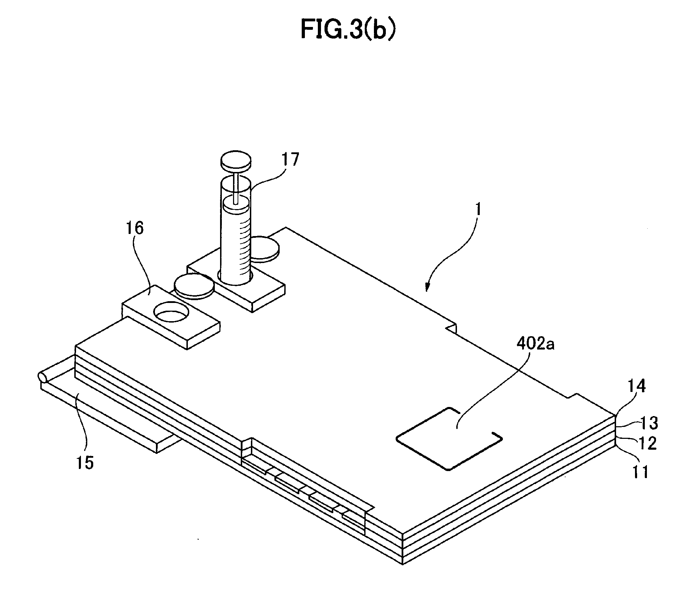

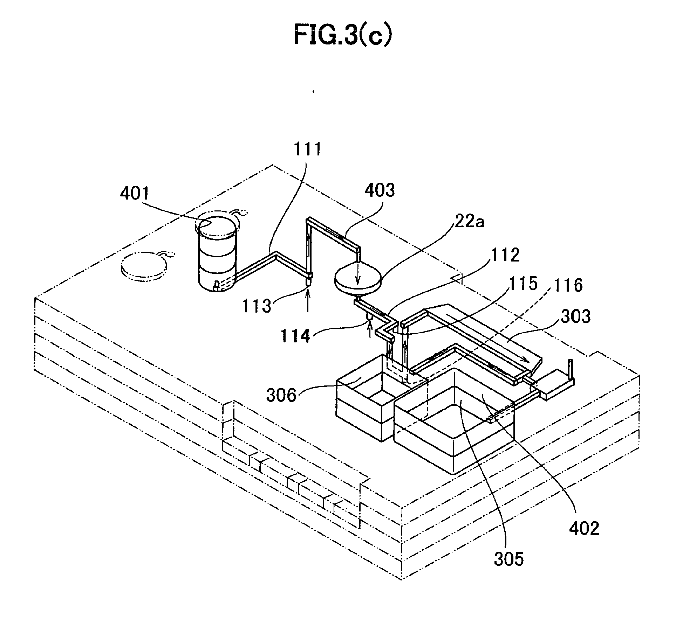

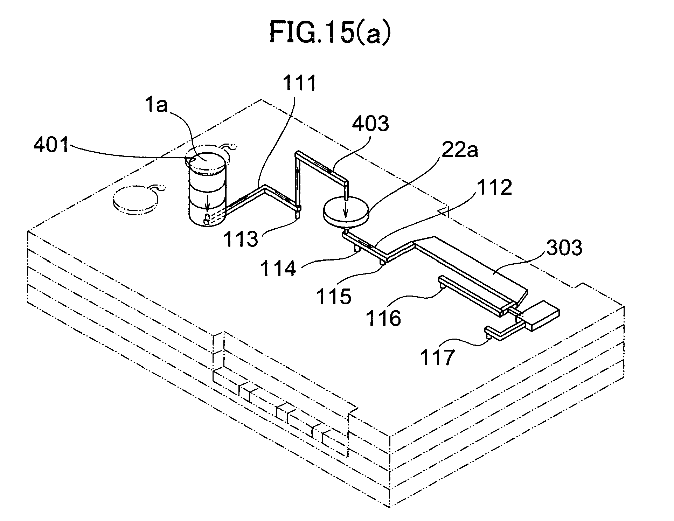

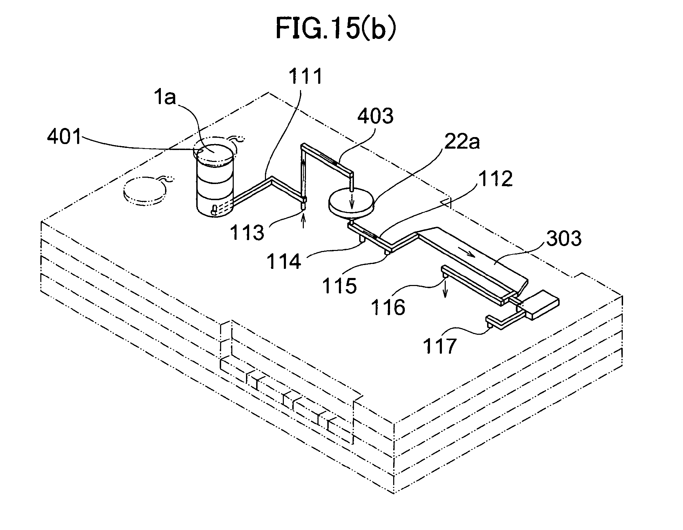

FIG 15(a) is a perspective view showing the flow of a liquid in the detection cartridge in FIG 14(a), which corresponds to FIG 3(a).

FIG 15(b) is a perspective view showing the flow of a liquid in the detection cartridge in FIG 14(a), which corresponds to FIG 3(c).

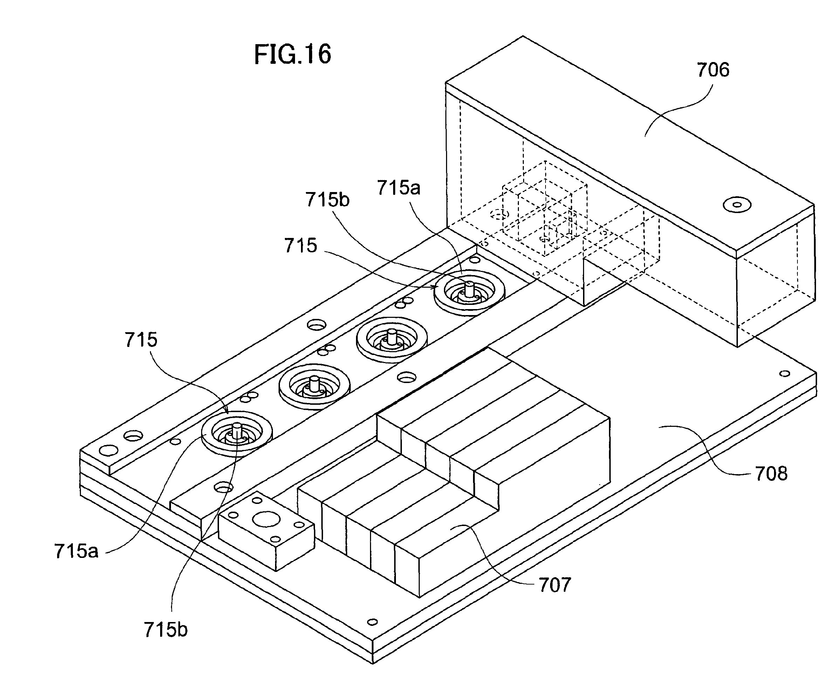

FIG 16 is a perspective view showing a positional relationship of a tank-switching line plate, a reagent tank and a switching valve mechanism.

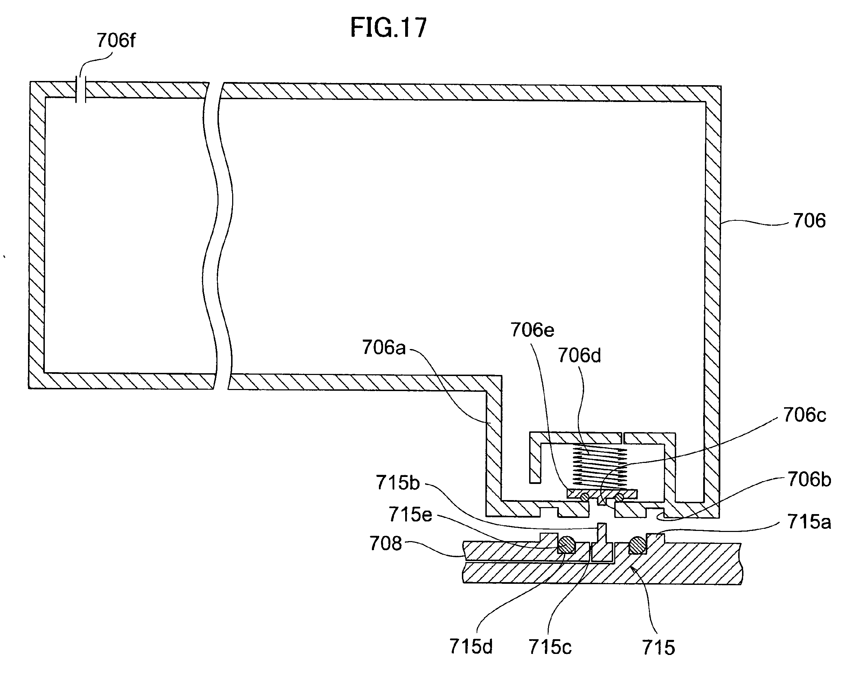

FIG 17 is a sectional view showing a connection mechanism for the reagent tank.

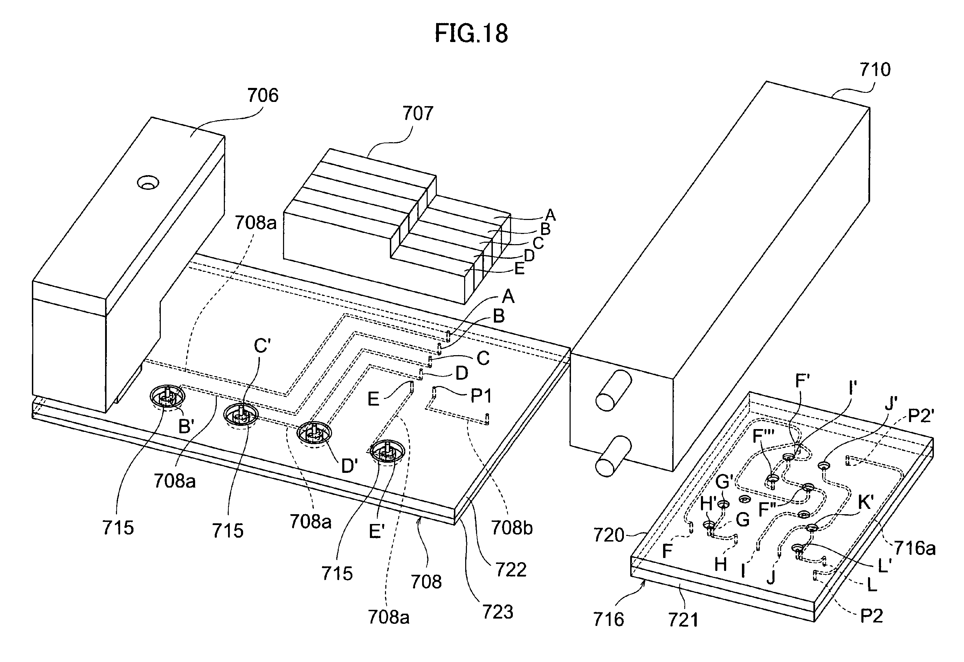

FIG 18 is a perspective view showing an arrangement of various components in an upper body of the analysis unit.

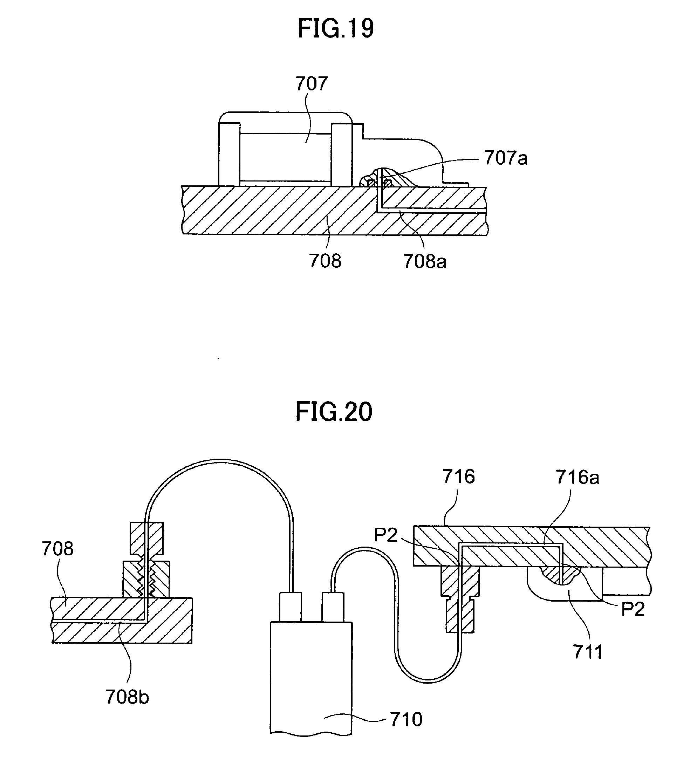

FIG 19 is a sectional view showing a connection between the tank-switching line plate and the switching valve mechanism.

FIG 20 is a schematic diagram showing a relationship of connection of a liquid feed pump, the tank-switching line plate and a destination-switching line plate.

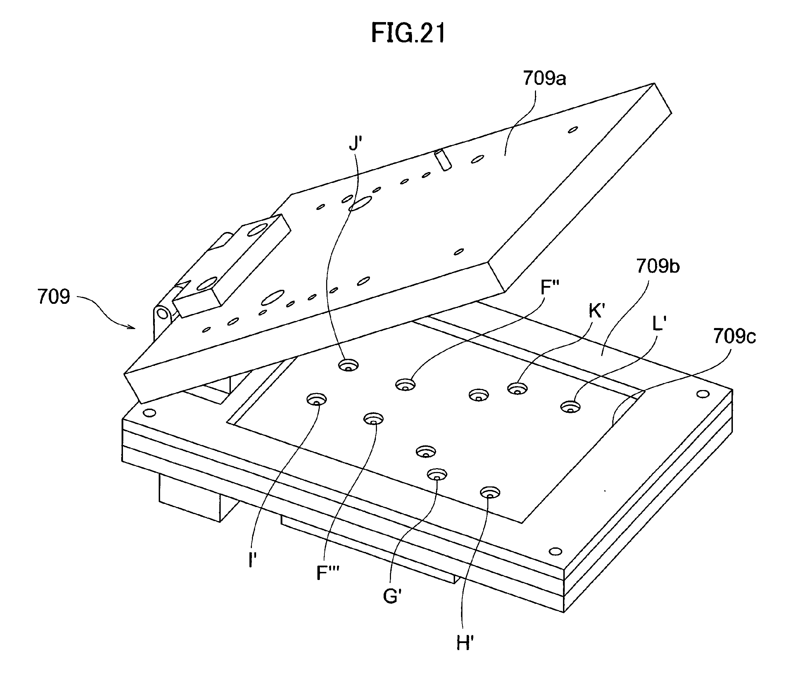

FIG 21 is a perspective view showing a cartridge holder for use in the analysis unit.

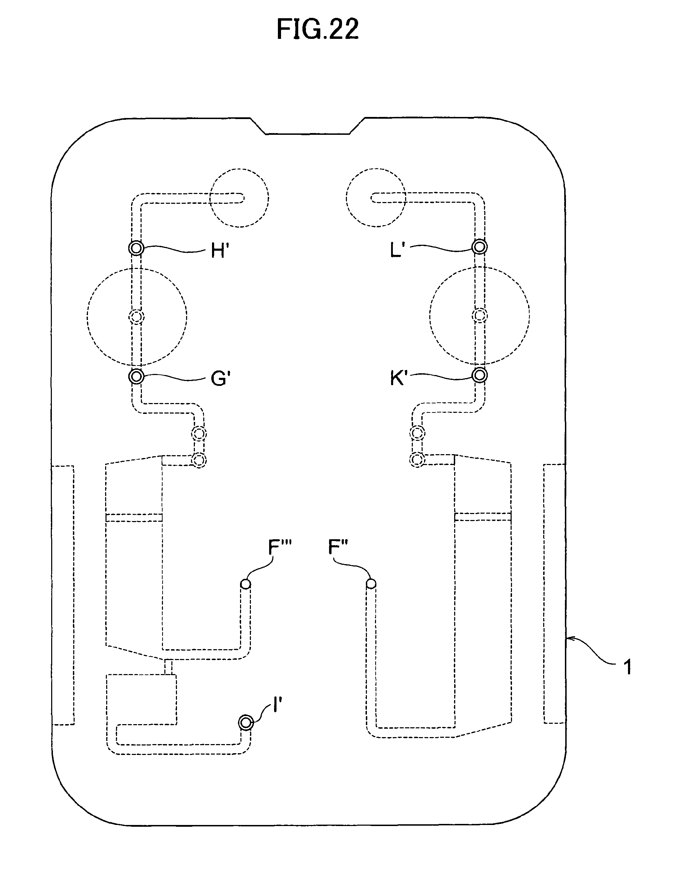

FIG. 22 is a plan view showing a plurality of ports in a bottom surface of the detection cartridge.

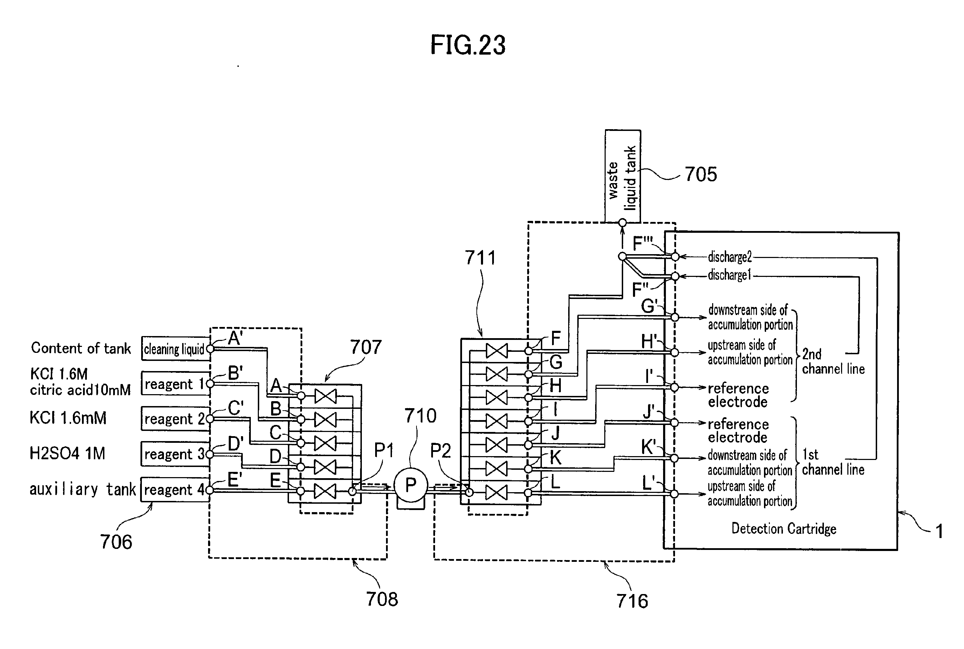

FIG 23 is a system diagram showing a relationship of connection of respective switching valves in the analysis unit.

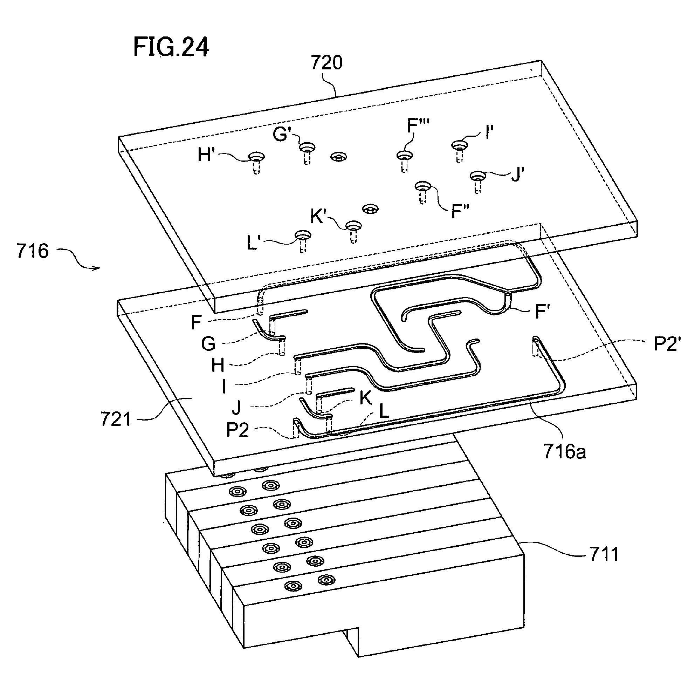

FIG 24 is an exploded perspective view showing the structure of the destination-switching line plate.

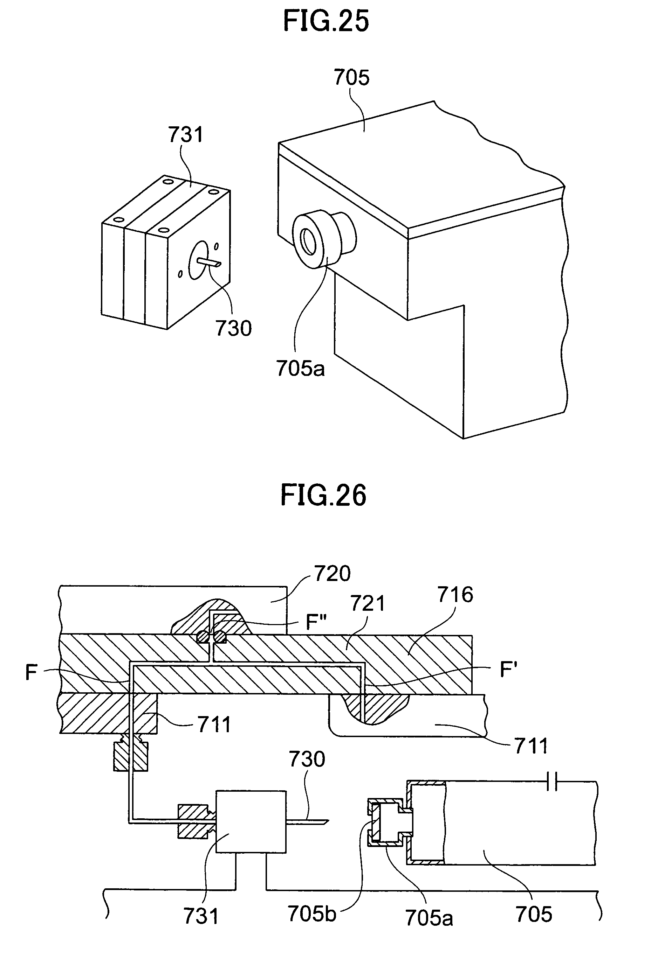

FIG 25 is an exploded fragmentary perspective view showing a connection with a waste tank.

FIG 26 is a schematic diagram showing a connection with the waste tank.

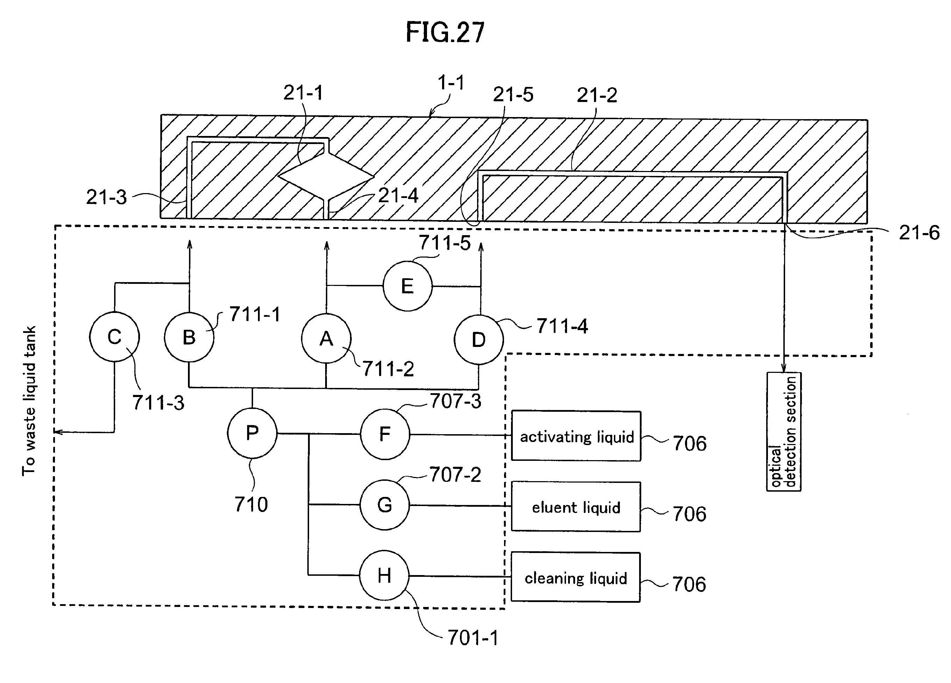

FIG 27 is a system diagram showing a detection apparatus for use in chromatography analysis, according to another embodiment of the present invention.

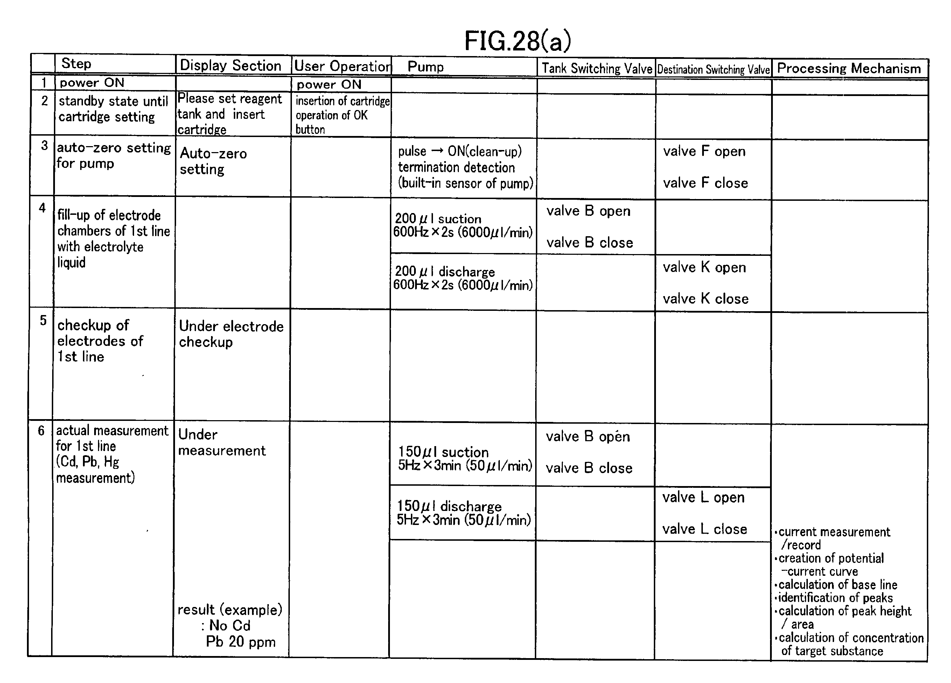

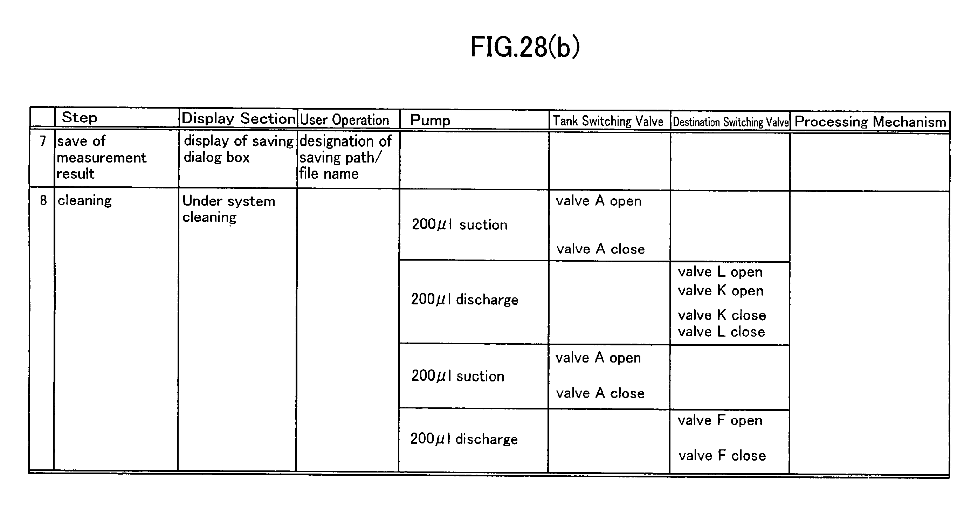

FIG 28(a) is a table showing an initial stage of a detection process which is performed using a first detection passage line of a detection cartridge for concentration analysis.

FIG 28(b) is a table showing a last stage of the detection process which is performed using the first detection passage line of the detection cartridge for concentration analysis.

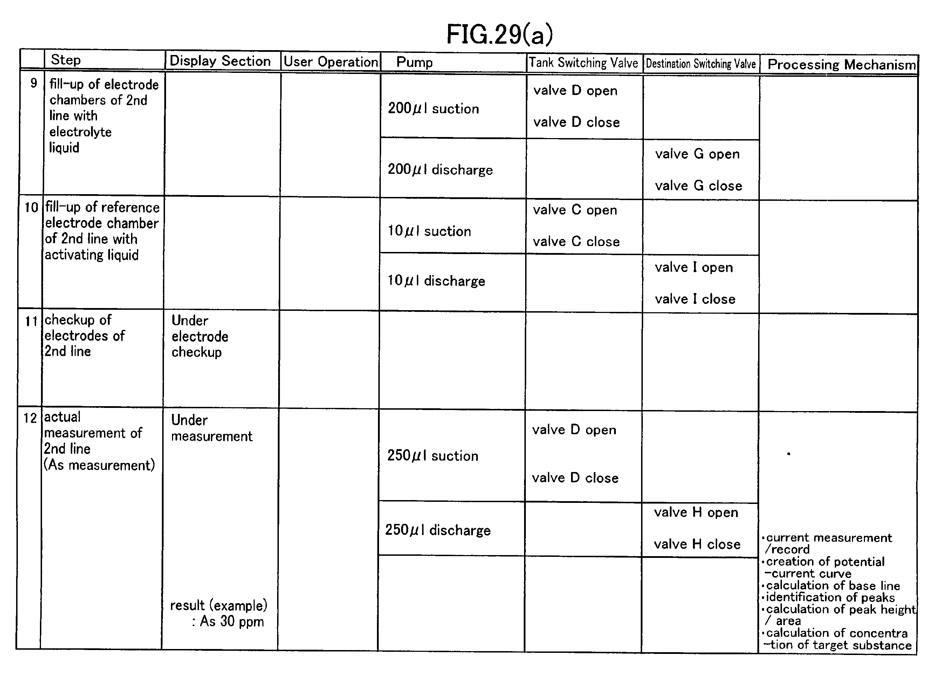

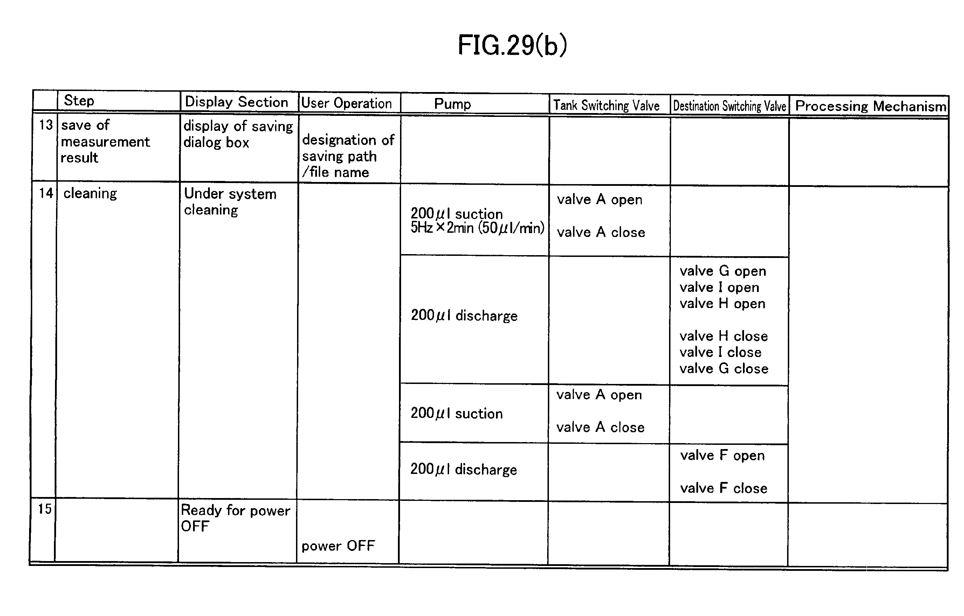

FIG 29(a) is a table showing an initial stage of a detection process which is performed using a second detection passage line of the detection cartridge for concentration analysis.

FIG 29(b) is a table showing a last stage of the detection process which is performed using the second detection passage line of the detection cartridge for concentration analysis.

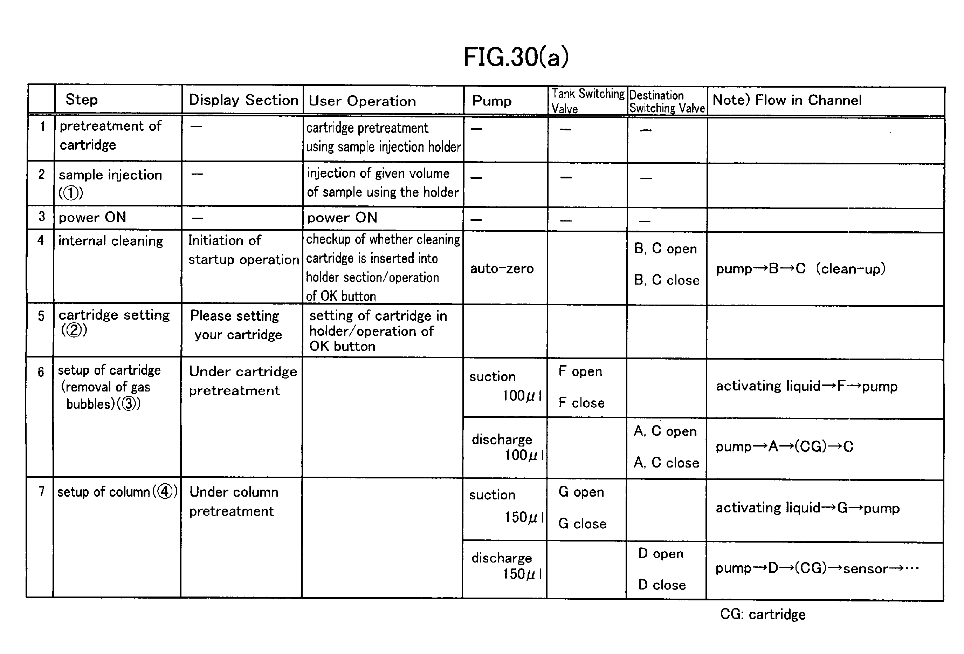

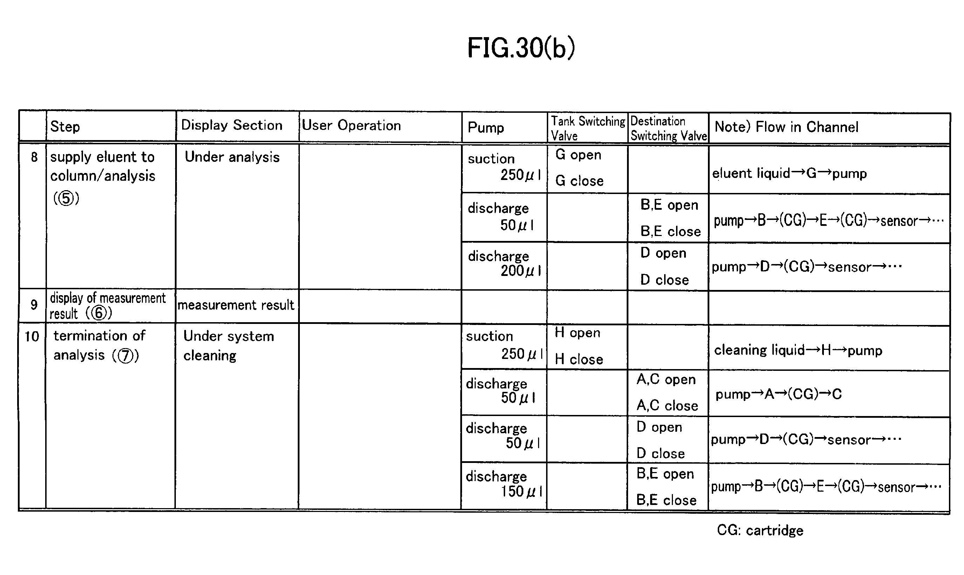

FIG 30(a) is a table showing an initial stage of a detection process which is performed using a detection cartridge for chromatography analysis.

FIG 30(b) is a table showing a last stage of the detection process which is performed using the detection cartridge for chromatography analysis.

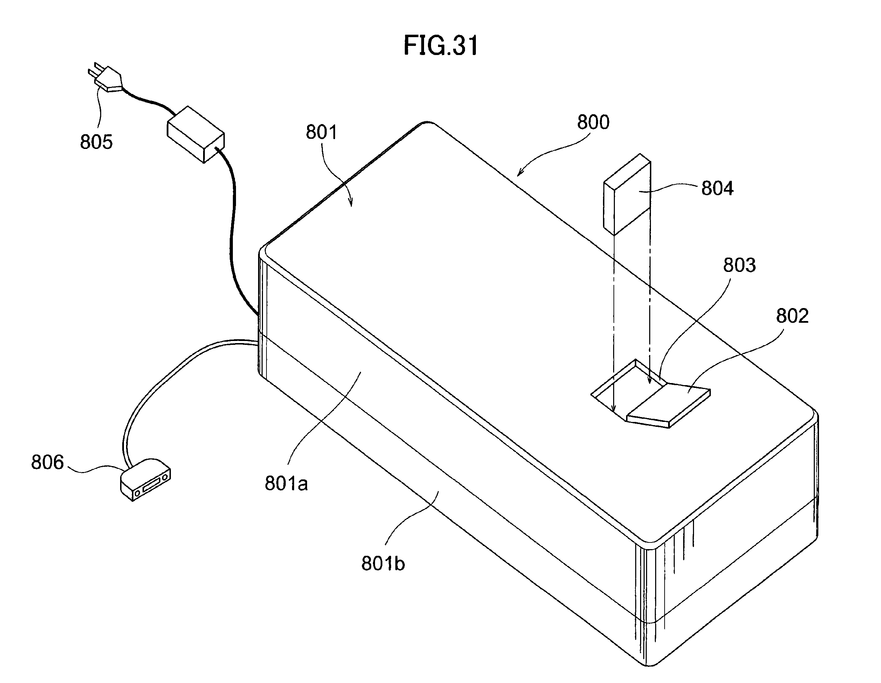

FIG 31 is a perspective external view showing a liquid-chromatography analysis apparatus according to another embodiment of the present invention.

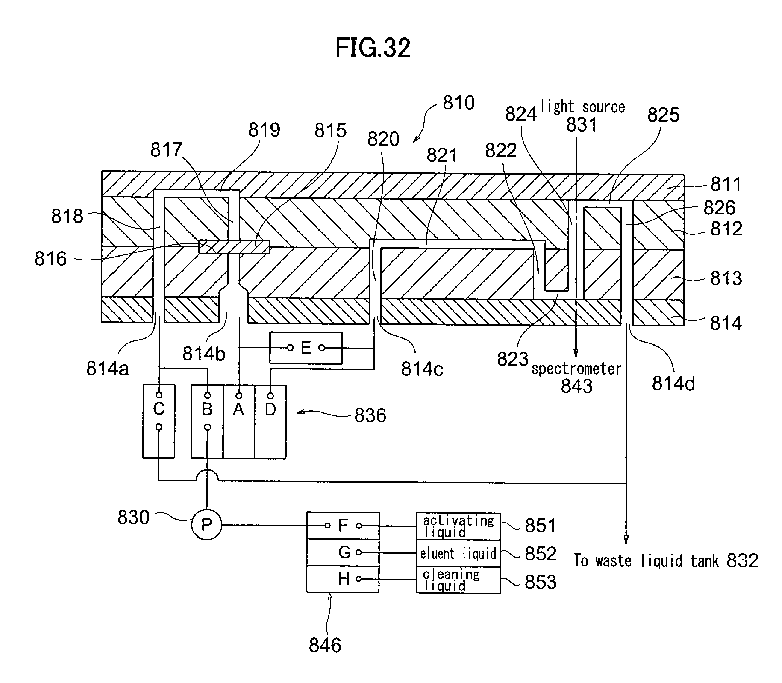

FIG 32 is a sectional view showing a detection cartridge for use in the analysis apparatus in FIG 31, together with a relationship of connection with a switching valve mechanism.

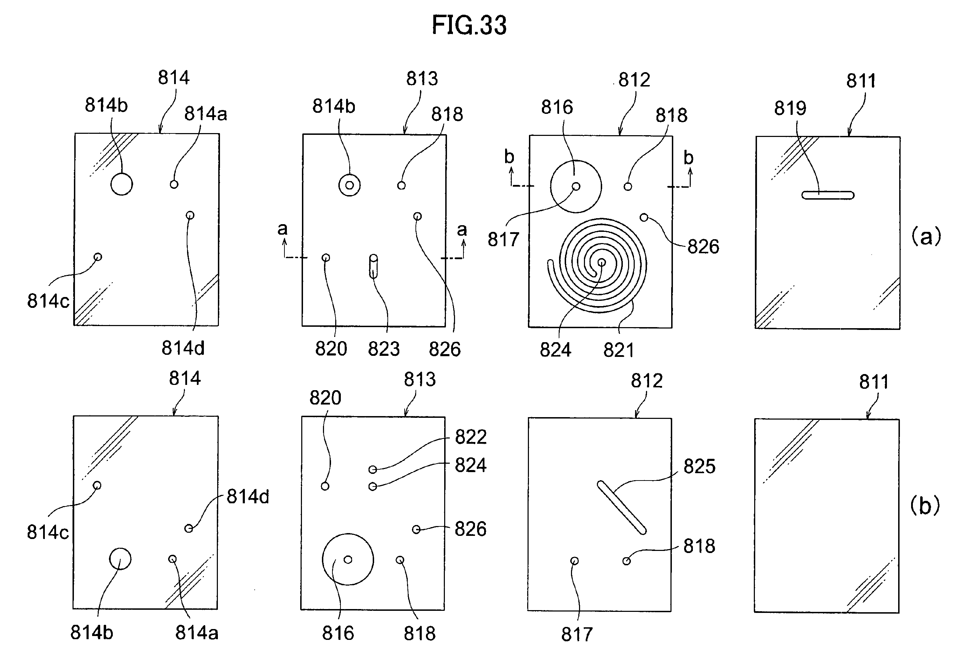

FIGS. 33(a) and 33(b) are exploded diagrams showing the structure of the detection cartridge in FIG 32, wherein FIG 33(a) shows respective bottom surfaces of plastic plates constituting the detection cartridge, and FIG. 33(b) shows respective top surface of the plastic plates.

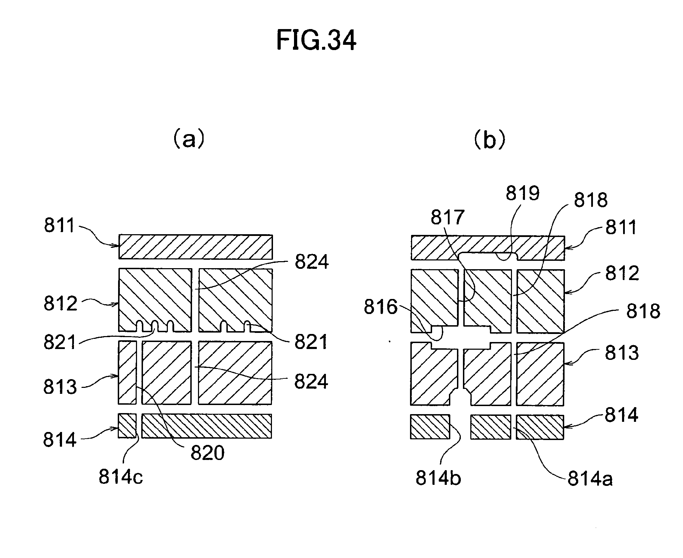

FIGS. 34 (a) and 34(b) are, respectively, a sectional view taken along the line "a"-"a" in FIG. 33(a), and a sectional view taken along the line "b"-"b" in FIG 33(a).

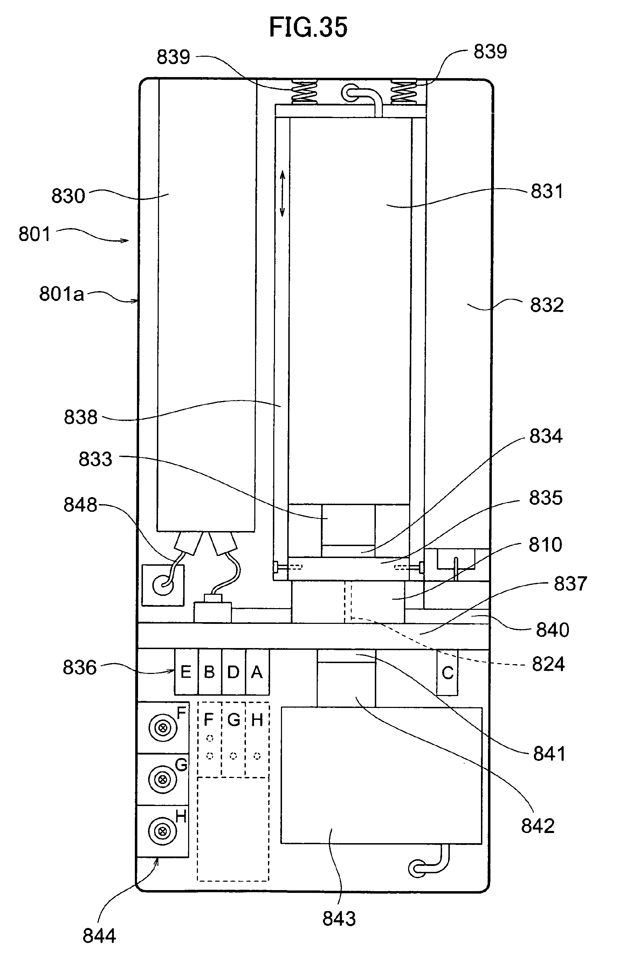

FIG. 35 is a top plan view showing an internal structure of an upper housing in an analysis unit of the analysis apparatus in FIG 31.

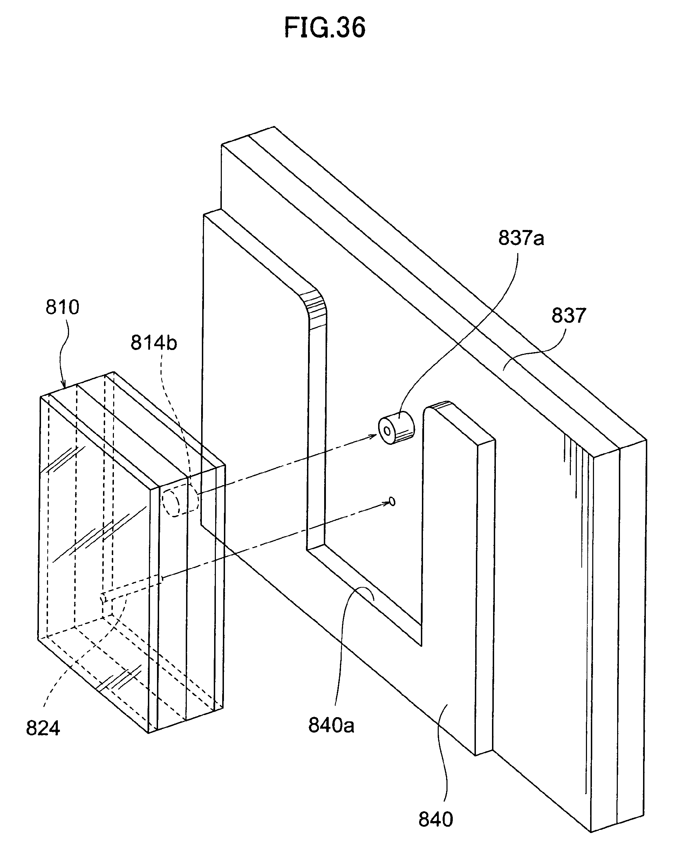

FIG 36 is a perspective view showing a relationship of a passage-switching-valve plate, a guide member and the detection cartridge.

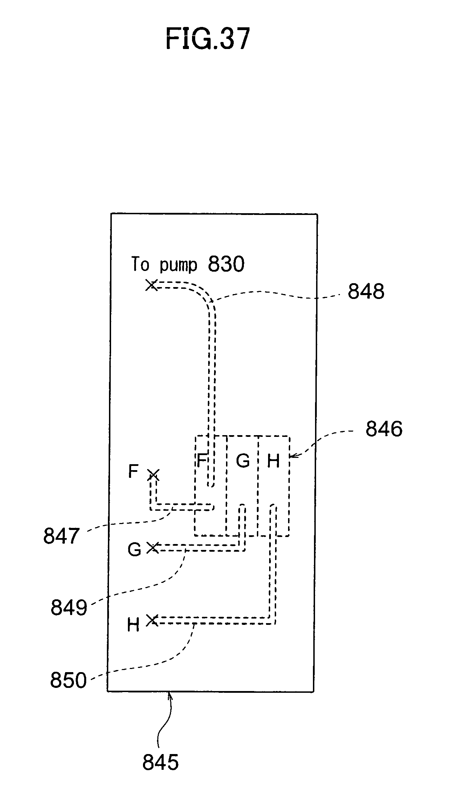

FIG 37 is a top plan view showing a tank-switching-valve plate.

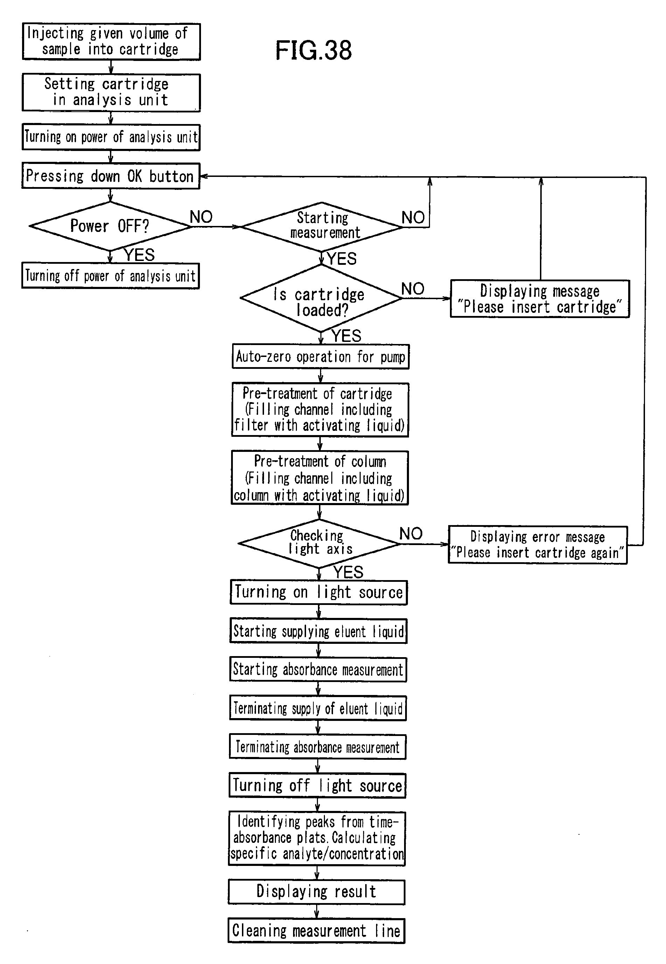

FIG 38 is a flowchart showing an operational process of the liquid-chromatography analysis apparatus in FIGS. 31 to 37.

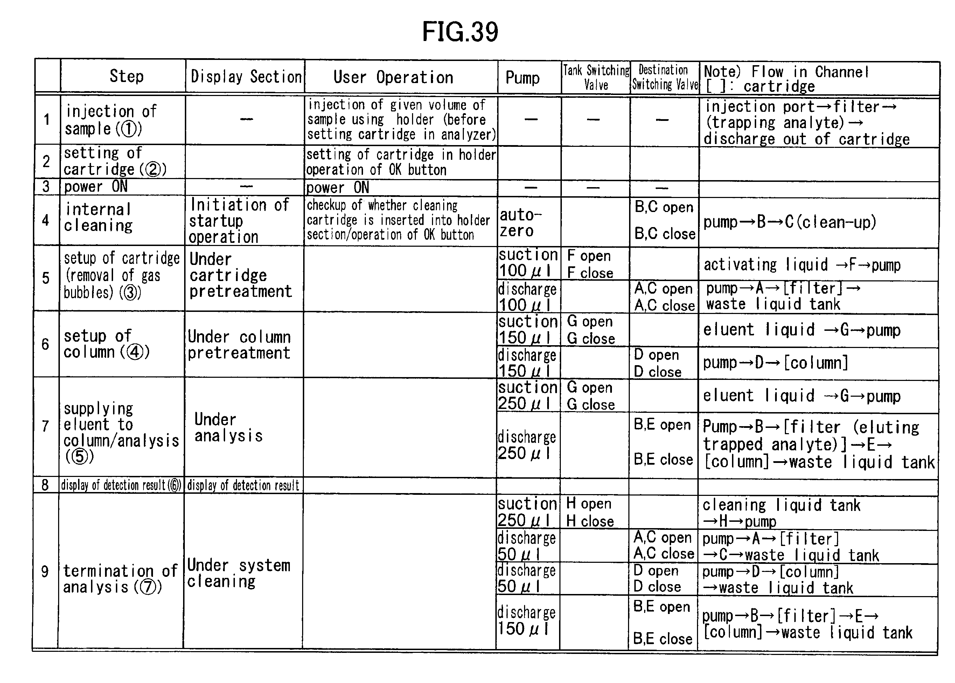

FIG 39 is a table showing an analytic operation in a time-series manner.

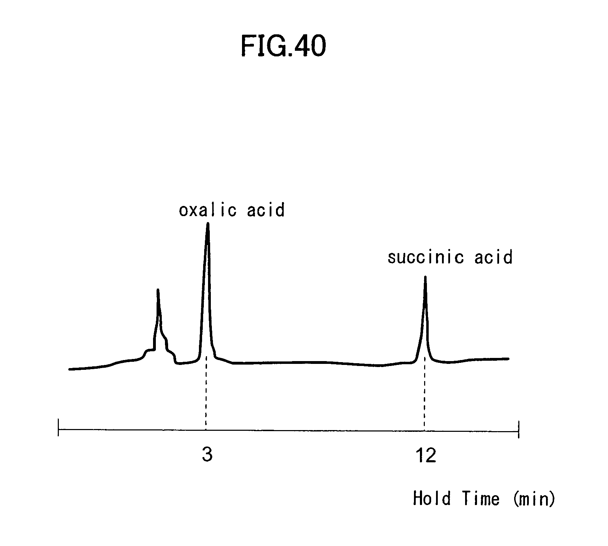

FIG 40 is a graph showing a detection result in Example 1.

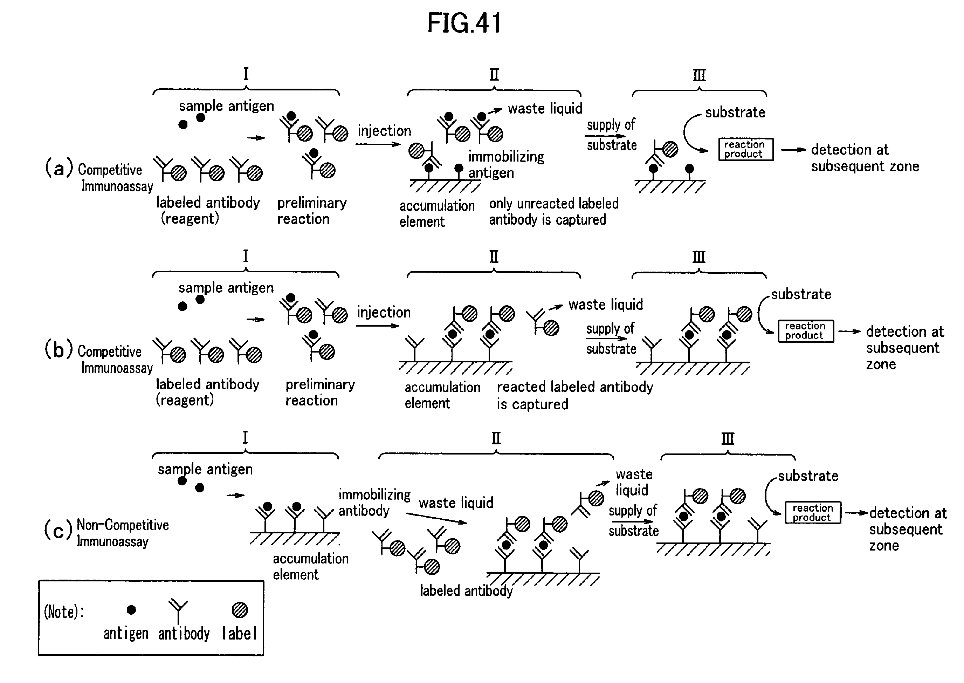

FIGS. 41 (a) to 41(c) are process flow diagrams schematically showing several examples where the present invention is applied to immunoassay, wherein: FIG 41(a) shows one example of application to competitive immunoassay; FIG 41(b) shows one example of application to so-called sandwich immunoassay in the competitive immunoassay; and FIG 41(c) shows one example of application to non-competitive immunoassay.

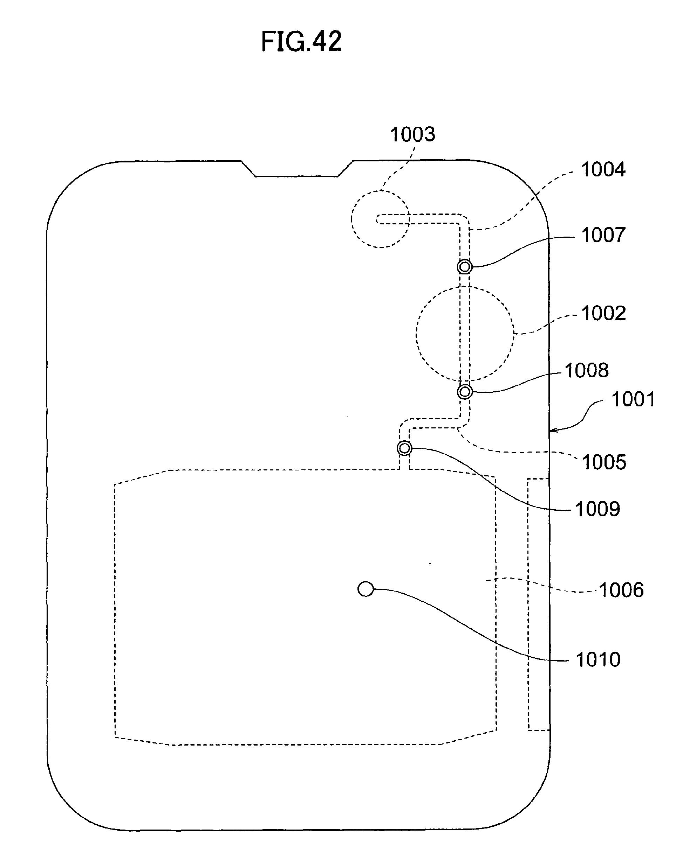

FIG 42 is a bottom view showing a detection cartridge used in Example 5.

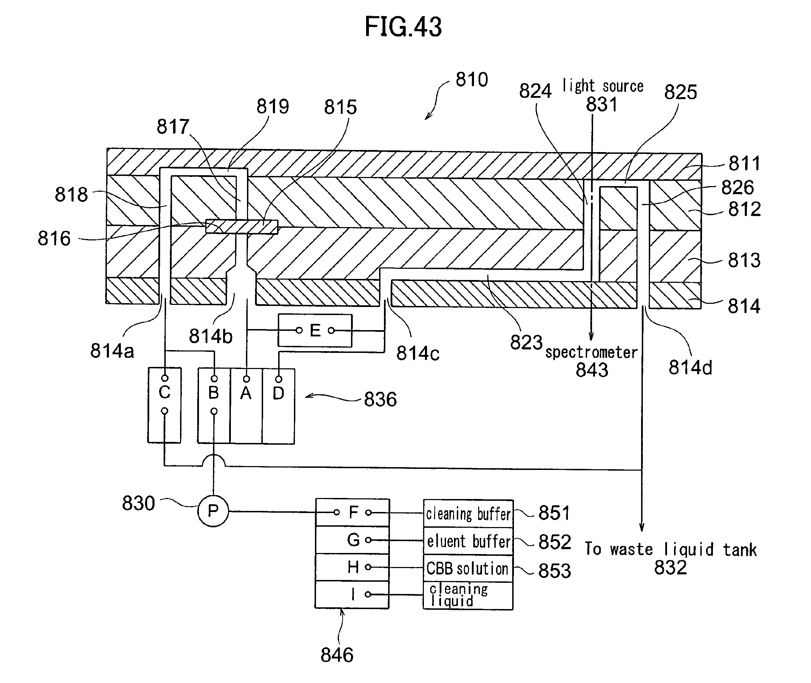

FIG 43 is a sectional view showing a detection cartridge used in Example 6.

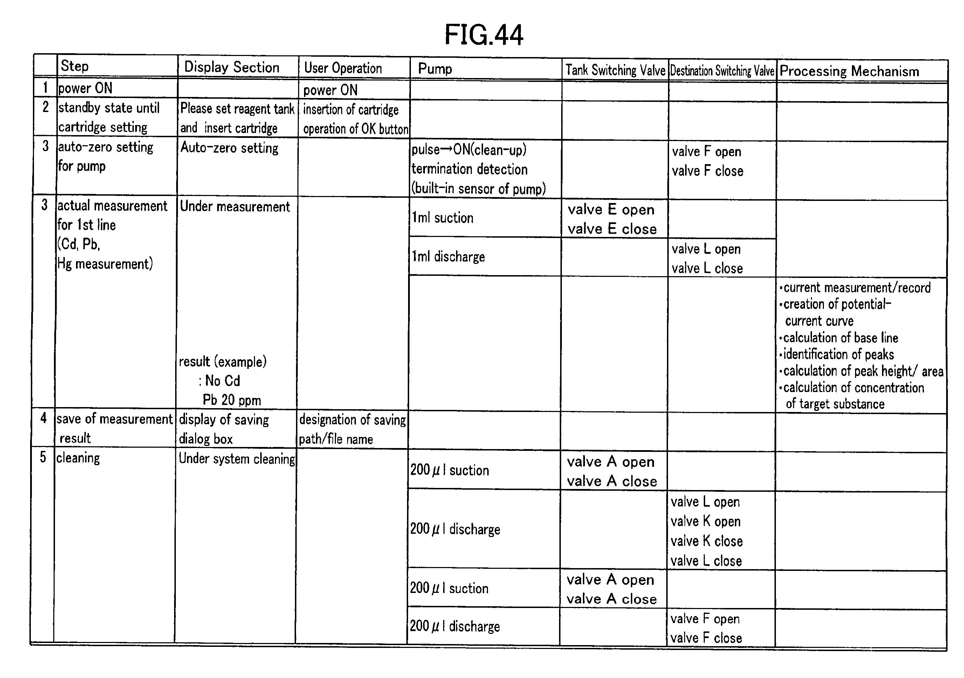

FIG 44 is a table showing a process flow in Example 5.

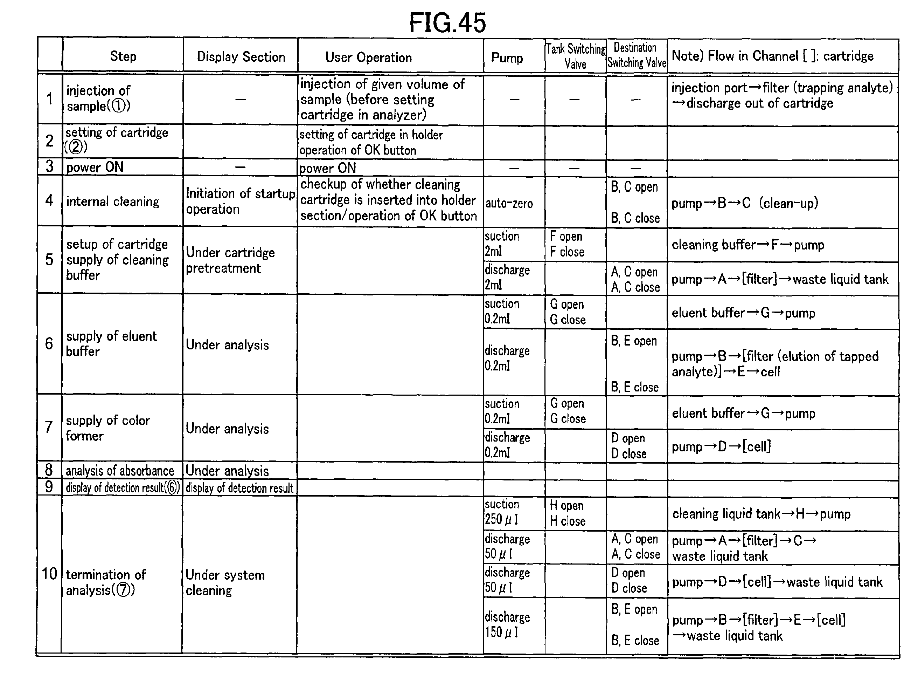

FIG 45 is a table showing a process flow in Example 6.

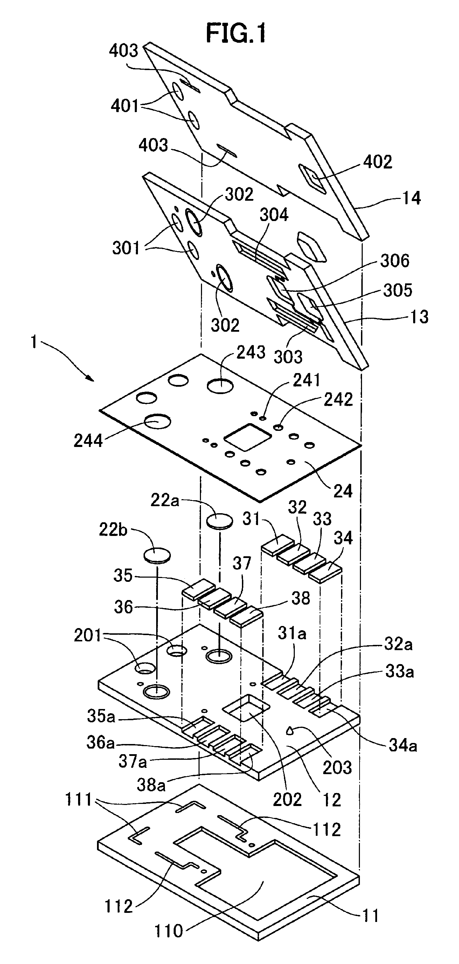

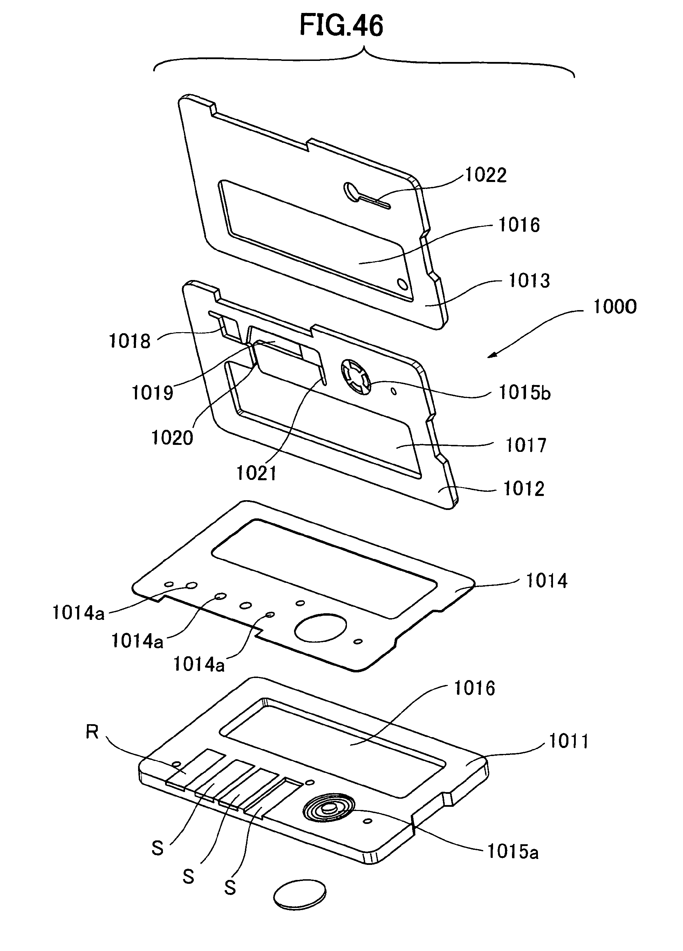

FIG 46 is an exploded perspective view showing one example of a detection cartridge comprising three plastic base plates, which approximately corresponds to FIG 1.

BEST MODE FOR CARRYING OUT THE INVENTION

The present invention will now be described-type on an embodiment thereof illustrated in the drawings.

[Measurement of Various Heavy Metals using Detection Cartridge]

FIG 1 is an exploded diagram showing a detection cartridge suitable for use in a concentration analysis of heavy metals, according to one embodiment of the present invention. In the illustrated embodiment, the detection cartridge 1 comprises, in combination, four resin base plates 11, 12, 13, 14 superimposed on each other in this order in an upward direction. In a typical example, each of the base plates has a planar size of 35 mm × 50 mm and a thickness of 1 mm, and the base plates are superimposed on each other to have a thickness of about 4 mm.

An absorptive element 22a adapted to absorb an anionic substance, an anion electrode arrangement for detecting an anionic substance, a cation-absorptive element 22b adapted to absorb a cationic substance, a cation electrode arrangement for detecting a cationic substance, are disposed between the base plates 12, 13. The anion electrode array includes two working electrodes 31, 32, a counter electrode 33 corresponding to the working electrodes 31, 32 and a reference electrode 34, and the cation electrode array includes two working electrodes 35, 36, a reference electrode 37 and a counter electrode 38. The base plate 12 is formed with a plurality of concave portions for receiving therein these electrodes at respective predetermined positions. Specifically, as shown in FIG 1, two concave portions 31a, 32a are formed as two working electrode chambers for receiving therein the respective working electrodes 31, 32. Further, a concave portion 33a is formed as a counter electrode chamber for receiving therein the counter electrode 33, and a concave portion 34a is formed as a reference electrode chamber for receiving therein the reference electrode 34. In the same manner, two concave portions 35a, 36a, a concave portion 37a and a concave portion 38a are formed, respectively, as two working electrode chambers, a reference electrode chamber and a counter electrode chamber. Each of the absorptive elements 22a, 22b serves as an storing section for temporarily storing a detection target substance, i.e., target substance.

Generally, a gold electrode (e.g., size: 3.5 mm × 8.4 mm × 0.5 mm) having a gold layer formed on a glass substrate is used as a working electrode for a measurement of arsenic and selenium (arsenic/selenium measurement), and a plate-shaped carbon electrode (e.g., size: 3.5 mm × 8.4 mm × 0.5 mm) is used as a working electrode for a measurement of cadmium, lead, mercury and hexavalent chromium (cadmium/lead/mercury/hexavalent-chromium measurement). In this embodiment, the working electrode 31 may be composed of a gold electrode for an arsenic/selenium measurement, and each of the working electrodes 32, 35, 36 may be composed of a plate-shaped carbon electrode for a cadmium/lead/mercury/hexavalent-chromium measurement). Each of the counter electrodes 33, 38 may be composed of the same plate-shaped carbon electrode (e.g., size: 3.5 mm × 8.4 mm × 0.5 mm) as that used as the working electrode 32, and each of the reference electrodes 34, 35 may be composed of an electrode (e.g., size: 3.5 mm × 8.4 mm × 0.5 mm) having an alumina substrate coated with a silver paste (6022 available from Acheson (Japan) Ltd.). It is understood that each of the electrodes may be formed in any other suitable configuration.

Each of the electrodes 31, 32, 33, 34, 35, 36, 37, 38 is formed to have a common size, and received in a corresponding one of the concave portions 31a, 32a, 33a, 34a, 35a, 36a, 37a, 38a in such a manner that an upper surface of the electrode becomes flush with an upper surface of the base plate 12. Three adhesive tapes are interposed, respectively, between the base plates 11, 12, between the base plates 12, 13 and between the base plates 13, 14, so as to allow the adjacent base plates to be liquid-tightly fixed together. In FIG 1, although only one adhesive tape 24 interposed between the base plates 12, 13 is illustrated, each of the remaining adhesive tapes interposed between the other base plates has the same configuration. Each of the adhesive tapes is formed with a communication-hole or through-hole at a desired position.

The respective upper surfaces of all the electrodes including the working electrodes 31, 32, 35, 36 are masked by the adhesive tape 24. As shown in FIG 1, the mask 24 is formed with eight through-holes at positions corresponding to the respective electrodes, to allow the electrodes to exposed therethrough. Specifically, the reference numerals 241, 242 in FIG 1 indicate two through-holes corresponding to the respective working electrodes 31, 32 in the cation electrode array for detecting a cationic substance. The through-holes corresponding to respective electrodes in the cation electrode array for detecting a cationic substance are formed in the same manner. In the illustrated embodiment, each of the through-hole 241 corresponding to the working electrode 31 and the through-hole corresponding to the working electrode 35 is formed as a circular-shaped through-hole having a diameter of 1 mm, and each of the through-holes corresponding to the respective remaining electrodes including the working electrodes 32, 36 is formed as a circular-shaped through-hole having a diameter of 2 mm. These through-holes are provided as a means to allow the electrodes 31 to 38 to come into contact with a liquid. Further, the adhesive tape 24 has two through-holes 243, 244 which are formed at positions corresponding to the respective absorptive elements 22a, 22b to have the same size of the absorptive elements 22a, 22b. Although not described individually, each of the three adhesive tapes including the adhesive tape 24 is formed with other communication-hole or through-hole at a position corresponding to other element, such as other concave portion, formed in each of the base plates.

As shown in FIG 1, the base plate 11 is formed with a concave portion defining a waste liquid reservoir 110, a first pair of passage grooves 111 defining a part of a liquid passage, and a second pair of passage grooves 112 defining a part of the liquid passage. The base plate 12 is formed with a pair of through-holes 201 for feeding a sample liquid therethrough. Each of the through-holes 201 is formed at a position where, when the base plate 12 is superimposed on the base plate 11, each of the through-holes 201 comes into liquid communication with one end (i.e., first end) of a corresponding one of the first pair of passage grooves 111. In this state, the other end (i.e., second end) of each of the first pair of passage grooves 111 comes into liquid communication with an after-mentioned passage groove of the base plate 14 via respective through-holes formed in the base plate 12 and the base plate 13. Each of the second pair of passage grooves 112 has one end (i.e., first end) in liquid communication with a corresponding one of the absorptive element 22a, 22b. The base plate 12 further has a communication-hole 202 formed at a position overlapping the waste liquid reservoir 110 of the base plate 11

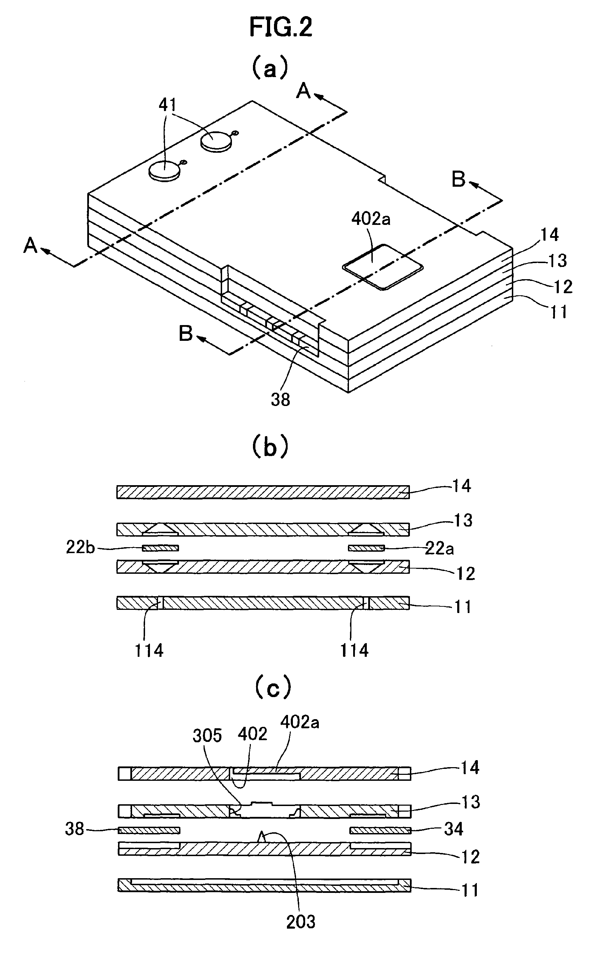

The base plate 13 is formed with a pair of through-holes 301 and a pair of concave portions 302 in such a manner that, when the base plate 13 is superimposed on the base plate 12, each of the through-holes 301 overlaps a corresponding one of the through-holes 201 of the base plate 12, and each of the concave portions 302 receives therein an upper portion of a corresponding one of the absorptive elements 22a, 22b. The base plate 13 further has a passage groove 303 formed at a position overlapping the anion electrode array (31, 32, 33, 34), and a passage groove 304 formed at a position overlapping the cation electrode array (35, 36, 37, 38). Furthermore, the base plate 13 is formed with an electrolyte-liquid chamber 305 for receiving therein an electrolyte-liquid pack containing an electrolyte liquid as a reference-electrode activating liquid, and a communication-hole 306 in liquid communication with each of the electrolyte-liquid chamber 305 and the communication-hole 202 of the base plate 12. The base plate 12 is provided with a needle-shaped member 203 protruding inside the electrolyte-liquid chamber 305 of the base plate 13. The base plate 14 is formed with a pair of through-holes 401 in liquid communication with the respective through-holes 301 of the base plate 13, and a communication-hole 402 in liquid communication with the electrolyte-liquid chamber 305 of the base plate 13. The base plate 14 is integrally formed with a flexible thin plate portion 402a on the side of an outer surface thereof in such a manner as to close the communication-hole 402 (see FIGS. 2(a) and 2(b)). An electrolyte liquid serving as a reference-electrode activating liquid is supplied in a state after being contained in an aluminum pack, and this electrolyte-liquid pack is set in the electrolyte-liquid chamber 305. The electrolyte-liquid chamber 305 is in liquid communication with the reference electrode chambers.

The base plate 14 is formed with a pair of passage grooves 403, and each of the passage grooves 403 has one end (i.e., first end) in liquid communication with a corresponding one of the second ends of the passage grooves 111 of the base plate 11 via the through-holes of the base plates 12, 13. As see in FIG 1, each of the anion electrode array (31, 32, 33, 34) and the cation electrode array (35, 36, 37, 38) is exposed to a corresponding one of opposite side edges of the base plate 12. The through-holes 201, 301, 401 collectively make up a sample-liquid inlet 1a.

FIG 2(a) shows the detection cartridge in a state after being assembled by superimposing the base plates 11, 12, 13, 14 in FIG 1 on each other. FIG 2(b) is a sectional view taken along the line A-A in FIG 2(a), which shows a section passing through the absorptive elements, and FIG 2(c) is a sectional view taken along the line B-B in FIG 2(a), which shows a section passing through the electrolyte-liquid chamber 305 for the reference electrodes.

FIG 3 schematically shows the liquid passage in the detection cartridge, wherein an upstream side and a downstream side of the liquid flow are located, respectively, on a left side and a right side of the drawing. Each reference numeral or code in FIG 3 corresponds to that in FIG 1. The following description will be made about a portion of the liquid passage for an arsenic/selenium measurement, which includes the anion-absorptive element 22a, as one example. The absorptive element 22a is formed as a circular-shaped membrane having a diameter of 5 mm and a thickness of 600 $\mu$m, and held in such a manner that an periphery of the absorptive element 22a is clamped between respective edges of opposed absorptive element receiving concave portions 21a of the base plates 12, 13. The edges of the absorptive element receiving concave portions 21a are formed to prevent a liquid from leaking along the periphery of the absorptive element 22a. Further, each of these concave portions is formed in a taper shape to prevent a passage wall from suddenly having a step in a liquid flow direction. This makes it possible to form a smooth liquid flow in the liquid flow direction and suppress the generation of gas bubbles. As shown in FIG 3, the base plate 11 has an outer surface formed with an external-connection port 113 connected to the passage groove 111, an external-connection port 114 connected to the passage groove 112 at a position adjacent to the absorptive element receiving concave portion 21a, and an external-connection port 115 connected to the passage groove 112 at a position adjacent to a downstream end of the passage groove 112. The outer surface of the base plate 11 is also formed with an external-connection port 116 connected to the passage groove 304 of the base plate 13 via a through-hole of the base plate 12. Each of the external-connection ports 113, 114, 115, 116 is controlled by a valve mechanism which will be described later and adapted to switch the respective ports in a desired manner. FIG 3 shows a part of a 5-way valve mechanism for the switching control. This valve mechanism will be specifically described later with reference to FIG 4.

In an operation of detecting an target substance using the detection cartridge according to this embodiment, the external-connection ports 113, 114, 116, 117 are closed, and the port 115 is opened. Then, with assistance of a syringe holder, a sample liquid containing an target substance is injected into the sample-liquid inlet portion 1a of the detection cartridge consisting of the through-holes 401, 301, 201, together with an anion-absorptive element activating liquid according to need. FIG 3(a) shows a flow route of the sample liquid in this operation. The sample liquid injected from the sample-liquid inlet portion 1a of the detection cartridge, i.e., the through-holes 401, 301, 201, using a syringe, is passed through the liquid passage zone defined by the groove 111 and the through-holes of the base plates 12, 13 vertically upwardly. Then, the sample liquid flows through a liquid passage zone defined by the groove 403 of the base plate 14, and will reach the absorptive element 22a. The target substance contained in the liquid sample is absorbed to the absorptive element 22a, and the sample liquid after being passed across the absorptive element 22a is discharged from the external-connection port 115 which is set in the open state by valve mechanism. FIG 3(b) illustrates one example of the syringe holder for use in operating a syringe. The holder includes two clamp members 15, 16 designed to strongly clamp the detection cartridge 1 therebetween. The clamp member 15 has a structure capable of liquid-tightly close an unused one of the through-holes 40 of the sample-liquid inlet portion. The clamp member 16 is formed with a communication-hole for allowing the syringe 17 to be fitted therein.

An electrolyte liquid for activating the reference electrode 34 is supplied to the electrolyte-liquid chamber 305 from an external-connection port 117. Then, the valve mechanism is shifted to close the external-connection port 115, and open the external-connection port 113 to provide liquid communication between an after-mentioned eluent-liquid supply section and the external-connection port 113.

FIG 3(c) shows a flow route of an eluent liquid. An eluent liquid sent from the after-mentioned eluent-liquid supply section reaches the absorptive element 22a. Then, the eluent liquid after being passed across the absorptive element 22a flows along the liquid passage zone defined by the groove 112. During this process, the target substance absorbed to the absorptive element 22a is eluted in the eluent liquid. The eluent liquid containing the target substance flows along the respective upper surfaces of the anion detection electrodes 31, 32, 33, 34 through the liquid passage zone of the groove 112 and a liquid passage zone defined by the groove 303 of the base plate 13. The target substance-containing eluent liquid flowing along the upper surfaces of the anion electrode array causes the anion electrode array to generate an electric signal indicative of information about a concentration of the target substance.

FIG 4 shows the structure of a processing unit 2. The processing unit 2 comprises a processing device 21 and an eluent-liquid supply section 22 which are housed in a casing 20 thereof. The eluent-liquid supply section 22 includes an eluent-liquid-tank cassette 50 storing a plurality (in this embodiment, three) of eluent liquid tanks 54, 55, 56, a valve mechanism 51 for switching each of the external-connection ports 113, 114, 115, 116 of the base plate 11, and a pump 52. The eluent-liquid-tank cassette 50 is designed to be inserted into the casing 20, and the valve mechanism 51 and the pump 52 are actually housed in the casing 20 although they are shown outside the casing 20 in FIG 4 for the sake of illustrative convenience. The valve mechanism 51 includes a 5-way valve 53a and a 4-way valve 53b. The 5-way valve 53a is connected to each of the eluent-tanks 54, 55, 56 via the pump 52 and the 4-way valve 53b. The 5-way valve 53a is connected to each of the outlet-side external-connection ports 115, 116, 117. Although not illustrated in FIG 3 which shows only the external-connection ports for detecting an anionic substance, the base plate 11 is further formed with three similar external-connection ports for detecting a cationic substance, and the 5-way valve is also operable to switch these external-connection ports. The entry-side external-connection port 113 as an eluent-liquid entry port is used for both the anionic-substance detection and the cationic-substance detection.

The 4-way valve 53b is provided as a means to switch liquid communication between each of the eluent liquid tanks 54, 55, 56 and the pump 52. The eluent liquid tanks 54, 55, 56 contain an eluent liquid for the anion-absorptive element, an eluent liquid for the cation-absorptive element (this eluent liquid is also used as an electrolyte liquid), and a cleaning liquid, such as water, respectively, and the 4-way valve 53b is operable to selectively sent one of these liquid to the pump 52.

The eluent-liquid-tank cassette 50 storing the eluent liquid tanks is designed to be detachably attached to the casing 20 of the processing unit 2. Thus, if a remaining volume of the liquid contained in one of the liquid tanks runs low, the liquid tank can be detached to supply the liquid thereto. As seen in the eluent liquid tank 56 in FIG 4, each of the liquid tanks has a detachable cap 57, and a rubber ring capable of fully sealing between a tank body and the cap 57 to prevent leakage of the eluent. Further, the cap 57 has an upper portion with a connector-like structure capable of being connected to a cover 58 of the eluent-liquid-tank cassette 50 through one-touch simple operation.

The processing device 21 housed in the casing 20 of the processing unit 2 includes an electronic board 66 mounting a microprocessor and various drivers, and has a top surface 67 provided with a display section and a user interface, such as a plurality of manual operation buttons. Further, the processing unit has an outer surface partially formed as a detection insertion case 62. This insertion case 62 is formed as a hinged lid, and designed to be selectively opened and closed relative to a cartridge holder 61 which allows the detection cartridge to be fitted thereinto. The cartridge holder 61 is designed to be selectively opened and closed.

FIG 4(a) is a schematic diagram showing the cartridge holder 61 in its open state, and FIG 4(b) is a schematic diagram showing the cartridge holder 61 in its closed state. The cartridge holder 61 has a structure as shown in FIG 4(c). Specifically, the cartridge holder 61 comprises an upper plate 613, a lower structure composed of a lower plate 612 and connected with the 5-way valve 53a and various liquid supply tubes, and a presser plate 614 movably coupled to the lower structure through a flange 611. As seen in FIG 4(c) which illustrates the presser plate 614 in an upside down manner, the presser plate 614 has a bottom surface provided with two arrays of electrode-contact pins 615, and a top surface having a wiring 616 connected to the pins 615, so that electric signals are exchanged between the pin array and the microprocessor of the processing unit. The two pin arrays 615 are arranged to correspond to the anion electrode array (31, 32, 33, 34) the cation electrode array (35, 36, 37, 38), respectively.

The bottom surface of the presser plate 614 is provided with a pair of resilient sealing members 411 adapted to be engaged with the respective through-holes 401 of the sample-liquid inlet portion 1a of the detection cartridge so as to prevent back-flow of the sample liquid, a protrusion 141 adapted to be inserted into the electrolyte-liquid chamber 305 so as to allow the reference-electrode activating liquid to be directed toward a desired one of the reference electrodes, and a plurality of resilient protrusions 412 adapted to press the detection cartridge 1 against the upper plate 613 so as to prevent leakage of the various liquids. The detection cartridge 1 is inserted into the cartridge holder 61 in a posture as shown in FIG 4(a).

In the example illustrated in FIG 3, the groove 304 facing the anion electrode array has a depth of 200 $\mu$m, and a width of 3 mm. The depth of the groove 304 is reduced in a liquid passage zone between the counter electrode 33 and the reference electrode 34 to form a liquid junction. This liquid junction has a depth of 100 $\mu$m, and a width of about 100 $\mu$m. The remaining liquid passage has a depth of 500 $\mu$m, and a width of 500 $\mu$m. The through-hole 401 of the sample-liquid inlet portion 1a has a size capable of conformably receiving therein a distal end of a syringe when it is used for injecting a sample liquid, and allowing the through-hole 401 to be closed after being set in the processing unit, without defining any liquid flow. A volume of the liquid passage in the detection cartridge, except the through-hole 401 of the sample-liquid inlet portion 1a, the waste liquid reservoir 110 and the communication-hole 202, is about 1 ml. The volume of each of two detection passage lines routed, respectively, through the anion electrode array and the cation electrode array, except the waste liquid tank 123 is 45 $\mu$l.

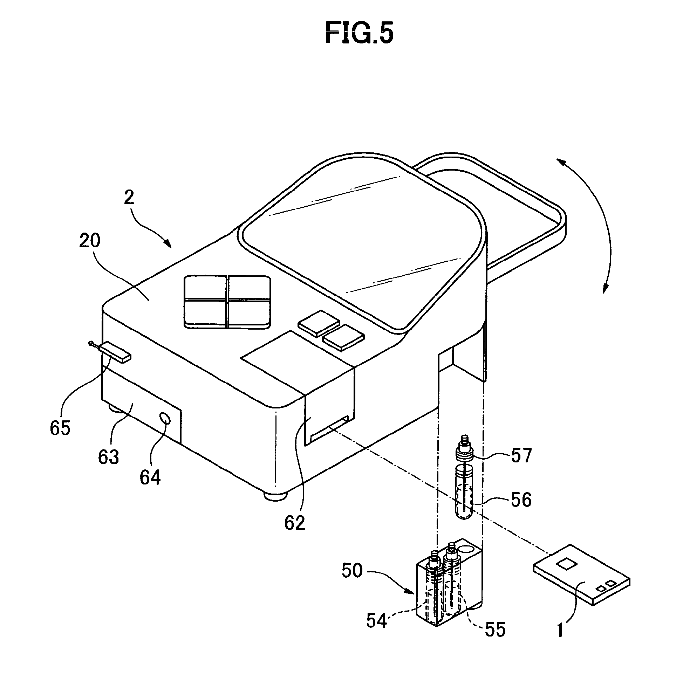

As shown in FIG 5, after opening the cartridge insertion case 62, the detection cartridge 1 can be loaded or unloaded into/from the processing unit through a lateral surface thereof as indicated by the one-dot chain line. The eluent-liquid-tank cassette 50 storing the liquid tanks 54, 55, 56 can be demounted from the casing of the processing unit as needed to facilitate filling-up and exchange of the liquids. The processing unit 2 includes a battery 63 capable of operating without being connected to a power cord, and a communication device 65 capable of wirelessly communicating with the outside.

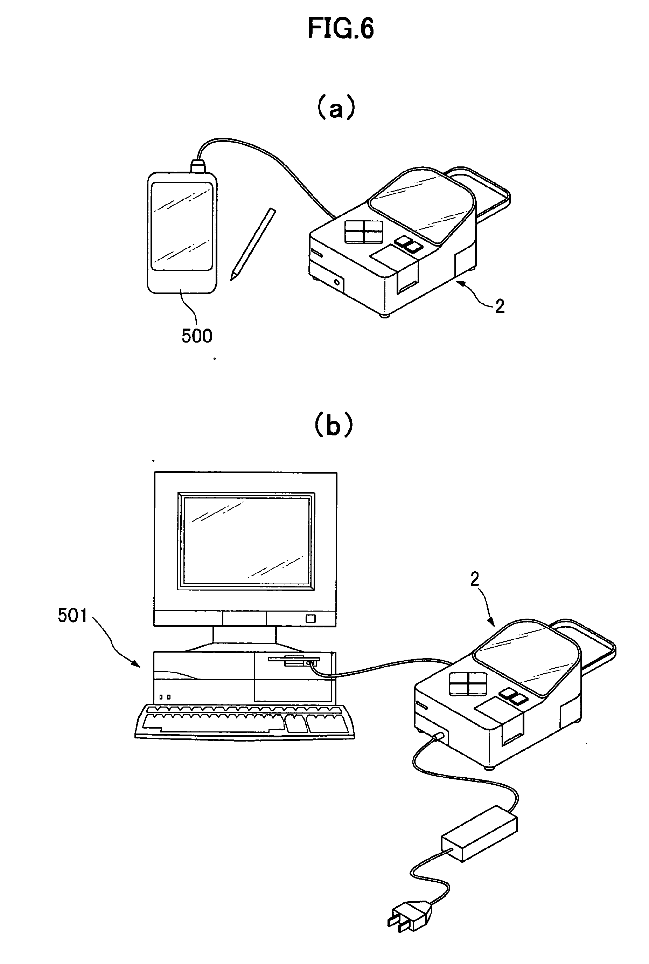

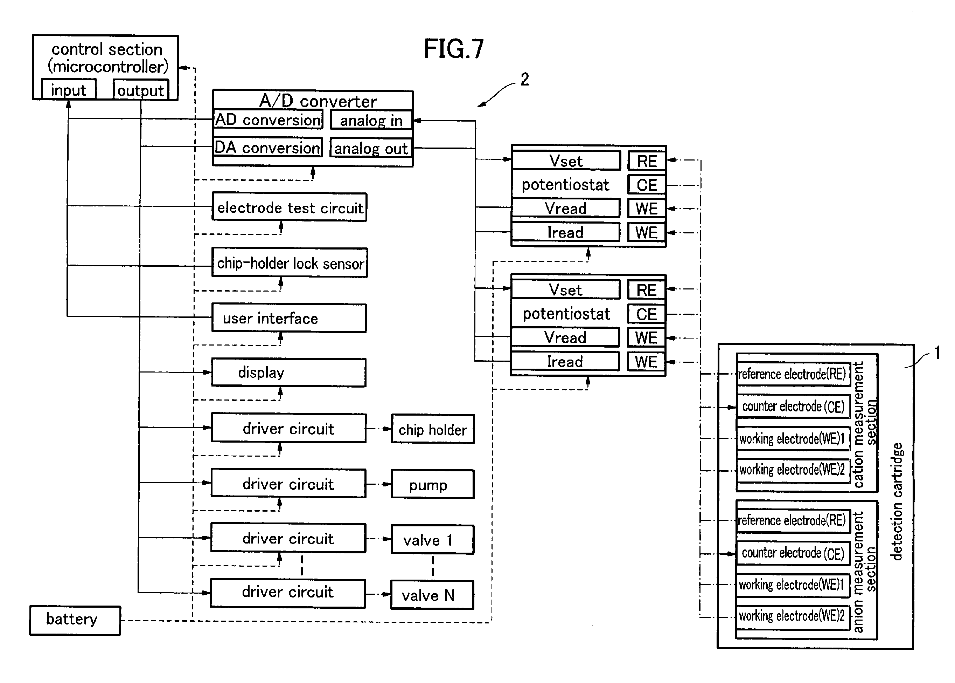

The processing unit 2 may be designed to be connected to a personal digital assistant (PDA) as shown in FIG 6(a), or to a computer 501 as shown in FIG 6(b), to facilitate recording and/or transfer of analysis data. Further, the processing unit 2 is may be designed to be usable in both a battery-powered mode suitable for mobile measurements, and an AC-powered mode suitable for stationary measurements. FIG 7 is a block diagram showing the configuration of a processing system in the procession unit. As shown in FIG 7, the processing unit 2 comprised a control section composed of a microcontroller, and various elements including an A/D converter, as indicated in each block. Each of these elements may be configured using commonly known techniques, and its detailed description will be omitted.

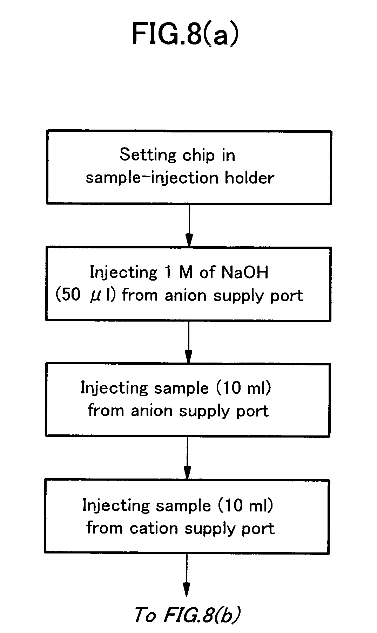

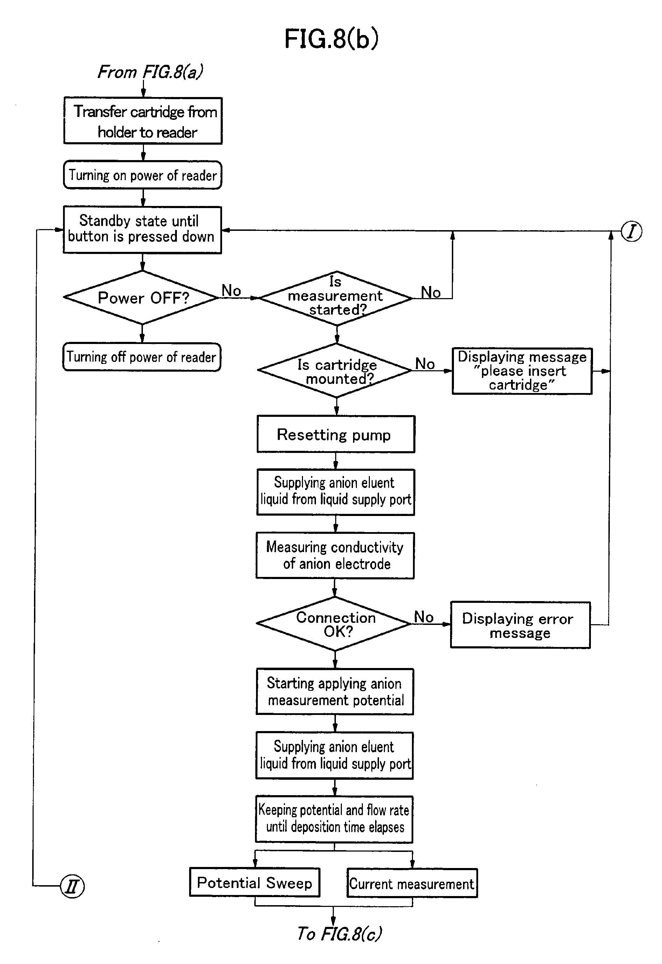

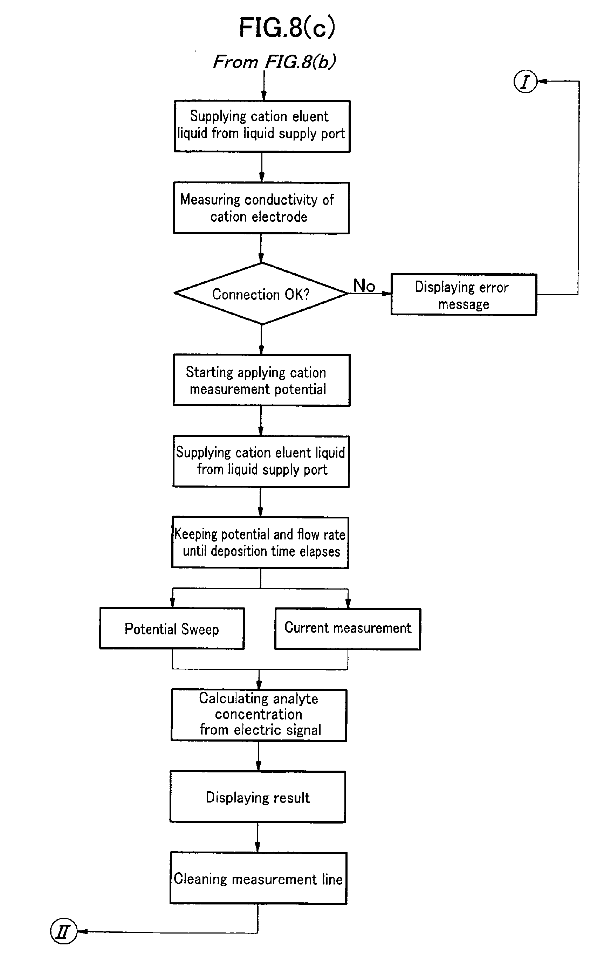

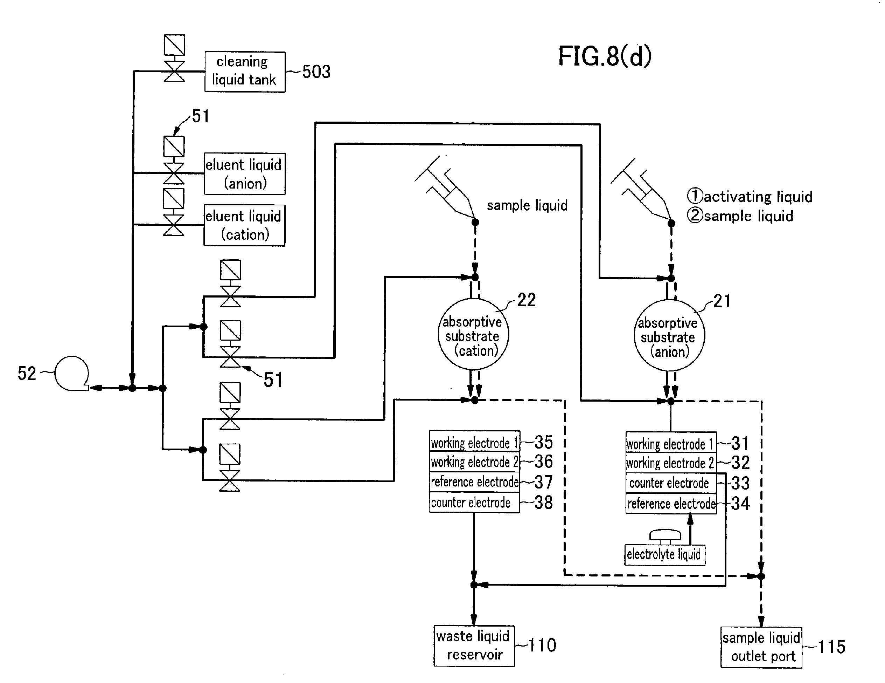

FIGS. 8(a) to 8(c) show flowcharts of a process of detecting an target substance using the detection cartridge 1 and the processing unit 2 illustrated in FIGS. 1 to 7. Referring to FIGS. 8(a) to 8(c), a measurement process will be described below while taking some examples.

In a first stage of the measurement process, the detection cartridge 1 is held by the clamp members 15, 16 of the syringe holder without being inserted into the processing unit 2. The syringe holder is designed to close the through-holes 401 of the sample-liquid inlet portion 1a when it holds the detection cartridge 1. In place of the syringe holder illustrated in FIG 3(b), a hole plug or cap 41 as shown in FIG 2(a) or a valve may be used. The reason for closing the through-hole 401 is to allow an eluent liquid after being supplied from the port 113 and passed across the absorptive element 22a, to be smoothly discharged from the port 115.

Firstly, in order to activate an absorption ability of the anion-absorptive element 22a, an anion-absorptive element activating liquid is injected from the through-hole 401 of the sample-liquid inlet portion 1a. A volume of the activating liquid may be set at a value allowing the anion-absorptive element 22a to be just wetted. Specifically, about 50 $\mu$l of the activating liquid may be all that is needed. Then, 10 ml of the sample liquid is injected from the through-hole 401 of the sample-liquid inlet portion 1a, and discharged from the external-connection port 115 after being passed through the anion-absorptive element 22a. During this process, arsenic and selenium as target substances are absorbed and captured by the anion-absorptive element 22a. Thus, no target substance is contained in the sample liquid when it is discharged. Then, in order to fill the reference electrode chamber with a potassium chloride solution as a reference-electrode activating liquid for creating silver chloride in the reference electrode 34, a pack containing the activating liquid is set in the electrolyte-liquid chamber 305 of the base plate 13, and a pressing portion, i.e., the flexible thin plate portion 402a (see FIGS. 1 and 2(a)) of the base plate 14, is pressed down. Thus, the activating liquid-containing pack is broken by the needle-shaped member 203 provided in the base plate 12 at a position corresponding to the pack, and the potassium chloride solution leaking from the pack flows into the reference electrode chamber which houses the reference electrode 34. The potassium chloride solution functions to form a silver/silver-chloride electrode on a surface of the reference electrode.

In a second stage of the measurement process, the detection cartridge 1 is insertingly loaded into the processing unit 2. In response to inserting the detection cartridge 1 into the processing unit 2, the valve mechanism 51 is operated to supply potassium chloride from the port 117 to the reference electrode chamber in the anion electrode array. Then, the eluent-liquid supply port 113 is opened to supply an eluent liquid therethrough. Simultaneously, the through-holes 401 of the sample-liquid inlet portion 1a are closed, and each of the electrode-contact pins 615 is brought into contact with a corresponding one of the working electrodes 31, 32, the counter electrode 33 and the reference electrode 34.

Then, when a measurement start button on the processing unit 2 is pressed down to initiate the measurement, an arsenic/selenium measurement eluent liquid (1 M of sulfuric acid; pH = about 2) is supplied from the port 113 located adjacent to the sample-liquid inlet portion. The eluent liquid flows across the absorptive element 22a, and then flows along the electrodes 31, 32, 33, 34. During this process, the through-holes 401 are closed, and therefore there is no risk of back-flow of the eluent liquid toward the sample-liquid inlet portion. Even though the liquid passage zone facing the electrodes 31, 32, 33 is continuous with the liquid passage zone facing to the reference electrode 34 via the liquid junction 133, the extremely-narrowed liquid junction 133 can prevent the potassium chloride solution in the reference electrode chamber to flow back toward the array of the electrodes 31, 32, 33. A downstreammost end of this liquid passage is connected to a waste liquid reservoir of the processing unit via the port 116.

Although the liquid passage zone facing the electrodes 31, 32, 33, the liquid junction 133, and the liquid passage zone facing the reference electrode 34 are illustrated in FIG 3 as if they are connected in series to the port 116 for the sake of simplicity, they are actually arranged as illustrated in FIG 1. That is, the liquid passage zone facing the reference electrode 34 is connected, through the liquid junction 133, to an intermediate position of a liquid flow passage zone extending from the liquid passage zone facing the electrodes 31, 32, 33 to the port 116. Thus, while the liquid passage zone facing the electrodes 31, 32, 33 is in liquid communication with the liquid passage zone facing the reference electrode 34, the potassium chloride solution on the side of the reference electrode 34 is never mixed with the eluent liquid.

A state of electrical connection is checked up after the liquid passage zone facing the electrodes 31, 32, 33 is filled with the eluent liquid. Specifically, a voltage or current in each of the working electrodes 31, 32 is checked up while applying a certain current or voltage between the working electrode and the counter electrode 33, to determine whether the eluent liquid is sufficiently supplied to ensure adequate electrical connection, and whether the contact pins are adequately in contact with the respective electrodes. A volume of the eluent liquid required for filling the electrode-facing liquid passage zone therewith is about 40 µl.

Then, a voltage (- 0.4 V) is applied to each of the working electrodes 31, 32 while supplying the arsenic/selenium measurement eluent liquid from the eluent-liquid supply port 113 at a constant flow rate, so as to deposit arsenic and selenium on the working electrodes 31, 32. The arsenic and selenium captured by the absorptive element 22a is eluted by the eluent liquid passed across the absorptive element, and mixed into the eluent liquid. Then, the arsenic and selenium reach around the electrodes together with the eluent liquid. The arsenic and selenium are deposited on the working electrodes 31, 32 through a reduction reaction occurring around the electrodes. The supply of the eluent liquid and the application of the deposition voltage will be continued until the absorbed arsenic and selenium are entirely desorbed. For example, when 300 µl of arsenic and selenium is deposited at a flow rate of 50 µl/min, the absorbed arsenic and selenium is almost fully eluted. Thus, a deposition time may be set at about 6 minutes. In a specific operation, the supply of the eluent liquid is continued for 5 minutes 50 seconds, and the last 10 seconds are used for stabilizing the eluent liquid in the liquid passage. Then, after an elapse of 6 minutes, a potential sweep operation is initiated. The potential sweep may be performed under the following conditions:

Conditions of Anodic Stripping Voltammetry (ASV)
Sweep System: LSV (Linear Sweep Voltammetry: potential sweep without applying a constant frequency)
Deposition potential: - 0.4 V
Deposition time: 6 minutes
Sweep rate: 0.2 V/s
Onset sweep potential: - 0.4 V
Termination sweep end potential: 1.2 V

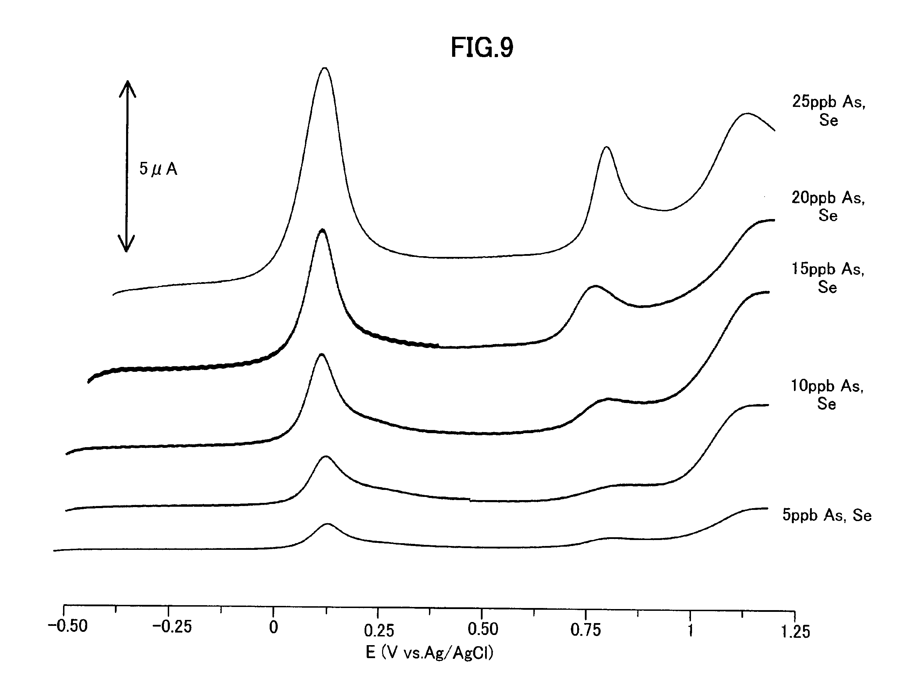

A potential-current curve obtained by the above operation is recorded. For example, a graph as shown in FIG 9 can be obtained-type on the record data. A peak area observed in this graph is a current which flowed at a time when the heavy metals deposited on the working electrode within the deposition time was re-dissolved due to an increase in potential, and corresponds to an amount of dissolved substance. In FIG 9, a potential-current curve as the result of the above measurement is shown in place of a concentration of arsenic and selenium.

The measurement process for arsenic and selenium is completed through the above operation. Successively, a measurement of cadmium, lead and mercury is initiated. Except for the following points, the cadmium/lead/mercury measurement is performed in the same manner as that in the selenium measurement. In the cation measurement, the mixing of potassium chloride ions into an eluent liquid is not a factor disturbing the measurement. Thus, a common liquid can be used as both the reference-electrode activating liquid and the eluent liquid. That is, there is no need for arranging the reference electrode-facing liquid passage zone independently of the liquid passage zone facing other electrodes, and the four electrodes 35, 36, 37, 38 may be arranged in series. In FIG 1, as the cation electrode array, the four electrodes are arranged to face a single liquid passage zone. In this case, the eluent liquid may have the following composition.

Cation Measurement Eluent Liquid

0.4 M of potassium chloride + 10 mM of citric acid + 3.5 mM of ethyl diamine (pH = about 4)
The cation-absorptive element 22b has no need for an activating liquid. Thus, an operation corresponding to the above first stage of measurement process may be completed only by injecting a sample liquid.
As shown in the flowchart in FIG 8(a) and (8c), the above control is performed by the processing device of the processing unit 2 according to a program.

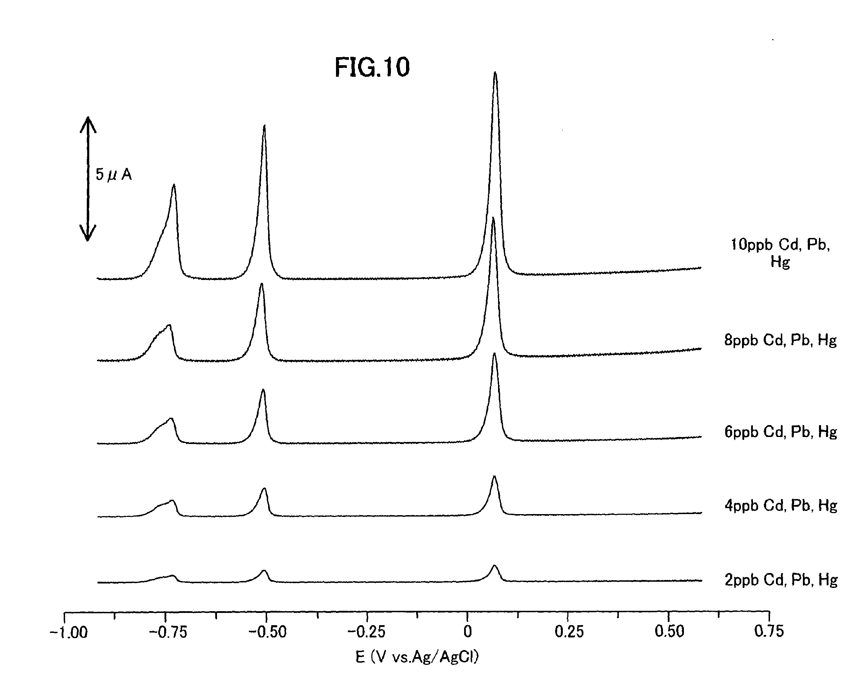

FIG 10 shows a potential-current curve obtained by a measurement performed in the same manner as that in the anion measurement except the above points. As seen in FIG 10, a sharp peak is obtained for each of cadmium, lead and mercury, and a different peak area appears depending on concentrations of the metals. A quantitative analysis can be performed-type on the obtained analytical curve. Conditions of potential sweep are as follows:

Conditions of ASV
Sweep System: SWV (Square Wave Voltammetry: potential sweep while applying a constant frequency)
Deposition potential: - 0.9 V
Deposition time: 6 minutes
Sweep rate: 0.225 V/s
Onset sweep potential: - 0.9 V
Termination sweep end potential: 0.6 V
Frequency: 100 Hz
Step potential: 2.55 mV

In case of successively performing the arsenic/selenium measurement and the cadmium/lead/mercury measurement, after setting the detection cartridge in the syringe holder, an absorptive element activating liquid is injected from the through-hole 401 of the arsenic/selenium sample-liquid inlet portion 1a. Then, 10 ml of sample liquid is injected into each of the through-hole 401 of the arsenic/selenium sample-liquid inlet portion 1a, and the through-hole 401 of the cadmium/lead/mercury sample-liquid inlet portion 1a. Then, the detection cartridge 1 is insertingly loaded into the processing unit 2. In response to pressing down the start button, respective operations of supplying an arsenic/selenium-measurement eluent liquid, checking up electrical connection, re-supplying the eluent liquid and carrying out an electrochemical measurement are sequentially performed. Subsequently, respective operations of supplying a cadmium/lead/mercury-measurement eluent liquid, checking up electrical connection, re-supplying the eluent liquid and carrying out an electrochemical measurement are sequentially performed. After completion of the cadmium/lead/mercury measurement, a cleaning liquid is supplied from the cleaning liquid tank 503 to clean the valve mechanism 51, the pump 52 and a plurality of lines connecting therebetween. FIG 8(d) generally shows this system. A volume of cleaning liquid capable of just washing away the liquid in the valve mechanism 51, the pump 52 and the lines is supplied from the cleaning water tank 503 while switching the lines in turn. A total volume of the cleaning liquid including the cleaning liquid in the valve mechanism 51, the pump 52 and the lines is about 600 $\mu$l. Thus, the eluent liquid residing in the detection cartridge is washed away in a volume equivalent to about 600 $\mu$l of the cleaning liquid, and reserved in the waste liquid reservoir 110. Except that the electrochemical measurement is carried out-type on cathodic stripping voltammetry (CSV), a hexavalent-chromium measurement is performed in the same manner as that in the arsenic/selenium measurement. Due to the difference in electrochemical measurement scheme between the hexavalent-chromium measurement and the arsenic/selenium measurement, the hexavalent-chromium measurement and the arsenic/selenium measurement are performed using individual detection cartridges instead of the simultaneous measurement. In contrast, the cadmium/lead/mercury measurement may be performed just after completion of the hexavalent-chromium measurement, using the same detection cartridge.

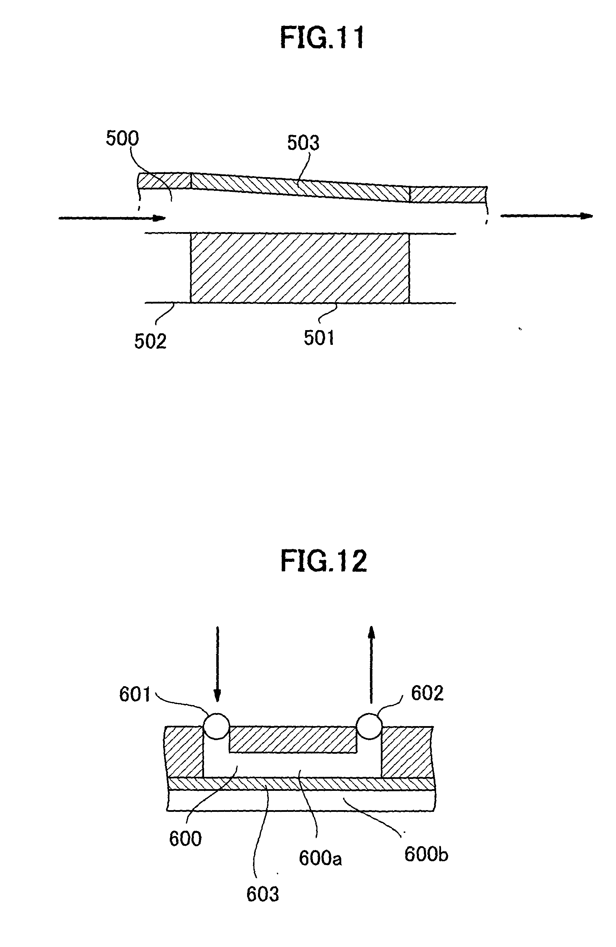

FIG 11 is a schematic sectional view showing one example of modification of the storing section in the detection cartridge of the present invention. In this modification, the storing section is formed as a sample-liquid enrichment section adapted to enrich a sample liquid by means of heating/evaporation. Specifically, a heating element 501, such as a Peltier element, is disposed to face a liquid passage 500 defined in the detection cartridge, and a current is supplied to the heating element 501 through a conductive wire 502. A film or membrane 503 made of a vapor-permeable transparent material is disposed on a sidewall of the liquid passage 500 opposed to the heating element 501, and thereby vapor generated by heating is released through the membrane 503 to enrich a sample liquid.

FIG 12 shows another example of the sample-liquid enrichment section. In this example, a porous membrane is used. A liquid passage 600 has a sample-liquid inlet provided with a valve 601, and a sample-liquid outlet provided with a valve 602. Further, a porous membrane 603 is disposed to separate the liquid passage between an enrichment chamber 600a and a drain chamber 600b.

In the above modification, a sample liquid is fed from the inlet valve 601 after closing the outlet valve 602, and an appropriate pressure is applied to the enrichment chamber 600a so as to enrich the sample liquid in the enrichment chamber 600a. After the enrichment, the outlet port 602 is opened to supply the enriched sample liquid to an electrode arrangement. This modification has an advantage of being able to reduce an enrichment time by freely increasing an area of the porous membrane 603.

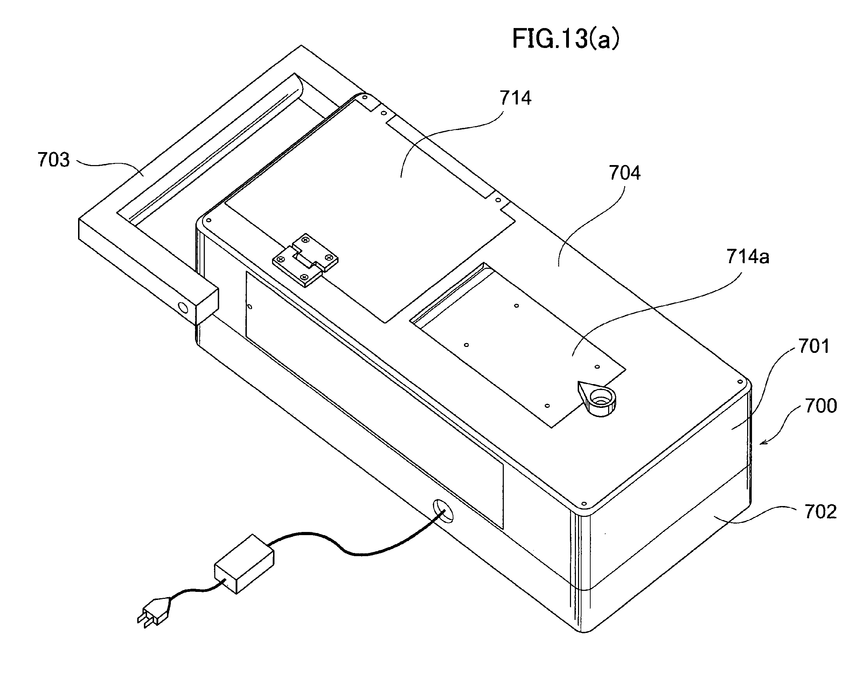

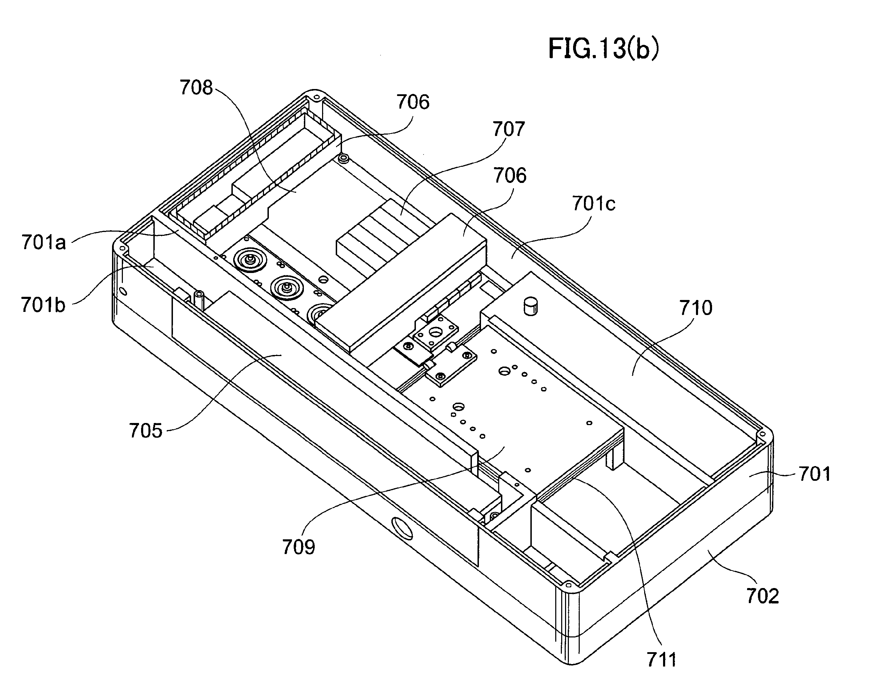

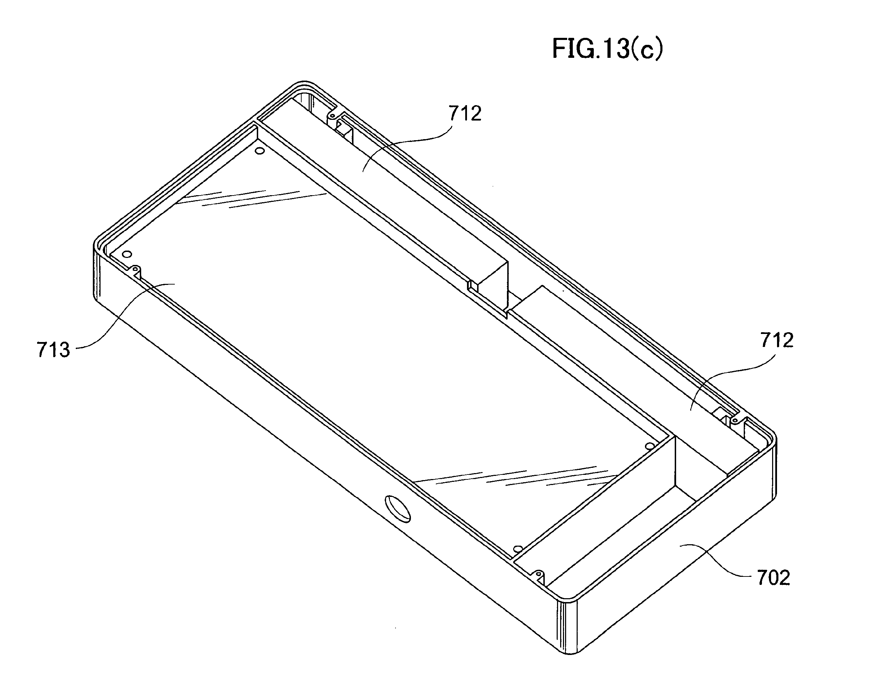

FIGS. 13(a), 13(b) and 13(c) are perspective views showing a portable analysis unit usable with the aforementioned detection cartridge, according to one embodiment of the present invention. As shown in FIG 13(a), the analysis unit comprises an analyzer body (i.e., housing) 700 generally having a rectangular parallelepiped shape. This analyzer body 700 has an upper body 701 and a lower body 702 which are vertically arranged in a superimposed manner. The analyzer body 700 is provided with a handle 703 for conveniently providing portability. The upper body 702 is opened upwardly, and a cover 704 is fixed to form a top wall thereof.

FIG 13(b) shows an internal structure of the upper body after the cover 704 is detached. The upper body 701 internally has a waste-liquid-tank chamber 701b defined by a partition wall 701a to extend along one longitudinally-extending lateral wall, and a waste liquid tank 705 is disposed in the waste-liquid-tank chamber 701b. The waste liquid tank 705 is detachably fixed to the upper body 701.

A chamber 701c for housing various functional components is provided on an opposite side of the waste-liquid-tank chamber 701b relative to the partition wall 701a, and a plurality of reagent tanks 706 is disposed in the chamber 701c over a range of from one longitudinal end wall to an approximately central region thereof and arranged in parallel relation to each other. In this embodiment, five reagent tanks are disposed therein. In FIG 14, only endmost two of the reagent tanks are illustrated for showing a structure below the reagent tanks. Further, the reagent tank 706 located adjacent to the longitudinal end wall is illustrated by cutting out an upper portion thereof to show an internal configuration thereof.

The chamber 701c houses a switching valve mechanism 707 comprising a plurality of switching valves, at a position below the reagent tanks 706, and a tank-switching line plate 708 at a position below the switching valve mechanism 707. The chamber 701c further housed a cartridge holder 709 serving as a cartridge loading portion, and a liquid feed pump 710 at a position on a lateral side of the cartridge holder 709. Furthermore, an after-mentioned destination switching valve mechanism 711 and destination-switching line plate are disposed below the cartridge holder 709.

FIG 13(c) shows an internal structure of the lower body 702. The lower body 702 internally has a battery box 712 disposed along one longitudinally-extending lateral wall, and an electronic board 713 disposed on a lateral side of the battery box 712. The electronic board 713 incorporates a required control circuit, and a processing device, such as a microprocessor.

Returning to FIG 13a, the cover 704 fixed to the top of the upper body 701 has an openable lid 714 disposed at a position corresponding to the mounting position of the reagent tanks 706 to allow each of the reagent tanks to be taken out thereof and therein, and an openable lid 714a disposed at a position corresponding to the loading position of the cartridge holder 709 and provided for a detection cartridge.

FIGS. 14(a), 14(b)-(i), 14(b)-(ii), 14(b)-(iii) and 14(c) show a detection cartridge for use in electrochemical analysis, according to another embodiment of the present invention, which correspond to FIGS. 1, 2(a), 2(b), 2(c) and 3, wherein a corresponding element or component therebetween is defined by the same reference numeral or code. In this embodiment, the detection cartridge has a bottom surface formed with a port 116 adapted to be connected to a waste liquid tank 705, and a port 117 adapted to be connected to a reagent tank for the reference-electrode activating liquid, in addition to three ports 113, 114, 115. A liquid passage between the ports 14, 15 is formed within the detection cartridge instead of forming outside the detection cartridge.

FIG 46 shows show a detection cartridge for use in electrochemical analysis, according to another embodiment of the present invention. This detection cartridge for electrochemical analysis includes a first sheet made of a resin material and a second sheet made of a resin material, which are laminated together. The first sheet is formed with an electrode-receiving concave portion, and an electrode is disposed in the concave portion. The second sheet has a liquid passage formed at a position corresponding to the electrode to allow a reagent to be passed therethrough. The detection cartridge further includes an insulation sheet interposed between the first and second sheets and formed with a hole having a predetermined area at a position corresponding to the electrode, an storing section formed at a position away from the electrode, and a third sheet disposed on either one or both of respective surfaces of the first and second sheets on opposite sides of opposed surfaces thereof. The third sheet is formed with a groove defining a liquid passage in liquid communication with the storing section. In the aforementioned detection cartridges illustrated in FIG 1 and 14(a) to 14 (c), the first sheet (base plate 12) and the second sheet (base plate 13) are laminated together, and the third sheets (the base sheets 11, 14) are disposed on both of respective surfaces of the first and second sheets on opposite sides of opposed surfaces thereof. In this embodiment, the third sheet is laminated on only one surface (upper surface in FIG 46). Further, this detection cartridge has a single detection passage line designed to be compatible with both anion and cation measurements. A sample liquid is supplied from a special holder (not shown) independent of the detection cartridge.

FIG 15(a) is a schematic diagram showing a liquid flow in a detection cartridge for chromatography analysis, and FIG 15(b) is a schematic diagram corresponding to FIG 3(c). A liquid passage 303 makes up a column for chromatography analysis. In this detection cartridge, at least a portion corresponding to the column 303 is made of a transparent plastic material, and the analysis is performed by transmitting detection light through this portion, as described in detail later.

Referring to FIGS. 16 and 17, a tank-switching line plate 708 has an upper surface formed with a plurality of reagent-tank receiving portions 715 arranged in a line. FIG 16 shows a state after a reagent tank 706 is attached to one of the receiving portions 715, and FIG 17 is a sectional view showing the receiving portion and the reagent tank during the attaching operation. The receiving portion 715 includes an annular-shaped protrusion 715a formed on the upper surface of the plate 708, and a reagent-tank-opening insertion pin 715b protruding upwardly from a center of the annular shape of the protrusion 715a. The receiving portion 715 is formed with four slits 715c each having an openings on the upper surface of the plate 708 and extending along a hypothetical arc, and an annular-shaped sealing groove 715d formed along and adjacent to an inner peripheral surface of the protrusion 715a. An O-ring 715e is installed in the sealing groove 715d.

The reagent tank 706 has a lower end formed with a convex portion 706a protruding downwardly. The convex portion 706a has a bottom surface formed with an annular-shaped groove 706b corresponding to the annular-shaped protrusion 715a of the plate 708. The reagent tank 706 also has a reagent outlet 706c formed at a position corresponding to the slits 715c of the plate 708. The outlet 706c is provided with a valve 706e biased toward its close position by a spring 706d. The reagent tank 706 has an upper surface formed with a vent hole 706f sealed by a gas permeable and liquid impermeable material.

The above reagent tank 706 is attached at a predetermined position by fitting the annular-shaped groove 706b onto the annular-shaped protrusion 715a. During this operation, the pin 715b of the plate 708 pushes the valve 706e of the reagent tank 706 upwardly to open the reagent outlet 706c so as to provide liquid communication between an internal space of the tank 706 and the slits 715c. The slits 715c are in liquid communication with a passage formed in the plate 708. The O-ring 715e functions to prevent liquid leakage between the reagent tank 706 and the plate 708.

FIG 18 specifically shows a reagent passage 708a and a pump inlet passage 708b formed in the tank-switching line plate 708, in relationship to the switching valve mechanism 707. The reagent passage 708a for each of the reagent tanks has a first end in liquid communication with the slits 715c formed in the receiving portion 715 of the plate 708, and a second end having an opening on the upper surface of the plate 708 at a position below the switching valve mechanism 707 and connected to the switching valve mechanism 707. FIG 19 shows one example of a relationship of connection between the passage 708a and a passage 707a of the switching valve mechanism 707.

FIG 18 further shows a liquid feed pump 710. While the liquid feed pump in FIG 1 is illustrated such that it is arranged to extend in the longitudinal direction of the upper body 701, the liquid feed pump 710 in FIG 18 is illustrated in a state after being rotated from the position in FIG 14 by 90 degrees for the sake of illustrative convenience. The tank-switching line plate 708 is formed with a pump inlet passage 708b. This passage 708a has one end port P1 connected to the switching valve mechanism 707, and the other end port P2 in liquid communication with an inlet (i.e., suction) port of the liquid feed pump 710. This liquid communication is achieved using a tube, as shown in FIG 20.

It is desirable that the liquid feed pump 710 is small in size and capable of stably feeding a small volume of liquid in a level of micro liter without pulsation. Further, when the liquid feed pump is used in portable analysis unit, it is required to have low power consumption. The liquid feed pump may have a flow rate of 5 to 100 micro liter/min and a discharge pressure of 0.01 to 10 MPa. A liquid feed pump meeting these requirements includes a syringe pump, such as "Pencil Pump" available from Uniflows Corp., and "Confluent" available from Scivex Inc.

Referring to FIG 18 again, it shows a destination-switching line plate 716. This plate 716 is disposed below a cartridge holder 709 and above a destination switching valve mechanism 711. As shown in FIG. 18, the destination-switching line plate 716 is formed with a pump outlet passage 716a. As shown in FIG 20, the pump outlet passage 716a has a first end in liquid communication with an outlet port of the pump 710 via a tube, and a second end connected to the destination switching valve mechanism 711.

Referring to FIG 21, it shows the detail of the cartridge holder 709. This cartridge holder 709 has the same fundamental structure as that illustrated in FIG 4(a), and comprises an upper plate 709a and a lower plate 709b which are openably and closably coupled to each other by a hinge. The lower plate 709b is formed with a concave portion 709c for fittingly receiving therein a detection cartridge. Although omitted in FIG 21, the upper plate 709a is provided with a wiring and a plurality of electrode-contact pins adapted to come into contact with respective electrodes of the detection cartridge, as with the upper plate illustrated in FIG 4(a). The upper plate 709a of the cartridge holder 709 has a rear surface provided with a resilient member (not shown). When the upper plate 709a is at its closed position, this elastic body can absorb variation in thickness of a detection cartridge to prevent liquid leakage.

The concave portion 709c formed in the lower plate of the cartridge holder 709 has a bottom surface formed with a plurality of liquid ports at positions corresponding to respective liquid ports formed in a bottom surface of a detection cartridge. As one example of the ports, FIG 22 shows a plurality of ports formed in a bottom surface of the aforementioned detection cartridge 1 illustrated in FIGS. 13(a) to 15(b), wherein these ports are defined by reference codes G', F", F''', H', I', K' and L', respectively. The port H' or L' corresponds to the port 113 in FIG 3, and the port G' or K' corresponds to the port 114 in FIG 3. The port F" or F''' corresponds to the waste liquid port 116. The port I' is the port 117 in liquid communication with the reference electrode of the detection cartridge 1.

Returning to FIG 18, it shows a plurality of passages and ports formed in the destination-switching line plate 716. In FIG 18, an upper surface of the plate 716 has respective openings of ports corresponding to the respective ports in FIG 22. These ports are defined by the same reference codes as those in FIG 22. A port J' is not used in this embodiment, and therefore there is no corresponding port in the detection cartridge 1.

The destination-switching line plate 716 has a lower surface having respective openings of ports G, F, H, I, J, K, L, P2, and these ports are selectively connected to the pump 10 by the switching valve mechanism 711 partly shown in FIG 20. The port P2 having an opening on the lower surface of the plate 716 is continuous with the pump outlet passage 716a, as shown in FIG 18, and in liquid communication with the pump 710, as shown in FIG 20. FIG 23 shows respective relationships of switching-type on the switching valve mechanism 707 between the liquid feed pump 710 and the tank-switching line plate 708 and switching-type on the switching valve mechanism 711 between the liquid feed pump 710 and the destination-switching line plate 716. In FIG 23, a tank containing a cleaning liquid, such as water, is used as one of the reagent tanks 706.

FIG 24 is an exploded perspective view showing the structure of the destination-switching line plate 716. The plate 716 has a laminated structure of an upper plate member 720 and a lower plate member 721 each formed by a molding process using a plastic material. Each of the passages including the passage 761 a is defined by a groove formed in an upper surface of the lower plate 721. During the molding process, each of the ports is simultaneously formed to have an opening on the lower surface. The upper plate 720 is molded with a required number of ports each penetrating in a thickness direction thereof to have an opening on an upper surface thereof at an appropriate position. These upper and lower plate members 720, 721 are adhesively fixed together to form the destination-switching line plate 716. Referring to FIG 18, the tank-switching line plate 708 also has a laminated structure of an upper plate member 722 and a lower plate member 723 each formed by a molding process using a plastic material. Further, each of the passages is defined by a groove formed in an upper surface of the lower plate member 723. During the molding process, the upper plate member 722 is molded with the ports and the slits 715c each having an opening on an upper surface thereof.

FIGS. 25 and 26 show a connection structure with a waste liquid tank 705. As shown in these figures, the waste liquid tank 705 has one lateral wall formed with a waste liquid inlet port 705a. This waste liquid inlet port 705a is provided with a sealing member 705a made, for example, of teflon. The waste-liquid-tank chamber 701b has a bottom wall formed with a convex portion, and a waste liquid discharge member 731 having a hollow needle is fixed to the convex portion. This member 731 has a passage which is continuous with the hollow needle 730 and in liquid communication with one of the ports of the lower surface of the destination-switching line plate 716 via a tube. The waste liquid tank 705 is attached at a predetermined position in a state after the sealing member 705b is pierced by the hollow needle 730 of the member 731.

In the above embodiment, the tank-switching line plate 708 and the destination-switching line plate 716 are formed as separate members. Alternatively, these members may be integrally molded in a single piece.

Although not illustrated in FIG 13(a), the analysis unit is provided with a required manual switch and a display on the cover 704 of the analyzer body 700, and these devices are appropriately connected to the electronic board 713.

FIG 27 shows a detection apparatus for use in chromatography analysis, according to another embodiment of the present invention. In this embodiment, three reagent tanks 706 of an analysis unit contain a cleaning liquid, an eluent liquid and an activating liquid, respectively. The reagent tanks 706 are switchably connected to the liquid feed pump 710 via a switching valve 707-1, 707-2, 707-3, respectively. A detection cartridge 1-1 comprises an storing section 21-1 having a sample accumulation filter, and a chromatography column 21-2. The storing section 21-1 is in liquid communication with each of a liquid inlet port 21-3 and a liquid outlet port 21-4 each having an opening on a lower surface of the cartridge 1-1, through a passage. The column 21-2 has a liquid inlet port 21-5 and a liquid outlet port 21-6.

The analysis unit has five switching valves 711-1, 711-2, 711-3, 711-4, 711-5 for switchably providing liquid communication between an outlet of a liquid feed pump 710 and each of the ports. In FIG 27, the alphabet codes A, B, C, D attached to the valves correspond to the respective reference codes of the valves in FIGS. 18 and 23.

A process of this chromatography analysis is as follows:

(1) Before setting to the analysis unit, a sample is injected into the detection cartridge, and passed through the filter serving as the accumulation (i.e., enrichment) portion (a target substance, i.e., target substance, is trapped by the filter);

(2) The detection cartridge is loaded into the analysis unit;

(3) The activating liquid is supplied to the accumulation filter (for pre-removing gas bubbles to prevent gas bubbles from mixing in the column during a substantial measurement);

(4) The eluent liquid is supplied to the column;

(5) The substantial measurement is carried out. The eluent liquid is passed through the storing section → the passage in the analysis unit → the column → an optical detection section of the analysis unit → the waste liquid tank;

(6) An operation of identification and quantification of the target substance is performed-type on a signal detected by the optical detection section; and

(7) The passage is cleaned.

FIGS. 28(a), 28(b), 29(a) and 29(b) are tables showing a time-series operation in case of using the analysis unit in combination with the detection cartridge for concentration measurement. In these figures, the term "first line" means one of the electrode arrays in FIG 1, for example, the array of electrodes 35, 36, 37, 38, and the term "second line" means the electrode array, for example, the array of electrodes 31, 32, 33, 34. In these figures, the alphabet codes attached to the valves correspond to the respective reference codes of the valves in FIG 23. Further, the term "auto-zero" means that the liquid feed pump is automatically set at a zero position.

FIGS. 30(a) and 30 are tables showing a time-series operation in case of using the analysis unit in combination with the detection cartridge for chromatography analysis. In these figures, the alphabet codes correspond to the respective reference codes of the valves in FIG 27. Further, the code "CG" means that liquid is passed through the detection cartridge.

FIG 31 is a perspective external view showing an analysis apparatus for use in liquid chromatography analysis, according to another embodiment of the present invention. This analysis apparatus comprises an analysis unit 800 incusing a housing (i.e., body) 801 generally having a rectangular parallelepiped shape. The housing 801 has a top wall provided with a swingable lid 802 adapted to selectively open and close a cartridge insertion opening 803 which is formed in the top wall to allow a liquid-chromatography analysis cartridge 804 to be inserted therethrough. The housing 801 of the analysis unit 800 in FIG 31 has an upper body 801a and a lower body 801b, as with the housing 700 of the analysis unit described in connection with FIG 13(a) to 13(c). The lower body 801b has the same structure as that of the lower body 702 of the housing 700 in FIG 13(c), and houses the same battery box and electronic board (not shown) as those illustrated in FIG 13(c). FIG 31 also shows an electrical connection plug 805 and a plug 806 for connection to a personal computer, which are connected to the battery box through an AC adaptor.

FIG 32 is a schematic sectional view of a liquid-chromatography analysis cartridge 810. The cartridge 810 has a laminated structure of four plastic plates 811, 812, 813, 814 formed though a molding process, and at least the uppermost and lowermost plastic plates 811, 814 are made of a transparent plastic material.

The lowermost plastic plate 814 has four ports 814a, 814b, 814c, 814d. The port 814a serves as a reagent supply port, and the port 814b serves as a sample-liquid injection port. The port 814c serves as a sample-liquid circulation port, and the port 814d serves as a waste-liquid port. The intermediate plastic plates 812, 831 are formed to define a filter concave portion 816 between their contact surfaces to receive therein a filter 815 which serves as an storing section for temporarily storing an target substance. Further, the intermediate plastic plates 812, 831 are formed with a passage segment 817 which extends from the port 814b and penetrates therethrough in a thickness direction of the cartridge 810 while crossing the filter concave portion 816, and a liquid circulation passage segment 818 which extends from the port 814a and penetrates therethrough in the thickness direction. The uppermost plastic plate 811 has an inner surface formed with a groove defining a passage segment 819 which provides liquid communication between the passage segments 817, 818.

The plastic plate 813 is formed with a liquid passage segment 820 which has one end extending from the port 814c and penetrates therethrough in the thickness direction. The contact surface of the plastic plate 812 with the plastic plate 813 is formed with a groove defining a liquid-chromatography column 821, and the other end of the liquid passage segment 820 is connected to one end of the liquid-chromatography column 821. The other end of the liquid-chromatography column 821 is connected to one end of a liquid passage segment 823 via a liquid passage segment 822 which penetrates the plastic plate 813 in the thickness direction. The liquid passage segment 823 is defined by a groove formed in a contact surface of the plastic plate 813 with the plastic plate 814.

The other end of the liquid passage segment 823 is connected to one end of an absorbance measurement cell 824 which comprises a liquid passage segment formed to penetrate the plastic plates 812, 813 in the thickness direction. The other end of the absorbance measurement cell 824 is connected to the waste liquid port 814d via a liquid passage segment 825 defined by a groove formed in a contact surface of the plastic plate 812 with the plastic plate 811 and a liquid passage segment 826 formed to penetrate the plastic plates 812, 813 in the thickness direction.

FIGS. 33 (a) and 33(b) show an arrangement of ports, groove and concave portions of upper and lower surfaces of each of the plastic plates 811, 812, 813, 814 of the cartridge 810, wherein FIG 33(a) shows the lower surfaces, and FIG 33(b) shows the upper surfaces. The upper surface in FIG 33(b) is illustrated in vertically inversed relation relative to the corresponding lower surface in FIG 33(a). That is, a lower edge of the lower surface in FIG 33(a) corresponds to an upper edge of the corresponding upper surface in FIG 33(b).

Referring to FIG 33(a), the uppermost plastic plate 811 has the groove which defines the liquid passage 819 in cooperation with a contact surface of the plastic plate 812 therewith. As seen in FIG 33(b), the uppermost plastic plate 811 has an outer (i.e., upper) surface which is entirely flat and smooth. As mentioned above, the plastic plate 811 is made of a transparent plastic material.

In the second plastic plate 812, the filter-defining concave portion 816 and the passages 818, 817, 826 are arranged as shown in FIG 33a. The column 821 is formed as a spiral-shaped passage, and the absorbance measurement cell 824 is arranged at a center of the spiral shape. The contact surface of the plastic plate 812 with the plastic plate 811 is formed with the groove defining the passage 825 for providing liquid communication between the absorbance measurement cell 824 and the passage 826. The ports and the liquid passages formed in the plastic plates 813, 814 are defined by the common reference numerals or codes as those of the corresponding ports and liquid passages in FIG 32, and a detailed description about their arrangement will be omitted. The plastic plate 814 is made of a transparent material. Each of the plastic plates 812, 813 is not essential to being transparent, but may be transparent.

FIGS. 34(a) and 34(b) are sectional views taken along the line "a"-"a" and the line "b"-"b" in FIG 33(a), respectively, wherein each of the adjacent plastic plates are illustrated in slightly spaced-apart relation to each other.

FIG 35 is a top plan view showing an internal structure of the upper body 801a of the housing 801. A liquid feed pump 830, a light source 831 and a waste liquid tank 832 are disposed inside the upper body 801a in a range of from one longitudinal end to a longitudinally central region of an inner space of the upper body 801 a, and arranged in parallel relation to each other. The liquid feed pump has the same structure as that of the liquid feed pump 710 in the aforementioned embodiment illustrated in FIGS. 14(a) to 14(c). The waste liquid tank 832 has the same structure as that of the waste liquid tank 705 in the aforementioned embodiment.

In this embodiment, the light source 831 is provided for liquid chromatography analysis. The light source is not limited to a specific type, but may be any suitable type capable of emitting light having a wavelength of 200 to 1100 nm, and being received in the inner space of the upper body 801a. The wavelength is adjusted depending on types of target substances. A light source suitable for the light source 831 includes "FiberLight" (combination of a deuterium lamp and a tungsten lump) available from Sentronic GmbH. A collimating lens 833 is disposed at an exit of the light source 831 to collimate an emitted beam. A slit plate 834 having a slit for reducing a diameter of the emitted beam at a predetermined value is fixed on an exit side of the collimating lens 833, and a cartridge presser plate 835 is disposed outside the slit plate 834.

A destination-switching valve plate 837 associated with five switching valves 836 indicated by codes A, B, C, D, E is disposed in the upper body 801a in fixed relation thereto, in the same manner as that in the switching valve mechanism 707 in the aforementioned embodiment. This destination- switching valve plate 837 is disposed to extend vertically. The cartridge 810 is inserted between the cartridge presser plate 835 and the destination-switching valve plate 837 vertically from above.

In order to facilitate an operation of inserting the cartridge 810, the cartridge presser plate 835 is designed to be moved in a direction away from the destination-switching valve plate 837, i.e., upwardly from the illustrated position. Specifically, each of the light source 831, the collimating lens 833, the slit plate 834 and the cartridge presser plate 835, are mounted on a base plate 838 in an integrally movable manner, and the base plate is supported by a rail (not shown) movably in the direction indicated by the arrow in FIG 35. A coil spring 839 is disposed at an end of the base plate 838 to bias the base plate 838 toward the destination-switching valve plate 837. Thus, the cartridge can be insertingly loaded in such a manner that the base plate 838 supporting the light source 831, the collimating lens 833, the slit plate 834 and the cartridge presser plate 835, is moved against the biasing force of the coil spring 839 to increase a distance between the cartridge presser plate 835 and the destination-switching valve plate 837.

As shown in FIG. 35, a guide member 840 is fixed to the destination-switching valve plate 837 at an insertion position of the cartridge 810 so as to guide and load the cartridge 810 at a proper position. FIG 36 is a perspective view showing the respective structures of the destination-switching valve plate 837 and the guide member 840 in association with the cartridge 810 in an easy-to-understand manner. The guide member 840 has an angular C-shaped guiding notch 840a. The cartridge 810 is fittingly positioned by the notch 840a. The destination-switching valve plate 837 has a protrusion 837a protruding toward the insertion position of the cartridge 810. The protrusion 837a is arranged so as to be fitted into the port 814b of the cartridge 810 when the cartridge 810 is loaded at a predetermined position.

Referring to FIG 35 again, a second slit plate 841 and a focusing lens 842 are fixed to the destination-switching valve plate 837 at a position aligned with the collimating lens 833 and the slit of the slit plate 834 in a light axis direction. Although not illustrated, the destination-switching valve plate 837 is formed with a through-hole for allowing light passed through the slit of the slit plate 834, the cartridge presser plate 835 and the measurement cell 824 of the cartridge 810 to pass therethrough. Further, a spectrometer 843 as analysis means is disposed in the upper body 801a to receive light from the focusing lens 843. The spectrometer 843 may be composed using "SAS-series OEM module" (equipped with 1024 element CMOS) available from Ocean Optics Inc. This spectrometer has an analysis ability in a wavelength range of 200 to 700 nm.

As shown in FIG 35, three reagent-tank mounting portions 844 indicated by codes F, G and H are provided in a bottom plate of the upper body 801a. Each of the reagent-tank mounting portions 844 has the same structure as that of the receiving portion 715 illustrated in FIG. 16. A tank-switching-valve plate 845 as shown in FIG. 37 is disposed above the bottom plate of the upper body 801a. The tank-switching-valve plate 845 has a bottom surface associated with three switching valves 846 indicated by codes F, G, H. The valve F is connected to the reagent-tank mounting portion indicated by the code F via a line 847, and further connected to the liquid feed pump 830 via a line 848. Among the switching valves 846, the valve G and the valve H are connected to the reagent-tank mounting portions indicated by the codes G and H via lines 849 and 850, respectively.

Returning to FIG 32, an activating liquid tank 851, an eluent liquid tank 852 and a cleaning liquid tank 853 are shown as three reagent tanks to be connected to the switching valves 846 indicated by the codes F, G and H, respectively. Each of these tanks may be designed to have the same structure as that of a corresponding one of the reagent tanks in the aforementioned embodiment, and mounted to the respective reagent-tank mounting portions 844.

FIG 32 also shows a relationship of connection of the five switching valves 835 associated with the destination-switching-valve plate 837. Among the switching valves 836, the valve B is connected to the liquid feed pump 830, and further connected to the port 814a of the cartridge 810. The valve B is also in liquid communication with the valves A, D. The valve A is connected to the port 814b of the cartridge 810, and further connected to the port 814c via the valve E. The valve D is directly connected to the port 814c. The valve C is adapted to selectively provide liquid communication between the port 814a and the waste liquid tank 832. These liquid communications are achieved through passages defined by grooves formed in the switching plates 837, 845 and appropriate lines, in the same manner as that in the aforementioned embodiment. Respective positions of each of the ports of the cartridge 810 and a corresponding one of the passages of the destination-switching plate 837 are pre-determined to allow the port of the cartridge 810 and the corresponding passing the destination-switching plate 837 to come liquid communication with each other when the cartridge 810 is loaded at the predetermined position. The protrusion 837a illustrated in FIG 36 is provided as a means to provide liquid communication between the valve A and the port 814b of the cartridge 810. For this purpose, the protrusion 837a is internally formed with a liquid passage segment.

An operation of the analysis apparatus according to this embodiment will be described below. Firstly, the cartridge 810 is prepared, and a given volume of sample liquid is injected from the port 814b into the cartridge 810. Through this operation, an target substance contained in the sample is stored in the filter 815 of the cartridge 810. The remaining liquid other than the target substance is discharged from the port 814a. Then, the apparatus is turned on to start a measurement program. This measurement program performs a operational process as shown in FIG 38. FIG 39 shows the operation in a time-series manner. Referring to FIGS. 38 and 39, the cartridge 810 having the sample liquid injected thereinto is insertingly loaded into the analysis unit 800. Then, the valves B, C of the designation-switching-valve plate 836 are opened, and the pump 830 is activated. This operation is performed to clean out the passages of the destination-switching-valve plate 873. Then, the valve F of the tank-switching-valve plate 846 is opened to suck a predetermined volume of activating liquid into the liquid feed pump 830. The activating liquid has a function of allowing the target substance stored in the filter 815 to be readily eluted from the filter 815. Then, after closing the valve F and opening the valves A, C, the liquid feed pump 830 is operated to feed the activating liquid to the filter 815. After passing through the valve A and the port 814b, the activating liquid enters the filter 815, and then flows to the waste liquid tank 832 via the passage segments 817, 819, 818, the port 814a and the valve C.

Then, after opening the valve G, a predetermined volume of eluent liquid is sucked to the liquid feed pump 830, and then the valve G is closed. Then, after opening the valve D, the pump 830 is operated to feed the eluent liquid to the column 821 via the port 814c. This operation is performed as a pre-treatment of the column. Subsequently, after closing the valve D and opening the valve G, a predetermined volume of eluent liquid is sucked to the liquid feed pump 830. Then, after opening the valves B, E, the liquid pump 830 is operated. The eluent liquid enters the filter 815 via the valve B, the port 814a, and the passage segments 818, 819, 817 to elute the target substance stored in the filter 815, and then reaches the column 821 via the port 814b, the valve E, the port 814c and the passage segment 821. Then, the eluent liquid passed across the column 821 is discharged to the waste liquid tank 832 via the passage segments 822, 823, the absorbance measurement cell 824, the passage segments 825, 826, and the port 814d. During this process, the light source 831 is turned on (see FIG 38), and an absorbance of the liquid passed through the cell 824 is measured by the spectrometer 843.

Each of the filter 815 and the column 821 contains a functional group having a chemical interaction with the target substance. The functional group contained in the filter 815 functions to trap the target substance. The eluent liquid serves as a means to elute the trapped target substance and carry the eluted target substance to the column 821. The functional group contained in the column has a fine particle size, and the column has a relatively large passage length. Thus, the target substance contained in the eluent liquid is passed through the column while chemically interacting with the functional group contained in the column, and a level of the interaction is varied depending of types of target substances. That is, respective rates of absorption and desorption of the target substance by the column 821 during passing the eluent liquid through the column are varied depending on types of target substances. Thus, a timing when the target substance is detected as a change in absorbance by the spectrometer is varied depending on types of target substances. This makes it possible to distinctly detect the target substance contained in the eluent liquid.

The detection result may be indicated on a display window appropriately provided in a top surface of the analysis unit or on a display unit of a computer connected to the analysis unit.

Subsequently, an operation of cleaning the analysis unit is performed. This cleaning operation is performed, for example, in the following process. Firstly, the valve H of the switching valves 846 is opened, and the pump 830 is operated to suck a predetermined volume of cleaning liquid. Then, after closing the valve H and opening the valves A, C, the pump 830 is operated to clean the filter 815. Then, after closing the valves A, C and opening the valve D, the pump 830 is operated to pass the cleaning liquid through the column 821. Further, after closing the valve D and opening the valves B, E, the pump 830 is operated to pass the cleaning liquid through both the filter 815 and the column 821.

A detection apparatus applied to immunoassay, according to another embodiment of the present invention, will be described below. FIGS. 41(a) to 41(c) show a principle of immunoassay analysis. FIG 41(a) shows one example of competitive immunoassay, and FIG 41(b) shows another example of competitive immunoassay. FIG 41(c) shows one example of non-competitive immunoassay.

In the example illustrated in FIG 41(a), a stage I is a treatment to be performed out of a detection cartridge. Specifically, a sample antigen is added to a reagent containing a labeled antibody, i.e., antibody with a label, to induce a preliminary reaction. Through the preliminary reaction, the sample antigen is bonded to a part of the labeled antibody contained in the reagent, by a reaction between the part of the labeled antibody and the sample antigen. The remaining labeled antigen is left in an unreacted state. In this state, the reagent is supplied to an storing section to perform a stage II of treatment. An immobilizing antigen is pre-attached to the storing section, and the unreacted antibody in the reagent is captured by the immobilizing antigen in the stage II. The reagent is discharged from the storing section while leaving the labeled antibody captured by the immobilizing antigen, in the storing element. Then, in a stage III, a substrate for a reaction with the labeled antibody is sent to an storing section to promote a reaction between the substrate and the labeled antibody. A resulting reaction product is sent to a detection mechanism in the detection cartridge, and detected by the detection mechanism. Thus, when the present invention is applied to competitive immunoassay, the storing section achieves a part of the function of the detection mechanism.

In the example illustrated in FIG 41b, a pre-treatment in a stage I is performed in the same manner as that in the example illustrated in FIG 41a. Differently from the example illustrated in FIG 41a, an immobilizing antibody is pre-attached to an storing section. The reagent subjected to the preliminary reaction in the stage I is supplied to the storing section in a detection cartridge, and only the labeled antibody reacted with the sample antigen is captured by the antibody of the storing section in a stage II. This capture is performed in such a manner that the antigen is sandwiched between the antibody pre-attached to the storing section and the labeled antibody carried by the reagent, and thereby this immunoassay is called "sandwich immunoassay". Then, in a stage III, a substrate for a reaction is sent to the storing section to promote a reaction, and a resulting reaction product is detected by the detection mechanism.

In the example illustrated in FIG 41c, an immobilizing antibody is pre-attached to an storing section. In a stage I, a sample antigen is supplied to the storing section, and bonded and captured to/by a part of the immobilizing antibody. Then, in a stage II, a reagent containing a labeled antibody is sent to the storing section, and the labeled antibody is captured in the same manner as that in the stage II illustrated in FIG 41b. In a stage III, a substrate for a reaction is sent to the storing section to promote a reaction with the labeled antibody captured by the storing section. The subsequent process is the same as that in the examples illustrated in FIGS. 41(a) and 41(b).

The present invention can be applied to the above immunoassays and any other conventional immunoassays. There are many publications about immunoassay, for example, JP 2000-155122 A and JP 2003-987171. While the examples in FIGS. 41(a) to 41(c) employ an enzyme label, any other label may be used.

When the present invention is applied to immunoassay, the detection of the reaction product may be performed by an electrochemical analysis or an optical analysis. As mentioned above, in case of detecting hydrogen peroxide produced by an oxidation/reduction enzyme, an electrochemical analysis may be used. Further, depending on types of target substances, an optical analysis may be used. FIG 42 is a bottom view showing a detection cartridge 1001 for immunoassay using detection-type on an electrochemical analysis. A passage segment 1004 from a liquid inlet port 1003 is connected to an storing section 1002, and a liquid exit of the storing section 1002 is connected to an electrode chamber via a passage segment 1005. As with the embodiment illustrated in FIG 1, a working electrode, a counter electrode and a reference electrode are disposed, but not shown, in the electrode chamber 10006. That is, this detection cartridge 1001 has the same fundamental structure as that of the detection cartridge illustrated in FIG 1. Thus, the detection cartridge 1001 has a bottom surface formed with five external-connection ports 1007, 1008, 1009, 1010. In this embodiment, the same processing unit as that for the concentration detection apparatus may be used therewith. FIG 43 is a sectional view showing the detection cartridge for immunoassay-type on an optical analysis. This figure corresponds to FIG 32 illustrating the detection cartridge for chromatography. Thus, a corresponding element or component is defined by the same reference numeral or code. This embodiment is different from the embodiment illustrated in FIG 32 only in a point that the passage segment 824 in FIG 32 is omitted, and the port 814c is directly connected to the passage segment 823. Three reagent tanks to be disposed in the processing unit contain a cleaning buffer, an eluent buffer and a CBB solution.

FIG 47 is an exploded perspective view showing a detection cartridge comprising three plastic base plates, which corresponds to FIG 1. The cartridge 1000 comprises three base plates 1011, 1012, 1013 each made of a plastic material. These base plates are joined to each other while interposing an adhesive sheet between the adjacent base plates. Each of the base plates 1011, 1012 is formed with a concave portion 1015a and a through-hole 1015b which define an accumulation chamber for receiving therein an storing section. A reference electrode R and three working electrodes S are arranged in the base plate 1011, and the adhesive sheet 1014 is formed with three small holes 104a at positions corresponding to the respective electrodes. In this embodiment, a waste liquid reservoir is provided in the detection cartridge 1000. For this waste liquid reservoir, each of the base plates 1011, 1013 is formed with a concave portion, and the intermediate base plate 1012 is formed with a through-hole 1017 for provide liquid communication therebetween. The base plate 1012 is formed with a groove 1018 serving as a reference electrode chamber corresponding to the reference electrode R, and a groove 1019 serving as a working electrode chamber corresponding to the working electrodes S. The base plate 1012 and the base plate 1013 are formed with three grooves 1020, 1021, 1022 defining a passage segment for providing liquid communication between respective ones of the accumulation chamber, the electrode chamber and the waste liquid reservoir. The remaining structure is the same as that of the aforementioned structure.

The present invention will be more specifically described-type on several Examples.

[EXAMPLE 1]

(Production Method for Substance Concentration Detection Cartridge)

Step 1: Injection Molding

1) Preparation of Molded Base Plates

Four base plates 11 to 14 were molded using an injection molding machine (produced by MEIKI Co., Ltd.). The injection molding was performed under the following conditions: cylinder temperature = 280°C; metering-section temperature = 290°C; and a mold temperature = 60°C. A runner was separated from each molded product to obtain desired molded base plates.

2) Mounting of Elements to Molded Base Plates

A carbon electrode and silver paste serving as a working electrode and a counter electrode were attached to the molded base plate 12. Specifically, after applying an adhesive on intended attachment positions of the base plate 12, and then each electrode was attached on the adhesive and fixed. A container containing potassium chloride for wetting a reference electrode and creating a silver/silver-chloride electrode during an arsenic/selenium measurement was mounted to the molded base plate 13. This container was arranged immediately above a needle portion formed on the molded base plate 12 and immediately below a pressing portion formed in the base plate 14.

Step 2: Attachment of Adhesive Tape

1) Preparation of Adhesive Tape

A double-faced adhesive tape was set in a punching machine, and subjected to punching in conformity to a shape of the molded base plate to prepare a punched adhesive tape.

2) Fixing of Molded Base Plates

A first one of the molded base plates having the adhesive tape attached on an upper surface thereof, and a second one of the molded base plates to be superimposed on the first base plate, were set in a positioning apparatus with a vacuum chamber. The position apparatus has an image-recognition-type position adjustment mechanism, and a vacuuming mechanism for an adjustment area. This apparatus makes it possible to fix the molded base plates at a proper position without intrusion of gas bubbles therebetween.

[EXAMPLE 2]

The chromatography analysis unit and cartridge according to the aforementioned embodiment illustrated in FIGS. 31 to 37 were used for a separation test of an organic-acid mixed liquid consisting of oxalic acid and succinic acid. Specifically, the following analysis apparatus was used in the test:

> Light source: FiberLight (200 to 1100 nm) produced by Sentronic GmbH
> Spectrometer: SAS-series OEM module (equipped with 1024 element CMOS; 200 to 700 nm) produced by Ocean Optics Inc.
> Cartridge: the structure illustrated in FIGS. 32 to 37
> Column filler: Wakosil-II 5C18-100 (particle size: 5 $\mu$m) produced by Wako Pure Chemical Industries, Ltd.
> Filter filler: Wakosil-II 25C18 (particle size: 25 to 30 $\mu$m; 70% up) produced by Wako Pure Chemical Industries, Ltd.
> Flow rate/temperature: 10 $\mu$l/min. room temperatures
> Measurement wavelength: 210 nm
> 0.1g of oxalic acid and 0.1g of succinic acid were mixed with 100 ml of purified water (produced by Wako Pure Chemical Industries, Ltd.) to prepare the following sample.
> Oxalic acid: 10 $\mu$g/10$\mu$l (MW = 126: dihydrate)
> Succinic acid: 10 $\mu$g/10$\mu$l (MW = 60)

-type on the process in FIGS. 38 and 39, the analysis was performed in the following steps. A liquid was fed from a liquid feed pump in a volume as shown in the column "Pump" of FIG 39. The analysis was performed by monitoring a time and an absorbance in the analysis unit. The pump was stopped at a time when an eluent liquid was fully discharged from the pump. An obtained result is shown in FIG 40. In Example 2, a peak of oxalic acid was detected at a point of 3 minutes, and a peak of succinic acid was detected at a point of 12 minutes.

[EXAMPLE 3]

The present invention was applied to a measurement of BNP (Brain Natriuretic Peptide) which is a hormone useful in estimating cardiac diseases. A method used in Example 3 is classified into enzyme immunoassay, homogeneous immunoassay, and competitive immunoassay,-type on classification of immunoassay.
A BNP antigen was used as an storing section, and a method of electrochemically detecting an action of an enzyme label was used as the detection mechanism. Example 3 is one application of a technique by Matsuura et al., described in "Analytical Chemistry, Vol. 77, No. 13, 2005, pp. 4235-4240", to the cartridge and processing unit of the present invention. Related substances were as follows:

> AChE: acetylcholinesterase
> ACh: acetylcholin
> sulfo-SMCC: sulfosuccinimidyl-4-(N-maleimidomethyl)cyclohexane-1-carboxylate
> PBS: phosphate buffer solution (phosphate buffer)
> EDC: 1-Ethyl-3-(3-dimethylaminopropyl) carbodiimide

[Preparation]

(1) A carbon fiber filter (Product No. P-1611H produced by Toyobo Co., Ltd., thickness: 0.32 mm) was immersed in 100 mg/l of gold solution (IM-sulfate), and a voltage of - 4V was applied for a holding time of 20 minutes while stirring the solution, to deposit gold on a surface of the filter. Then, the filter was subjected to cleaning at a voltage of + 0.75 V for 2 minutes to prepare a carbon fiber filter having a surface plated with gold. This gold-plated filter was immersed in 0.1 mM of cysteamine hydrochloride solution for 2 hours to bond cysteamine onto the Au surface. 0.1 g/l of EDC and 30 mg/l of BNP-32 were added to the filter, and cultured for 1 hour. Then, the filter was rinsed with PBS to prepare a gold-plated filter with BNP (antigen) immobilized on the surface thereof.
(2) The BNP immobilized gold-plated filter was cut into a piece having a diameter of 6.5 mm and a thickness of 0.4 mm, and mounted as the storing section as shown in FIG 42. A PFCE-coated electrode and an Ag-coated electrode were used as a working electrode and a reference electrode, respectively. As to a detection cartridge, while an electrode size and an external-connection port position in Example 3 were set to be the same as those of a first line in Example 1, each of an storing section and an electrode chamber was designed to have different sizes from each other. A common processing unit was used between Examples 2 and 3. The tank E in FIG 23 (reagent 4) contained 1 mM of acetylthiocholine chloride (PBS solution). The processing unit used in Example 3 was designed to be compatible with both a heavy metal analysis and immunoassay analysis only by replacing a cartridge.

(3) 0.4 g/l of BNP antigen (rabbit BNP-32 IgG) and 0.4 g/l of sulfo-SMCC were added to 0.1 M of PBS (pH = 8), and the mixture was cultured for 1 hour. Then, the cultured mixture was filtered to remove excess sulfo-SMCC. In the same manner, 1g/l of choline acetylase and 0.3 g/l of s-acetyl mercaptosuccinic anhydride were added to 0.1 M of BNP. Then, the mixture was cultured for 10 minutes, and filtered. These were mixed together at a ratio of antigen : AVh = 1 : 0.7 (mol ratio), and then cultured for 1 hour to obtain an AChE-labeled BNP antigen. (4) 2 ml of AChE-labeled BNP antigen (0.4 mg/l in PBS solution) was stirringly mixed with a BNP-containing sample (human blood) at a ratio of 1 : 1, to produce a reaction therebetween for 30 minutes. An obtained reaction product was injected from an inlet port of the cartridge using a commercially available syringe. The injected liquid was passed through the BNP immobilized gold-plated filter, and discharged from an intermediate port 115 (Step (1)). Then, 4 ml of PBS solution was supplied from the same inlet port to clean the BNP immobilized gold-plated filter, and the cleaned filter was set in the analysis unit. Through this operation, a BNP(in the sample)-BNP antigen (AChF-labeled) reaction product was discharged from the intermediate port, and this reaction product was loaded into the analysis unit in such a manner that an unreacted component of the AChF-labeled BNP antigen was captured by the BNA on the gold-plated filter as the storing section.

(5) 1 mM of acetylthiocholine chloride (PBS solution) was supplied as a substrate from the port 1007 at 200 $\mu$l/min and in a volume of 1.0 ml, and successively passed through the storing section and the electrode chamber (Step (2)). When the substrate reaches the storing section, thiocholine was produced by the action of the AChE label in an amount proportional to an amount of AChE label, and sent to the electrode chamber. Then, a LSV measurement was performed under the following conditions, and an electrode activity of thiocholine was measured from a voltage-current curve obtained by the LSV measurement. The amount of thiocholine corresponds to an amount of the unreacted BNP antigen, and has a given relationship with an amount of BNP in the sample. Thus, the measurement was performed while changing a concentration of BNP in the sample, so as to obtain an analytical curve.

$$\text{LSV conditions: } -0.7 \text{ V} \times 2 \text{ min} \rightarrow 1.4 \text{ V (sweep rate: 50 mV/sec)}$$

FIG 44 shows an operational process in the processing unit.

[EXAMPLE 4]

The present invention was applied to a separation/quantitative analysis of a specific protein-type on immobilized metal affinity chromatography (IMAC).

(1) Preparation of Cartridge

A detection cartridge having the same outer shape and external-connection ports as those illustrated in FIGS. 32, 33 and 34, except the passage section illustrated in FIG 43, was prepared through an injection molding process. Specifically, a difference is in that the chromatography column 821 is omitted, and a diameter of the cell is set at 5 mm. The base plates were made of acrylic resin. Further, an IMAC resin (Vivapure Metal Chelate resin produced by VIVA science) was used as the storing section. In Example 4, the same processing unit as that in Example 3 was used.

(2) Preparation of Sample

A roughly extracted protein liquid as a sample was prepared as follows.
500 ml of Escherichia coli carrying a poly (histidine)-tag-LacZ protein-expression vector were cultured. An obtained culture was centrifugalized at 4°C for 15 minutes at 300 × g to collect Escherichia coli. The collected Escherichia coli were suspended in 40 ml of extraction buffer using a Vortex mixer. Then, a cleaning buffer was added to the extraction buffer at a concentration of 0.50 mg/ml, and the mixture was left at room temperatures for 30 minutes. Then, Escherichia coli in the mixture were fragmented for 10 seconds using an ultrasonic disintegrator, and the fragmented Escherichia coli liquid was cooled on ice. Then, the fragmented Escherichia coli liquid was centrifugalized at 4°C for 20 minutes at 10000 × g to precipitate insoluble fractions, and supernatant fluid was collected separately. The collected rough extraction liquid was passed through a disposable filter to obtain a sample.

(3) Preparation/Pretreatment of Cartridge

2 ml of 0.1 M sodium chloride solution, 2 ml of 0.5 M nickel sulfate solution and 4 ml of 0.1 M sodium chloride solution were supplied to the cartridge in turn in this other from an inlet port thereof. Then, 2 ml of membrane equilibrating buffer solution (solution of 50 mM $NaH_2PO_4$, 300 mM NaCl and 10 mM imidazole; pH = 8.0) was passed through the cartridge. In this operation, the solution was supplied from the inlet port 814b, and discharged from the intermediate port 814a via the storing section 816.

(4) Injection of Sample to Cartridge

5 ml of the sample prepared in the Step (2) was injected from the inlet port of the cartridge using a commercially available syringe. The sample liquid was supplied from the inlet port 814b, and discharged from the intermediate port 814a via the storing section 816.

(5) Analysis Operation

The cartridge was set in the analysis unit, and 2 ml of cleaning buffer solution (solution of 50 mM $NaH_2PO_4$, 300 mM NaCl and 20 mM imidazole; pH = 8.0) was supplied from the port 814b at 1 ml/min, and discharged from the intermediate port 814a. Then, the valve 836 is switched to provide liquid communication between the port 814b and the port 814c, and 0.2 ml of eluent buffer solution (solution of 50 mM $NaH_2PO_4$, 300 mM NaCl and 250 mM imidazole; pH = 8.0) was supplied from the port 814a at 50 $\mu$l/min. This eluent buffer was sent to the cell 814 via the storing section 816. Then, the valve 836 was re-switched, and 0.2 ml of protein assay CBB solution (produced by Nacalai Tesque Inc.) was supplied from the port 814c at 500 $\mu$l/min. Through this operation, liquid in the cell 824 was approximately entirely replaced with the eluent buffer and the protein assay CBB solution. Then, the built-in spectrometer and light source (in common with those for high-performance liquid chromatography (HPLC)) of the analysis unit were activated to observe an absorbance at 595 nm, and an amount of collected protein was quantitatively calculated from a peak intensity of the absorbance.

(6) Result

FIG 45 shows the above operational process. -type on this process, the entire operation of collecting a target protein between preparation of the cartridge and termination of the analysis could be completed at a high speed of about 15 minutes.
The present invention may be applied to various measurement methods as well as the aforementioned methods. Some examples of other measurement methods which can effectively utilize the present invention are shown in FIG 46 together with their detection principles and general operational process.

As described above, the present invention provides a cartridge-type detection apparatus comprising a detection cartridge having a passage for passing a sample liquid containing an target substance, and a processing unit adapted to be loaded with the detection cartridge so as to produce information about the target substance contained in the sample liquid passed through the detection cartridge. The detection cartridge includes an storing section for temporarily storing the target substance, a liquid passage routed through said storing section, and a plurality of ports in liquid communicate with said liquid passage. The detection cartridge is provided with a part or entirety of a detection mechanism on a downstream side relative to the storing section. The processing unit includes a liquid feed pump and a line switching valve mechanism adapted to switchingly provide liquid communication between the liquid feed pump and a selected one of the plurality of ports of the detection cartridge. The valve mechanism is operable to switch between a first passage connection mode for allowing the sample liquid supplied into the detection cartridge to be passed through the storing section and then discharged out of the detection cartridge, and a second passage connection mode for allowing a reagent to be supplied from one of the plurality of ports to the storing section of the detection cartridge by an action of the liquid feed pump, and allowing the reagent passed across the storing section to be discharged out of the detection cartridge from one of the remaining ports.

In a specific embodiment of the present invention, a detection cartridge includes an storing section for temporarily storing the target substance, a liquid passage routed through said storing section, and a plurality of ports in liquid communicate with said liquid passage. A processing unit for performing analysis and/or processing using the detection cartridge includes a plurality of reagent tanks, a liquid feed pump, a tank-switching-valve plate having a tank switching valve mechanism adapted to switchingly provide liquid communication between the liquid feed pump and a selected one of the plurality of reagent tanks, and a line-switching-valve plate having a line switching valve mechanism adapted to switchingly provide liquid communication between the liquid feed pump and a desired one of the plurality of ports of the detection cartridge. The processing unit is operable to selectively shift of the valve mechanism of the tank-switching-valve plate and the valve mechanism of the line switching valve mechanism to desired valve positions while activating the liquid feed pump, so as to perform an analysis of an target substance.

Thus, the cartridge-type analysis apparatus of the present invention can arrange a functional element or component essential to analysis, within a housing of the processing unit in a significantly compact manner to facilitating providing a simplified portable analysis apparatus. Thus, the analysis apparatus of the present invention makes it possible to perform a speedy analysis at a sampling location of an target substance so as to provide a highly useful apparatus. Although the invention has been specifically shown and described with reference to specific embodiments, this description is not meant to be construed in a limiting sense. The scope of the invention should be determined by the appended claims and their legal equivalents.

**Claims**

1. A cartridge-type detection apparatus comprising a detection cartridge having at least one passage for passing a sample liquid containing a detection-target substance, and a processing unit adapted to be loaded with said detection cartridge so as to produce information about the target substance contained in the sample liquid passed through said detection cartridge, wherein:

   said detection cartridge includes an storing section for temporarily storing the target substance, at least a part of a detection mechanism, a liquid channel passing through either one or both of said storing section and said at least a part of said detection mechanism, and a plurality of ports in communication with said liquid passage; and said processing unit includes a reagent tank and a liquid feed pump,

   wherein:

   said detection cartridge and said processing unit provide in combination a first liquid passage for the target substance supplied to said detection cartridge to be discharged through said storing section out of said detection cartridge and a second liquid passage for a reagent supplied from said liquid feed pump through one of said ports in said detection cartridge to said storing section to be passed successively through said storing section and said at least a part of said detection mechanism, said first and said second liquid passages being made alternately switchable passage.

2. The cartridge-type detection apparatus as defined in claim 1, further including a thrid liquid passage formed when said detection cartridge is not connected with said processing unit for discharging said target substance supplied into said detection cartridge through said storing section out of the detection cartridge, and a fourth liquid passage formed when said detection cartridge is connected with said processing unit for a reagent to be supplied by means of said liquid feed pump from one of the ports in said detection cartridge to the storing section and successively passed through said storing section and said at least a part of said detection mechanism.

3. The cartridge-type detection apparatus as defined in claim 1 or 2, wherein said processing unit includes a passage switching valve mechanism adapted to selectively connect said liquid feed pump to a selected one of said plurality of ports in said detection cartridge.

4. The cartridge-type detection apparatus as defined in claim 3, wherein said passage switching valve mechanism is capable of switching between said second liquid passage passing successively through said storing section and said at least a part of said detection mechanism, and another liquid passage leading from a port downstream of said storing section and passing through said at least a part of said detection mechanism.

5. The cartridge-type detection apparatus as defined in claim 3, wherein said passage switching valve mechanism is capable of switching between said second liquid passage passing successively through said storing section and said at least a part of said detection mechanism, and another liquid passage for discharging liquid through a port upstream of said liquid passage passing through said storing section and then through said at least a part of said detection mechanism.

6. The cartridge-type detection apparatus as defined in claim 3, wherein said passage switching valve mechanism is capable of switching between said second liquid passage passing successively through said storing section and said at least a part of said detection mechanism, another liquid passage leading from a port downstream of said storing section and passing through said at least a part of said detection mechanism, and a further liquid passage for discharging liquid through a port upstream of said liquid passage passing through said storing section and then through said at least a part of said detection mechanism.

7. The cartridge-type detection apparatus as defined in any one of claims 1 to 6, wherein said liquid passage passing through at least a part of said detection mechanism is connected at a downstream portion to a waste liquid reservoir provided in said cartridge.

8. The cartridge-type detection apparatus as defined in any one of claims 1 to 6, wherein said passage passing through at least a part of said detection mechanism is connected at a downstream portion to a waste liquid reservoir outside the cartridge.

9. The cartridge-type detection apparatus as defined in any one of claims 1 to 8, which further includes located in said processing unit at a detection cartridge loading section, said plastic plate member being formed with channel grooves for establishing respective liquid communications between said liquid feed pump and said plurality of ports of said detection cartridge.

10. The cartridge-type detection apparatus as defined in claim 9, wherein said liquid passage passing successively through said storing section and said at least a part of said detection mechanism is formed in said plate member at least at a portion between said storing section and said at least a part of said detection mechanism.

11. The cartridge-type detection apparatus as defined in claim 9 or 10, wherein each of said detection cartridge and said plate member has a flat plate shape allowing them to be in flush contact with each other.

12. A cartridge-type detection apparatus comprising a detection cartridge having a passage for passage of a sample liquid containing an analyte, and a processing unit adapted to be loaded with said detection cartridge so as to produce information about the analyte contained in the sample liquid passed through said detection cartridge, wherein:

said detection cartridge includes an accumulation element for temporarily accumulating the analyte, a liquid channel routed through said accumulation element, and a plurality of ports in liquid communicate with said liquid channel, said detection cartridge being provided with a part or entirety of a detection mechanism on a downstream side relative to said accumulation element; and
said processing unit includes a liquid feed pump and a line switching valve mechanism adapted to switchingly provide liquid communication between said liquid feed pump and a selected one of the plurality of ports of said detection cartridge, said valve mechanism being operable to switch between a first channel connection mode for allowing the sample liquid supplied into said detection cartridge to be passed through said accumulation element and then discharged out of said detection cartridge, and a second channel connection mode for allowing a reagent to be supplied from one of the plurality of ports to the accumulation element of said detection cartridge by an action of said liquid feed pump, and allowing the reagent passed across the accumulation element to be discharged out of said detection cartridge from one of the remaining ports.

13. A cartridge-type concentration detection apparatus comprising a detection cartridge adapted to generate an electric signal indicative of a concentration of a detection-target substance contained in a sample, and a processing unit adapted to be loaded with said detection cartridge for reading said electric signal from said detection cartridge to produce information about a concentration of said target substance, wherein:

said detection cartridge includes a sample-liquid inlet portion for introducing a sample liquid having said sample dissolved therein, and a liquid passage extending from said sample-liquid inlet portion, said liquid passage being provided with an enrichment means for enriching the target substance, and a detection electrode arrangement operable, when the target substance after being enriched is being passed through said liquid passage, to generate an electric signal indicative of a concentration of said target substance; and
said processing unit includes processing means operable, in response to receiving said electric signal from said detection cartridge, to process said received electric signal so as to produce information about a concentration of said target substance in said sample.

14. A cartridge-type concentration detection apparatus comprising a detection cartridge adapted to generate an electric signal indicative of a concentration of a heavy metal contained in a sample liquid, and a processing unit adapted to be loaded with said detection cartridge for reading said electric signal from said loaded detection cartridge to produce information about a concentration of said heavy metal;

Wherein said detection cartridge includes a sample-liquid inlet portion for introducing the sample liquid, a liquid passage in liquid communication with said sample-liquid inlet portion, enrichment means disposed in said liquid passage extending from said sample-liquid inlet portion and adapted to enrich the sample liquid, and a detection electrode arrangement,

wherein said enrichment means includes an absorptive element disposed in said liquid passage and adapted to absorb the heavy metal,

wherein said enrichment means is associated with an eluent-liquid supply section for directing an eluent-liquid for eluting the heavy metal absorbed by said absorptive element, through said absorptive element and toward said detection electrode arrangement,

whereby the heavy metal absorbed in said absorptive element is eluted by a predetermined volume of the eluent liquid from said eluent-liquid supply section, and the eluted heavy metal is brought into contact with said detection electrode arrangement to allow said detection electrode arrangement to generate an electric signal indicative of a concentration of the heavy metal; and

wherein said processing unit includes processing means operable, in response to receiving said electric signal from said detection cartridge, to process said received electric signal so as to produce information about a concentration of said heavy metal in said sample.

15. The cartridge-type concentration detection apparatus as defined in claim 14, wherein said sample liquid is prepared by dissolving a soil sample.

16. A cartridge-type concentration detection apparatus comprising a detection cartridge adapted to generate an electric signal indicative of a concentration of an analyte contained in a sample, and a processing unit provided with a loading section for loading said detection cartridge thereinto and adapted to read said electric signal from said detection cartridge so as to produce information about a concentration of said analyte, wherein:

said detection cartridge includes a sample-liquid inlet portion for feeding therethrough a sample liquid having said sample dissolved therein, an enrichment element for enriching the sample liquid fed in the sample-liquid inlet portion, a detection electrode system, and a channel for providing liquid communication between respective ones of the sample-liquid inlet portion, the enrichment element and the detection electrode system, wherein said enrichment element includes an absorptive substrate adapted to receive the sample liquid from the sample-liquid inlet portion and absorb the analyte contained in the sample liquid, and

wherein said concentration detection apparatus further includes an eluent-liquid supply section adapted to associated with said enrichment element and supply an eluent liquid for eluting the analyte absorbed to said absorptive substrate, through said absorptive substrate and toward said detection electrode system, whereby the analyte absorbed to said absorptive substrate is eluted by a predetermined volume of the eluent liquid from said eluent-liquid supply section, and the eluted analyte is brought into contact with said detection electrode system to allow said detection electrode system to generate an electric signal indicative of a concentration of the analyte; and

said processing unit includes processing means operable, in response to receiving said electric signal from said detection cartridge, to process said received electric signal so as to produce information about a concentration of said analyte in said sample.

17. A cartridge-type concentration detection apparatus comprising a detection cartridge adapted to generate an electric signal indicative of a concentration of a target substance contained in a sample, and a processing unit provided with a loading section for loading said detection cartridge thereinto and adapted to read said electric signal from said detection cartridge so as to produce information about a concentration of said target substance;

wherein said detection cartridge includes a sample-liquid inlet portion for introducing a sample liquid having said sample dissolved therein, enrichment means for enriching the sample liquid introduced into the sample-liquid inlet portion, a detection electrode arrangement, and passages for providing liquid communication between respective ones of said sample-liquid inlet portion, said enrichment means and said detection electrode arrangement;

wherein said enrichment means includes an absorptive element adapted to receive the sample liquid from the sample-liquid inlet portion and absorb the target substance contained in the sample liquid, and

wherein said enrichment means is associated with an eluent-liquid supply section for directing an eluent-liquid for eluting the target substance absorbed by said absorptive element, through said absorptive element and toward said detection electrode arrangement,

wherein the apparatus further comprising a passage switching valve mechanism adapted to be selectively shifted between a sample-liquid feed position for opening a sample-liquid feed passage extending from said sample-liquid inlet portion to said enrichment means and closing an eluent-liquid supply passage extending from said eluent-liquid supply section to said enrichment means, and an eluent-liquid supply position for closing said sample-liquid feed passage extending from said sample-liquid inlet portion to said enrichment means and opening said eluent-liquid supply passage extending from said eluent-liquid supply section to said enrichment means, and pumping means for supplying an eluent liquid from said eluent-liquid supply section to said enrichment means when said passage switching valve mechanism is at said eluent-liquid supply position, whereby the target substance absorbed by said absorptive element is eluted by a predetermined volume of the eluent liquid from said eluent-liquid supply section, and the eluted target substance is brought into contact with said detection electrode arrangement to allow said detection electrode arrangement to generate an electric signal indicative of a concentration of the target substance; and

wherein said processing unit includes processing means operable, in response to receiving said electric signal from said detection cartridge, to process said received electric signal so as to produce information about a concentration of said target substance in said sample.

18. The cartridge-type concentration detection apparatus as defined in claim 17, wherein each of said eluent-liquid supply section, said valve mechanism and said pumping means is installed in a common casing of said processing unit.

19. The cartridge-type concentration detection apparatus as defined in any one of claims 14 to 18, wherein:

said detection cartridge is formed with an external-connection port for discharging the sample liquid passed through said absorptive element, outside said detection cartridge; and
said concentration detection apparatus includes a valve mechanism operable, in a process of feeding the sample liquid, to open said external-connection port so as to allow the sample liquid to be passed through said absorptive element and then discharged out of said detection cartridge via said external-connection port, and, in a process of supplying the eluent liquid, to close said external-connection port so as to allow the eluent liquid to be passed through said absorptive element and said electrode arrangement in turn.

20. The cartridge-type concentration detection apparatus as defined in any one of claims 14 to 19, wherein said absorptive substrate is formed as any one of a membrane, a fine particle and a porous body.

21. The cartridge-type concentration detection apparatus as defined in claim 20, wherein said absorptive substrate consists of a cationic substance-absorptive substrate.

22. The cartridge-type concentration detection apparatus as defined in claim 20, wherein said absorptive substrate is formed of a material having a surface modified with a sulfonic acid group.

23. The cartridge-type concentration detection apparatus as defined in claim 20, wherein said absorptive substrate consists of an anionic substance-absorptive substrate.

24. The cartridge-type concentration detection apparatus as defined in claim 23, wherein said absorptive substrate is formed of a material having a surface modified with a quaternary amine group.

25. The cartridge-type concentration detection apparatus as defined in any one of claims 13 to 24, wherein:

said detection cartridge has a card shape; and
said processing unit has a casing formed with a cartridge insertion portion for allowing insertion of said card-shaped detection cartridge.

26. The cartridge-type concentration detection apparatus as defined in any one of claims 13 to 25, wherein said electrode system includes a plurality of working electrode elements, at least one counter electrode element, and at least one reference electrode element.

27. The cartridge-type concentration detection apparatus as defined in any one of claims 13 to 26, wherein said electrode system includes a plurality of working electrode elements, at least one counter electrode element, and at least one reference electrode element, wherein each of said plurality of working electrode elements is formed to have a different area so as to work for a measurement of a different concentration range.

28. The cartridge-type concentration detection apparatus as defined in any one of claims 13 to 20, wherein:

said enrichment element has a structure where a cation-absorptive substrate adapted to absorb a cationic substance and an anion-absorptive substrate adapted to absorb an anionic substance are arranged in parallel relation to each other; and
said electrode system includes two electrode pairs each associated with a corresponding one of said cation-absorptive substrate and said anion-absorptive substrate.

29. A portable, cartridge-type concentration detection apparatus comprising a flat, card-shaped cartridge, and a processing unit having a casing formed with an insertion portion for allowing insertion of said card-shaped cartridge, wherein:

said cartridge includes a sample-liquid inlet portion for feeding therethrough a sample liquid which is a concentration-detection target, a liquid channel in liquid communication with said sample-liquid inlet portion, and a concentration-detection electrode system disposed in said liquid channel in such a manner as to come into contact with the sample liquid fed in said liquid channel to generate an electric signal indicative of a concentration of a specific substance contained in said sample liquid, said electrode system including a plurality of working electrode elements, at least one counter electrode element, and at least one reference electrode element, wherein each of said plurality of working electrode elements is formed to have a different area so as to work for a measurement of a different concentration range; and
said processing unit includes processing means operable, in response to receiving said electric signal from said cartridge, to process said received electric signal so as to produce information about a concentration of said substance in said sample.

30. A portable, cartridge-type concentration detection apparatus comprising a flat, card-shaped cartridge, and a processing unit having a casing formed with an insertion portion for allowing insertion of said card-shaped cartridge, wherein:

said cartridge includes therewithin a sample-liquid inlet portion for feeding therethrough a sample liquid as a concentration-detection target, a waste liquid reservoir, a liquid channel extending from said sample-liquid inlet portion to said waste liquid reservoir, and a concentration-detection electrode system disposed in said liquid channel in such a manner as to come into contact with the sample liquid fed in said liquid channel to generate an electric signal indicative of a concentration of a specific substance contained in said sample liquid, said electrode system including at least one working electrode element, at least one counter electrode element, and at least one reference electrode element; and
said processing unit includes processing means operable, in response to receiving said electric signal from said cartridge, to process said received electric signal so as to produce information about a concentration of said substance in said sample.

31. A cartridge-type concentration detection apparatus comprising a flat, card-shaped cartridge, and a processing unit having a casing formed with an insertion portion for allowing insertion of said card-shaped cartridge, wherein:

said cartridge includes therewithin a sample-liquid inlet portion for feeding therethrough a sample liquid as a concentration-detection target, a liquid channel in liquid communication with said sample-liquid inlet portion, and a concentration-detection electrode system disposed in said liquid channel and adapted to generate an electric signal indicative of a concentration of a specific substance contained in said sample liquid which is fed in said liquid channel and passed through said concentration-detection electrode system, said cartridge including a first sheet made of a resin material and formed to have one surface with a concave portion defining at least a part of said liquid channel, a second sheet made of a resin material and formed with a through-hole constituting said sample-liquid inlet portion and a concave portion receiving therein said concentration-detection electrode system, and a third sheet made of a resin material and formed with a through-hole constituting said sample-liquid inlet portion,

wherein:

said first, second and third sheets being laminated in turn while interposing an insulation sheet between adjacent ones thereof;
said concentration-detection electrode system includes an electrode element disposed in the electrode-receiving concave portion of said second sheet;
said second sheet and said third sheet are laminated to allow said electrode element to face said third sheet; and

the insulation sheet interposed between said second sheet and said third sheet has a hole formed at a position corresponding to said electrode element to expose a predetermined area of said electrode element, so that a liquid channel for leading the sample liquid to said electrode element is defined on a side of a surface of said third sheet facing said second sheet.

32. The cartridge-type concentration detection apparatus as defined in claim 31, wherein said first sheet of said cartridge has a surface located to face said second sheet and formed with a concave portion constituting a waste liquid reservoir for receiving therein a waste liquid from said electrode element.

33. The cartridge-type concentration detection apparatus as defined in claim 32, wherein said cartridge includes first, second and third sheets made of a resin material and laminated together while interposing an insulation sheet between adjacent ones thereof, wherein: said first sheet has a surface located to face said second sheet and formed with a concave portion constituting at least a part of said liquid channel and said waste reservoir; said second sheet is formed with a through-hole constituting said sample-liquid inlet portion, a concave portion receiving therein said absorptive substrate and a concave portion receiving therein said concentration-detection electrode system, and a third sheet made of a resin material and formed with a through-hole constituting said sample-liquid inlet portion, wherein
said concentration-detection electrode system includes an electrode element disposed in the electrode-receiving concave portion of said second sheet;
said second sheet and said third sheet are laminated to allow said electrode element to face said third sheet; and the insulation sheet interposed between said second sheet and said third sheet has a hole formed at a position corresponding to said electrode element to expose a predetermined area of said electrode element, so that a liquid channel for leading the sample liquid to said electrode element is defined on a side of a surface of said third sheet facing said second sheet.

34. The cartridge-type concentration detection apparatus as defined in claim 33, wherein:

said processing unit has an eluent-liquid supply section provided in the casing thereof; and
said first sheet is formed with a connection hole for providing liquid communication between said eluent-liquid supply section and said absorptive substrate-receiving concave portion of said second sheet.

35. A detection cartridge for use in the detection apparatus as defined in any one of the preceding claims 1 to 34.

36. A detection cartridge for a cartridge-type concentration detection apparatus including a detection cartridge adapted to generate an electric signal indicative of a concentration of an analyte contained in a sample and a processing unit adapted to be loaded with said detection cartridge so as to read said electric signal from said loaded cartridge to produce information about a concentration of said analyte, said detection cartridge comprising:

a sample-liquid inlet portion for feeding therethrough a sample liquid having a sample dissolved therein; and
a liquid channel extending from said sample-liquid inlet portion, said liquid channel being provided with an enrichment element for enriching an analyte, and a detection electrode system which is operable, when the analyte after being enriched is being passed through said liquid channel, to generate an electric signal indicative of a concentration of said analyte.

37. A detection cartridge for generating an electric signal indicative of a concentration of an analyte contained in a sample, comprising:

a sample-liquid inlet portion for feeding therethrough a sample liquid having said sample dissolved therein;
an enrichment element for enriching the sample fed through said sample-liquid inlet portion;
a detection electrode system; and
a channel for providing liquid communication between respective ones of said sample-liquid inlet portion, said enrichment element and said detection electrode system,

wherein:

said enrichment element includes an absorptive substrate adapted to receive the sample liquid from the sample-liquid inlet portion and absorb the analyte contained in the sample liquid, said enrichment element being associated with an eluent-liquid supply section adapted to supply an eluent liquid for eluting the analyte absorbed to said absorptive substrate, through said absorptive substrate and toward said detection electrode system.

38. A sample-liquid concentration detection cartridge comprising therewithin:

a sample-liquid inlet portion for feeding therethrough a sample liquid which is a concentration-detection target;
a liquid channel in liquid communication with said sample-liquid inlet portion;
a concentration-detection electrode system disposed in said liquid channel and adapted to generate an electric signal indicative of a concentration of a specific substance contained in said sample liquid which is fed in said liquid channel and passed through said concentration-detection electrode system;
a first sheet made of a resin material and formed to have one surface with a concave portion defining at least a part of said liquid channel;
a second sheet made of a resin material and formed with a through-hole constituting said sample-liquid inlet portion and a concave portion receiving therein said concentration-detection electrode system; and
a third sheet made of a resin material and formed with a through-hole constituting said sample-liquid inlet portion,

wherein:

said first, second and third sheets being laminated in turn while interposing an insulation sheet between adjacent ones thereof;
said concentration-detection electrode system includes an electrode element disposed in the electrode-receiving concave portion of said second sheet;
said second sheet and said third sheet are laminated to allow said electrode element to face said third sheet; and
the insulation sheet interposed between said second sheet and said third sheet has a hole formed at a position corresponding to said electrode element to expose a predetermined area of said electrode element, so that a liquid channel for leading the sample liquid to said electrode element is defined on a side of a surface of said third sheet facing said second sheet.

39. A processing unit for use with a detection cartridge including a passage for passing a sample liquid containing a target substance and at least one detection electrode adapted to generate an electric signal indicative of the target substance contained in the sample liquid passed through said passage, comprising;
a unit body which has a cartridge loading portion for detachably loading said detection cartridge thereinto, and a reagent-tank mounting portion for detachably mounting a reagent tank thereto, said unit body including:

a liquid feed pump;
a switching valve;
a pump inlet channel for leading a reagent from said reagent tank mounted on said reagent-tank mounting portion to said liquid feed pump;
a pump outlet channel for providing liquid communication between an outlet port of said liquid feed pump and said switching valve; and
a reagent supply passage for providing liquid communication between said switching valve and a reagent port of said cartridge loading portion located at a position corresponding to a reagent inlet of said detection cartridge.

40. The processing unit as defined in claim 39, wherein said detection mechanism includes:

a power applying section for applying to said detection electrode a power necessary for detection by said detection electrode;
a signal-detection section for detecting an electric signal generated from the detection electrode of said detection cartridge; and
a power supply section installed in said unit body and adapted to supply a power necessary for said signal-detection by said detection mechanism.

41. The processing unit as defined in claim 39 or 40, wherein each of said pump inlet channel, said pump outlet channel and said reagent supply passage is defined by a groove formed in a plastic-molded plate member disposed at a bottom of said unit body.

42. A processing unit for use with a detection cartridge which has a channel for passage of a sample liquid containing an analyte and includes a detection electrode adapted to generate an electric signal indicative of the analyte contained in the sample liquid passed through said channel, comprising a unit body which has a body upper portion for receiving therein a processing-unit functional section, and a body lower portion for receiving therein a power supply section, wherein said body upper section includes therewithin a cartridge loading portion for detachably loading said detection cartridge thereto, a reagent-tank mounting portion for detachably mounting a reagent tank thereto, a liquid feed pump, and a switching valve, said body upper section having a plastic-molded plate member which is disposed along a bottom thereof, and formed with a groove defining a pump inlet channel for leading a reagent from the reagent tank mounted to said reagent-tank mounting portion, to said liquid feed pump, a pump outlet channel for providing liquid communication between an outlet port of said liquid feed pump and said switching valve, and a reagent supply channel for providing liquid communication between said switching valve and a reagent port of said cartridge loading portion located at a position corresponding to a reagent inlet of said detection cartridge.

**43.** The processing unit as defined in claim 41 or 42, wherein:

said plastic-molded plate member comprises two laminated plastic plates; and
said groove is formed in either one of contact surfaces between said two laminate laminated plastic plates.

**44.** A cartridge-type detection apparatus comprising a detection cartridge having a passage for passage of a sample liquid containing a target substance, and a processing unit adapted to be loaded with said detection cartridge so as to produce information about the analyte contained in the sample liquid passed through said detection cartridge, wherein:

said detection cartridge includes an storing element for temporarily storing the analyte, a liquid passage routed through said storing element, and a plurality of ports in liquid communication with said liquid passage; and
said processing unit includes a reagent tank, a liquid feed pump connected to said reagent tank, and a passage switching valve mechanism adapted to alternately provide liquid communication between said liquid feed pump and a desired one of the plurality of ports of said detection cartridge, said processing unit being operable to selectively shift said passage switching valve mechanism to a desired valve position while activating said liquid feed pump, so as to perform an analysis of said analyte.

**45.** The cartridge-type detection apparatus as defined in claim 44, wherein a plurality of said reagent tanks are provided, and said cartridge-type detection apparatus includes a tank switching valve mechanism adapted to alternately provide liquid communication between a selected one of said plurality of reagent tanks and said liquid feed pump, and a tank-switching-valve plate on which said tank switching valve mechanism is mounted.

**46.** The cartridge-type detection apparatus as defined in claim 45, wherein said plurality of reagent tanks comprise a cleaning liquid tank, an activating liquid tank and an eluent liquid tank.

**47.** The cartridge-type detection apparatus as defined in any one of claims 44 to 46, wherein said processing unit has a housing which houses electronic processing means including a power supply and information processing means.

**48.** The cartridge-type detection apparatus as defined in claim 47, wherein:

said detection cartridge includes an electrode; and
said information processing means of said processing unit is operable to read the electric signal from said detection cartridge so as to produce information about said analyte.

**49.** The cartridge-type detection apparatus as defined in any one of claims 44 to 48, wherein:

said detection cartridge includes an optical cell; and
said processing unit includes a light source, an incident optical system for directing light from said light source toward said absorbance measurement cell of said detection cartridge, and a spectrometer operable, in response to receiving light transmitted through said absorbance measurement cell, to produce information about the analyte.

**50.** The cartridge-type detection apparatus as defined in claim 49, wherein said detection cartridge includes a chromatographic column disposed on a downstream side relative to said accumulation element and on an upstream side relative to said optical cell.

**51.** The cartridge-type detection apparatus as defined in any one of claims 44 to 49, wherein said accumulation element of said detection cartridge comprises a substance which exhibits a specific binding reaction with the analyte.

52. The cartridge-type detection apparatus as defined in any one of claims 44 to 51, wherein said processing unit has a cartridge loading portion for detachably loading said detection cartridge thereto, and a reagent-tank mounting portion for detachably mounting said reagent tank thereto.

53. The cartridge-type detection apparatus as defined in any one of claims 1 to 12, wherein said detection cartridge is an immunoassay cartridge.

54. The cartridge-type detection apparatus as defined in claim 53, wherein said storing section of said detection cartridge is a filter having an antigen or antibody immobilized thereto.

55. The cartridge-type detection apparatus as defined in claim 53 or 54, wherein said detection mechanism includes a chromatographic column.

56. The cartridge-type detection apparatus as defined in any one of claims 1 to 12, wherein said detection mechanism includes an electrode.

57. (Amended) The cartridge-type detection apparatus as defined in any one of claims 1 to 12, wherein said detection mechanism includes an optical cell.

58. The cartridge-type detection apparatus as defined in any one of claims 1 to 12, wherein:

    said storing element comprises a substance which exhibits an ion exchange reaction.

59. The cartridge-type detection apparatus as defined in any one of claims 1 to 12, and 44, wherein said storing element comprises a substance which exhibits a specific binding reaction..

60. A detection cartridge for use in the cartridge-type detection apparatus as defined in claim 56, comprising a first sheet and a second sheet which are made of a resin material and laminated together, said first sheet being formed with an electrode-receiving concave portion, wherein said electrode is disposed in said concave portion, a part of said liquid channel for passage of the reagent being formed in said second sheet at a position corresponding to said electrode, said accumulation element being formed at a position away from said electrode;
    an insulating sheet disposed between said first sheet and said second sheet, said insulating sheet having a hole formed to have a predetermined sectional area, at a position corresponding to said electrode; and
    a third sheet disposed on either one or both of respective surfaces of said first and second sheets on opposite sides of opposed surfaces thereof, said third sheet being formed with a groove defining a part of said liquid channel in liquid communication with said accumulation element.

61. A detection cartridge for use in the cartridge-type detection apparatus as defined in claim 57, wherein said optical cell comprises a single first sheet having a through-hole, and two transparent second sheets disposed on respective opposite surfaces of a resin plate, said first sheet being formed with said accumulation element, said second sheet being formed with a groove defining a part of said liquid channel in liquid communication with said accumulation element.

62. The detection cartridge as defined in claim 60, which includes a chromatographic column disposed in said liquid channel at an intermediate position between said accumulation element and said electrode.

63. The detection cartridge as defined in claim 63, which includes a chromatographic column disposed in said liquid channel at an intermediate position between said accumulation element and said optical cell.


**Amended claims under Art. 19.1 PCT**

1. A cartridge-type detection apparatus comprising a detection cartridge having at least one passage for passing a sample liquid containing a detection-target substance, and a processing unit adapted to be loaded with said detection cartridge so as to produce information about the target substance contained in the sample liquid passed through said detection cartridge, wherein:

said detection cartridge includes an storing section for temporarily storing the target substance, at least a part of a detection mechanism, a liquid channel passing through either one or both of said storing section and said at least a part of said detection mechanism, and a plurality of ports in communication with said liquid passage; and said processing unit includes a reagent tank and a liquid feed pump,

wherein:

said detection cartridge and said processing unit provide in combination a first liquid passage for the target substance supplied to said detection cartridge to be discharged through said storing section out of said detection cartridge and a second liquid passage for a reagent supplied from said liquid feed pump through one of said ports in said detection cartridge to said storing section to be passed successively through said storing section and said at least a part of said detection mechanism, said first and said second liquid passages being made alternately switchable passage.

2. The cartridge-type detection apparatus as defined in claim 1, further including a thrid liquid passage formed when said detection cartridge is not connected with said processing unit for discharging said target substance supplied into said detection cartridge through said storing section out of the detection cartridge, and a fourth liquid passage formed when said detection cartridge is connected with said processing unit for a reagent to be supplied by means of said liquid feed pump from one of the ports in said detection cartridge to the storing section and successively passed through said storing section and said at least a part of said detection mechanism.

3. The cartridge-type detection apparatus as defined in claim 1 or 2, wherein said processing unit includes a passage switching valve mechanism adapted to selectively connect said liquid feed pump to a selected one of said plurality of ports in said detection cartridge.

4. The cartridge-type detection apparatus as defined in claim 3, wherein said passage switching valve mechanism is capable of switching between said second liquid passage passing successively through said storing section and said at least a part of said detection mechanism, and another liquid passage leading from a port downstream of said storing section and passing through said at least a part of said detection mechanism.

5. The cartridge-type detection apparatus as defined in claim 3, wherein said passage switching valve mechanism is capable of switching between said second liquid passage passing successively through said storing section and said at least a part of said detection mechanism, and another liquid passage for discharging liquid through a port upstream of said liquid passage passing through said storing section and then through said at least a part of said detection mechanism.

6. The cartridge-type detection apparatus as defined in claim 3, wherein said passage switching valve mechanism is capable of switching between said second liquid passage passing successively through said storing section and said at least a part of said detection mechanism, another liquid passage leading from a port downstream of said storing section and passing through said at least a part of said detection mechanism, and a further liquid passage for discharging liquid through a port upstream of said liquid passage passing through said storing section and then through said at least a part of said detection mechanism.

7. The cartridge-type detection apparatus as defined in any one of claims 1 to 6, wherein said liquid passage passing through at least a part of said detection mechanism is connected at a downstream portion to a waste liquid reservoir provided in said cartridge.

8. The cartridge-type detection apparatus as defined in any one of claims 1 to 6, wherein said passage passing through at least a part of said detection mechanism is connected at a downstream portion to a waste liquid reservoir outside the cartridge.

9. The cartridge-type detection apparatus as defined in any one of claims 1 to 8, which further includes located in said processing unit at a detection cartridge loading section, said plastic plate member being formed with channel grooves for establishing respective liquid communications between said liquid feed pump and said plurality of ports of said detection cartridge.

10. The cartridge-type detection apparatus as defined in claim 9, wherein said liquid passage passing successively through said storing section and said at least a part of said detection mechanism is formed in said plate member at least at a portion between said storing section and said at least a part of said detection mechanism.

**11.** The cartridge-type detection apparatus as defined in claim 9 or 10, wherein each of said detection cartridge and said plate member has a flat plate shape allowing them to be in flush contact with each other.

**12.** (Deleted)

**13.** A cartridge-type concentration detection apparatus comprising a detection cartridge adapted to generate an electric signal indicative of a concentration of a detection-target substance contained in a sample, and a processing unit adapted to be loaded with said detection cartridge for reading said electric signal from said detection cartridge to produce information about a concentration of said target substance, wherein:

said detection cartridge includes a sample-liquid inlet portion for introducing a sample liquid having said sample dissolved therein, and a liquid passage extending from said sample-liquid inlet portion, said liquid passage being provided with an enrichment means for enriching the target substance, and a detection electrode arrangement operable, when the target substance after being enriched is being passed through said liquid passage, to generate an electric signal indicative of a concentration of said target substance; and
said processing unit includes processing means operable, in response to receiving said electric signal from said detection cartridge, to process said received electric signal so as to produce information about a concentration of said target substance in said sample.

**14.** A cartridge-type concentration detection apparatus comprising a detection cartridge adapted to generate an electric signal indicative of a concentration of a heavy metal contained in a sample liquid, and a processing unit adapted to be loaded with said detection cartridge for reading said electric signal from said loaded detection cartridge to produce information about a concentration of said heavy metal;
Wherein said detection cartridge includes a sample-liquid inlet portion for introducing the sample liquid, a liquid passage in liquid communication with said sample-liquid inlet portion, enrichment means disposed in said liquid passage extending from said sample-liquid inlet portion and adapted to enrich the sample liquid, and a detection electrode arrangement,
wherein said enrichment means includes an absorptive element disposed in said liquid passage and adapted to absorb the heavy metal,
wherein said enrichment means is associated with an eluent-liquid supply section for directing an eluent-liquid for eluting the heavy metal absorbed by said absorptive element, through said absorptive element and toward said detection electrode arrangement,
whereby the heavy metal absorbed in said absorptive element is eluted by a predetermined volume of the eluent liquid from said eluent-liquid supply section, and the eluted heavy metal is brought into contact with said detection electrode arrangement to allow said detection electrode arrangement to generate an electric signal indicative of a concentration of the heavy metal; and
wherein said processing unit includes processing means operable, in response to receiving said electric signal from said detection cartridge, to process said received electric signal so as to produce information about a concentration of said heavy metal in said sample.

**15.** (Deleted)

**16.** (Deleted)

**17.** A cartridge-type concentration detection apparatus comprising a detection cartridge adapted to generate an electric signal indicative of a concentration of a target substance contained in a sample, and a processing unit provided with a loading section for loading said detection cartridge thereinto and adapted to read said electric signal from said detection cartridge so as to produce information about a concentration of said target substance;
wherein said detection cartridge includes a sample-liquid inlet portion for introducing a sample liquid having said sample dissolved therein, enrichment means for enriching the sample liquid introduced into the sample-liquid inlet portion, a detection electrode arrangement, and passages for providing liquid communication between respective ones of said sample-liquid inlet portion, said enrichment means and said detection electrode arrangement;
wherein said enrichment means includes an absorptive element adapted to receive the sample liquid from the sample-liquid inlet portion and absorb the target substance contained in the sample liquid, and
wherein said enrichment means is associated with an eluent-liquid supply section for directing an eluent-liquid for eluting the target substance absorbed by said absorptive element, through said absorptive element and toward said detection electrode arrangement,

wherein the apparatus further comprising a passage switching valve mechanism adapted to be selectively shifted between a sample-liquid feed position for opening a sample-liquid feed passage extending from said sample-liquid inlet portion to said enrichment means and closing an eluent-liquid supply passage extending from said eluent-liquid supply section to said enrichment means, and an eluent-liquid supply position for closing said sample-liquid feed passage extending from said sample-liquid inlet portion to said enrichment means and opening said eluent-liquid supply passage extending from said eluent-liquid supply section to said enrichment means, and pumping means for supplying an eluent liquid from said eluent-liquid supply section to said enrichment means when said passage switching valve mechanism is at said eluent-liquid supply position, whereby the target substance absorbed by said absorptive element is eluted by a predetermined volume of the eluent liquid from said eluent-liquid supply section, and the eluted target substance is brought into contact with said detection electrode arrangement to allow said detection electrode arrangement to generate an electric signal indicative of a concentration of the target substance; and

wherein said processing unit includes processing means operable, in response to receiving said electric signal from said detection cartridge, to process said received electric signal so as to produce information about a concentration of said target substance in said sample.

**18.** (Deleted)

**19.** (Amended) The cartridge-type concentration detection apparatus as defined in any one of claims 14 to 17, wherein:

said detection cartridge is formed with an external-connection port for discharging the sample liquid passed through said absorptive element, outside said detection cartridge; and

said concentration detection apparatus includes a valve mechanism operable, in a process of feeding the sample liquid, to open said external-connection port so as to allow the sample liquid to be passed through said absorptive element and then discharged out of said detection cartridge via said external-connection port, and, in a process of supplying the eluent liquid, to close said external-connection port so as to allow the eluent liquid to be passed through said absorptive element and said electrode arrangement in turn.

**20.** (Deleted)

**21.** (Deleted)

**22.** (Deleted)

**23.** (Deleted)

**24.** (Deleted)

**25.** (Deleted)

**26.** (Deleted)

**27.** (Deleted)

**28.** (Deleted)

**29.** (Deleted)

**30.** (Deleted)

**31.** (Deleted)

**32.** (Deleted)

**33.** (Deleted)

**34.** (Deleted)

**35.** (Amended) A detection cartridge for use in the detection apparatus as defined in any one of the preceding claims.

**36.** (Deleted)

**37.** (Deleted)

**38.** (Deleted)

**39.** A processing unit for use with a detection cartridge including a passage for passing a sample liquid containing a target substance and at least one detection electrode adapted to generate an electric signal indicative of the target substance contained in the sample liquid passed through said passage, comprising;
a unit body which has a cartridge loading portion for detachably loading said detection cartridge thereinto, and a reagent-tank mounting portion for detachably mounting a reagent tank thereto, said unit body including:

> a liquid feed pump;
> a switching valve;
> a pump inlet channel for leading a reagent from said reagent tank mounted on said reagent-tank mounting portion to said liquid feed pump;
> a pump outlet channel for providing liquid communication between an outlet port of said liquid feed pump and said switching valve; and
> a reagent supply passage for providing liquid communication between said switching valve and a reagent port of said cartridge loading portion located at a position corresponding to a reagent inlet of said detection cartridge.

**40.** (Deleted)

**41.** (Amended) The processing unit as defined in claim 39, wherein each of said pump inlet channel, said pump outlet channel and said reagent supply passage is defined by a groove formed in a plastic-molded plate member disposed at a bottom of said unit body.

**42.** (Deleted)

**43.** (Amended) The processing unit as defined in claim 41, wherein:

> said plastic-molded plate member comprises two laminated plastic plates; and
> said groove is formed in either one of contact surfaces between said two laminate laminated plastic plates.

**44.** (Amended) A cartridge-type detection apparatus comprising a detection cartridge having a passage for passing a sample liquid containing a target substance, and a processing unit adapted to be loaded with said detection cartridge so as to produce information about the target substance contained in the sample liquid passed through said detection cartridge, wherein:

> said detection cartridge includes an storing section for temporarily storing the target substance, a liquid passage routed through said storing section, and a plurality of ports in liquid communication with said liquid passage; and
> said processing unit includes a reagent tank, a liquid feed pump connected to said reagent tank, and a passage switching valve mechanism adapted to alternately provide liquid communication between said liquid feed pump and a desired one of the plurality of ports of said detection cartridge, said processing unit being operable to selectively shift said passage switching valve mechanism to a desired valve position while activating said liquid feed pump, so as to perform an analysis of said target substance.

**45.** The cartridge-type detection apparatus as defined in claim 44, wherein a plurality of said reagent tanks are provided, and said cartridge-type detection apparatus includes a tank switching valve mechanism adapted to alternately provide liquid communication between a selected one of said plurality of reagent tanks and said liquid feed pump, and a tank-switching-valve plate on which said tank switching valve mechanism is mounted.

**46.** (Deleted)

**47.** (Deleted)

**48.** (Deleted)

**49.** (Deleted)

**50.** (Deleted)

**51.** (Deleted)

**52.** (Deleted)

**53.** (Amended) The cartridge-type detection apparatus as defined in any one of claims 1 to 11, 44 and 45, wherein said detection cartridge is an immunoassay cartridge.

**54.** The cartridge-type detection apparatus as defined in claim 53, wherein said storing section of said detection cartridge is a filter having an antigen or antibody immobilized thereto.

**55.** (Deleted)

**56.** (Amended) The cartridge-type detection apparatus as defined in any one of claims 1 to 11, 44, 45, 53 and 54, wherein said detection mechanism includes an electrode.

**57.** (Amended) The cartridge-type detection apparatus as defined in any one of claims 1 to 11, 44, 45, 53, 54 and 56, wherein said detection mechanism includes an optical cell.

**58.** (Amended) The cartridge-type detection apparatus as defined in claim 57, wherein:

said detection cartridge includes an optical cell; and
said processing unit includes a light source, an incident optical system for directing light from said light source toward said absorbance measurement cell of said detection cartridge, and a spectrometer operable, in response to receiving light transmitted through said absorbance measurement cell, to produce information about the target substance.

**59.** (Amended) The cartridge-type detection apparatus as defined in any one of claims 1 to 11, and 44, wherein said storing section comprises a substance which exhibits an ion-exchange reaction.

**60.** (Amended) The cartridge-type detection apparatus as defined in any one of claims 1 to 11, and 44, wherein said storing section comprises a substance which exhibits a specific binding reaction.

**61.** (Amended) A detection cartridge for use in the cartridge-type detection apparatus as defined in claim 56, comprising;
a first sheet and a second sheet both of a resin material and laminated together, said first sheet being formed with at least one electrode-receiving recessed portion;
at least one electrode disposed in said recessed portion;
said second sheet being formed with a passage at a portion corresponding to said electrode for passing the reagent, an insulating sheet disposed between said first sheet and said second sheet, said insulating sheet having at least one aperture of a predetermined area at a position corresponding to said electrode, said storing section being formed at a position away from said electrode, and
a third sheet disposed on either one or both of respective surfaces of said first and second sheets on opposite sides of opposed surfaces thereof, said third sheet being formed with a groove defining a part of said liquid passage in liquid communication with said storing section.

**62.** (Amended) A detection cartridge for use in the cartridge-type detection apparatus as defined in claim 57, wherein said optical cell comprises a single first sheet having a through-aperture, and two transparent second sheets disposed on respective opposite surfaces of said first sheet, said first sheet being formed with said storing section, said second sheet being formed with a groove defining a part of said liquid passage in liquid communication with said storing section.

**63.** (Amended) The detection cartridge as defined in claim 60, wherein a chromatographic column is provided in said liquid passage at a portion connecting said storing section with said electrode.

**64.** (Addition) The detection cartridge as defined in claim 61, wherein a chromatographic column is provided in said liquid passage at a portion connecting said storing section with said optical cell.

# FIG.1

## FIG.2

### (a)

### (b)

### (c)

# FIG.3

# FIG.3(a)

# FIG.3(b)

# FIG.3(c)

# FIG.4

# FIG.4(a)

# FIG.4(b)

# FIG.4(c)

# FIG.5

# FIG.6

## (a)

500

2

## (b)

501

2

## FIG.7

# FIG.8(a)

```
┌─────────────────────────────┐
│      Setting chip in        │
│   sample-injection holder   │
└─────────────────────────────┘
              │
              ▼
┌─────────────────────────────┐
│     Injecting 1 M of NaOH   │
│ (50 μl) from anion supply port │
└─────────────────────────────┘
              │
              ▼
┌─────────────────────────────┐
│    Injecting sample (10 ml) │
│     from anion supply port  │
└─────────────────────────────┘
              │
              ▼
┌─────────────────────────────┐
│    Injecting sample (10 ml) │
│     from cation supply port │
└─────────────────────────────┘
              │
              ▼
```

*To FIG.8(b)*

## FIG.8(b)

*From FIG.8(a)*

Transfer cartridge from holder to reader

↓

Turning on power of reader

↓

Standby state until button is pressed down

↓

Power OFF? —No→ Is measurement started? —No→ ①

Power OFF? ↓ Yes

Turning off power of reader

Is measurement started? ↓

Is cartridge mounted? —No→ Displaying message "please insert cartridge"

↓

Resetting pump

↓

Supplying anion eluent liquid from liquid supply port

↓

Measuring conductivity of anion electrode

↓

Connection OK? —No→ Displaying error message

↓

Starting applying anion measurement potential

↓

Supplying anion eluent liquid from liquid supply port

↓

Keeping potential and flow rate until deposition time elapses

↓

Potential Sweep    Current measurement

②

*To FIG.8(c)*

# FIG.8(c)

*From FIG.8(b)*

Supplying cation eluent liquid from liquid supply port

↓

Measuring conductivity of cation electrode

↓

Connection OK? ──No──→ Displaying error message ──→ (I)

↓ (Yes)

Starting applying cation measurement potential

↓

Supplying cation eluent liquid from liquid supply port

↓

Keeping potential and flow rate until deposition time elapses

↓

Potential Sweep        Current measurement

↓

Calculating analyte concentration from electric signal

↓

Displaying result

↓

Cleaning measurement line

↓

(II)

## FIG.8(d)

cleaning liquid tank ~503

51

eluent liquid (anion)

eluent liquid (cation)

51

52

sample liquid

①activating liquid
②sample liquid

absorptive substrate (cation) ~22

absorptive substrate (anion) ~21

| working electrode 1 | ~35 |
| working electrode 2 | ~36 |
| reference electrode | ~37 |
| counter electrode | ~38 |

| working electrode 1 | ~31 |
| working electrode 2 | ~32 |
| counter electrode | ~33 |
| reference electrode | ~34 |

electrolyte liquid

waste liquid reservoir ~110

sample liquid outlet port ~115

EP 1 852 703 A1

# FIG.9

25ppb As, Se

20ppb As, Se

15ppb As, Se

10ppb As, Se

5ppb As, Se

5 μ A

E (V vs.Ag/AgCl)

-0.50   -0.25   0   0.25   0.50   0.75   1   1.25

EP 1 852 703 A1

FIG.10

EP 1 852 703 A1

# FIG.11

# FIG.12

EP 1 852 703 A1

FIG.13(a)

FIG.13(b)

EP 1 852 703 A1

FIG.13(c)

## FIG.14(a)

# FIG.14(b)

## ( i )

## ( ii )

## ( iii )

FIG.14(c)

# FIG.15(a)

# FIG.15(b)

FIG.16

## FIG.17

EP 1 852 703 A1

FIG.18

# FIG.19

# FIG.20

FIG.21

# FIG.22

# FIG.23

waste liquid tank — 705

711

707

710

Content of tank | cleaning liquid — A'

KCl 1.6M
citric acid10mM | reagent 1 — B'

KCl 1.6mM | reagent 2 — C'

H2SO4 1M | reagent 3 — D'

auxiliary tank | reagent 4 — E'

706

P1

P2

708

716

F''''

discharge2

F''

discharge1

G'

downstream side of
accumulation portion

H'

upstream side of
accumulation portion } 2nd
channel line

I'

reference
electrode

J'

reference
electrode

K'

downstream side of
accumulation portion } 1st
channel line

L'

upstream side of
accumulation portion

Detection Cartridge

1

EP 1 852 703 A1

FIG.24

EP 1 852 703 A1

# FIG.25

705

731

730

705a

# FIG.26

720

721

716

F''

F

F'

711

711

730

731

705b

705a

705

# FIG.27

EP 1 852 703 A1

# FIG.28(a)

| | Step | Display Section | User Operation | Pump | Tank Switching Valve | Destination Switching Valve | Processing Mechanism |
|---|---|---|---|---|---|---|---|
| 1 | power ON | | power ON | | | | |
| 2 | standby state until cartridge setting | Please set reagent tank and insert cartridge | insertion of cartridge operation of OK button | | | | |
| 3 | auto-zero setting for pump | Auto-zero setting | | pulse → ON(clean-up) termination detection (built-in sensor of pump) | | valve F open<br><br>valve F close | |
| 4 | fill-up of electrode chambers of 1st line with electrolyte liquid | | | 200 μl suction 600Hz×2s (6000μl/min) | valve B open<br><br>valve B close | | |
| | | | | 200 μl discharge 600Hz×2s (6000μl/min) | | valve K open<br><br>valve K close | |
| 5 | checkup of electrodes of 1st line | Under electrode checkup | | | | | |
| 6 | actual measurement for 1st line (Cd, Pb, Hg measurement) | Under measurement | | 150μl suction 5Hz×3min (50μl/min) | valve B open<br><br>valve B close | | |
| | | | | 150μl discharge 5Hz×3min (50μl/min) | | valve L open<br><br>valve L close | |
| | | result (example) : No Cd Pb 20 ppm | | | | | •current measurement /record •creation of potential -current curve •calculation of base line •identification of peaks •calculation of peak height / area •calculation of concentration of target substance |

EP 1 852 703 A1

## FIG.28(b)

| | Step | Display Section | User Operation | Pump | Tank Switching Valve | Destination Switching Valve | Processing Mechanism |
|---|---|---|---|---|---|---|---|
| 7 | save of measurement result | display of saving dialog box | designation of saving path/ file name | | | | |
| 8 | cleaning | Under system cleaning | | $200\mu$l suction | valve A open<br><br>valve A close | | |
| | | | | $200\mu$l discharge | | valve L open<br>valve K open<br><br>valve K close<br>valve L close | |
| | | | | $200\mu$l suction | valve A open<br><br>valve A close | | |
| | | | | $200\mu$l discharge | | valve F open<br><br>valve F close | |

EP 1 852 703 A1

# FIG.29(a)

| | Step | Display Section | User Operation | Pump | Tank Switching Valve | Destination Switching Valve | Processing Mechanism |
|---|---|---|---|---|---|---|---|
| 9 | fill-up of electrode chambers of 2nd line with electrolyte liquid | | | 200μl suction | valve D open<br><br>valve D close | | |
| | | | | 200μl discharge | | valve G open<br><br>valve G close | |
| 10 | fill-up of reference electrode chamber of 2nd line with activating liquid | | | 10μl suction | valve C open<br><br>valve C close | | |
| | | | | 10μl discharge | | valve I open<br><br>valve I close | |
| 11 | checkup of electrodes of 2nd line | Under electrode checkup | | | | | |
| 12 | actual measurement of 2nd line (As measurement) | Under measurement | | 250μl suction | valve D open<br><br>valve D close | | · |
| | | | | 250μl discharge | | valve H open<br><br>valve H close | ·current measurement /record |
| | | result (example)<br>: As 30 ppm | | | | | ·creation of potential −current curve ·calculation of base line ·identification of peaks ·calculation of peak height / area ·calculation of concentra −tion of target substance |

EP 1 852 703 A1

## FIG.29(b)

| | Step | Display Section | User Operation | Pump | Tank Switching Valve | Destination Switching Valve | Processing Mechanism |
|---|---|---|---|---|---|---|---|
| 13 | save of measurement result | display of saving dialog box | designation of saving path /file name | | | | |
| 14 | cleaning | Under system cleaning | | $200\mu$l suction 5Hz×2min (50$\mu$l/min) | valve A open<br><br>valve A close | | |
| | | | | $200\mu$l discharge | | valve G open<br>valve I open<br>valve H open<br><br>valve H close<br>valve I close<br>valve G close | |
| | | | | $200\mu$l suction | valve A open<br><br>valve A close | | |
| | | | | $200\mu$l discharge | | valve F open<br><br>valve F close | |
| 15 | | Ready for power OFF | power OFF | | | | |

EP 1 852 703 A1

# FIG.30(a)

| | Step | Display Section | User Operation | Pump | Tank Switching Valve | Destination Switching Valve | Note) Flow in Channel |
|---|---|---|---|---|---|---|---|
| 1 | pretreatment of cartridge | — | cartridge pretreatment using sample injection holder | — | — | — | |
| 2 | sample injection ((①)) | — | injection of given volume of sample using the holder | — | — | — | |
| 3 | power ON | — | power ON | — | — | — | |
| 4 | internal cleaning | Initiation of startup operation | checkup of whether cleaning cartridge is inserted into holder section/operation of OK button | auto-zero | | B, C open  B, C close | pump→B→C (clean-up) |
| 5 | cartridge setting ((②)) | Please setting your cartridge | setting of cartridge in holder/operation of OK button | | | | |
| 6 | setup of cartridge (removal of gas bubbles)((③)) | Under cartridge pretreatment | | suction 100μl | F open  F close | | activating liquid→F→pump |
| | | | | discharge 100μl | | A, C open  A, C close | pump→A→(CG)→C |
| 7 | setup of column((④)) | Under column pretreatment | | suction 150μl | G open  G close | | activating liquid→G→pump |
| | | | | discharge 150μl | | D open  D close | pump→D→(CG)→sensor→··· |

CG: cartridge

EP 1 852 703 A1

# FIG.30(b)

| | Step | Display Section | User Operation | Pump | Tank Switching Valve | Destination Switching Valve | Note) Flow in Channel |
|---|---|---|---|---|---|---|---|
| 8 | supply eluent to column/analysis (⑤) | Under analysis | | suction 250μl | G open / G close | | eluent liquid→G→pump |
| | | | | discharge 50μl | | B,E open / B,E close | pump→B→(CG)→E→(CG)→sensor→… |
| | | | | discharge 200μl | | D open / D close | pump→D→(CG)→sensor→… |
| 9 | display of measurement result (⑥) | measurement result | | | | | |
| 10 | termination of analysis (⑦) | Under system cleaning | | suction 250μl | H open / H close | | cleaning liquid→H→pump |
| | | | | discharge 50μl | | A,C open / A,C close | pump→A→(CG)→C |
| | | | | discharge 50μl | | D open / D close | pump→D→(CG)→sensor→… |
| | | | | discharge 150μl | | B,E open / B,E close | pump→B→(CG)→E→(CG)→sensor→… |

CG: cartridge

EP 1 852 703 A1

FIG.31

# FIG.32

EP 1 852 703 A1

# FIG.33

EP 1 852 703 A1

# FIG.34

(a)                                          (b)

# FIG.35

# FIG.36

810

814b

824

837a

837

840a

840

# FIG.37

To pump 830

F

G

H

F ×

G ×

H ×

F

G

H

848

846

847

849

850

845

FIG.38

```
┌─────────────────────────┐
│ Injecting given volume of │
│   sample into cartridge   │
└─────────────────────────┘
            │
            ▼
┌─────────────────────────┐
│    Setting cartridge     │
│    in analysis unit      │
└─────────────────────────┘
            │
            ▼
┌─────────────────────────┐
│Turning on power of analysis unit│
└─────────────────────────┘
            │
            ▼
┌─────────────────────────┐
│  Pressing down OK button │◄─────────────────────────
└─────────────────────────┘
            │
            ▼
        ╱Power OFF?╲  ──NO──►  ╱Starting╲  ──NO──┐
        ╲         ╱            ╲measurement╱       │
            │YES                    │YES           │
            ▼                        ▼              │
┌─────────────────────────┐    ╱Is cartridge╲ ─NO─►┌─────────────────────┐
│Turning off power of analysis unit│  ╲ loaded? ╱    │Displaying message   │
└─────────────────────────┘         │YES           │"Please insert cartridge"│
                                     ▼              └─────────────────────┘
                         ┌─────────────────────────┐
                         │ Auto-zero operation for pump │
                         └─────────────────────────┘
                                     │
                                     ▼
                         ┌─────────────────────────┐
                         │  Pre-treatment of cartridge  │
                         │  (Filling channel including  │
                         │ filter with activating liquid)│
                         └─────────────────────────┘
                                     │
                                     ▼
                         ┌─────────────────────────┐
                         │  Pre-treatment of column    │
                         │  (Filling channel including  │
                         │ column with activating liquid)│
                         └─────────────────────────┘
                                     │
                                     ▼
                              ╱Checking╲  ──NO──► ┌─────────────────────┐
                              ╲light axis╱         │Displaying error message│
                                  │YES             │"Please insert cartridge again"│
                                  ▼                └─────────────────────┘
                         ┌─────────────────────────┐
                         │   Turning on light source   │
                         └─────────────────────────┘
                                     │
                                     ▼
                         ┌─────────────────────────┐
                         │Starting supplying eluent liquid│
                         └─────────────────────────┘
                                     │
                                     ▼
                         ┌─────────────────────────┐
                         │Starting absorbance measurement│
                         └─────────────────────────┘
                                     │
                                     ▼
                         ┌─────────────────────────┐
                         │Terminating supply of eluent liquid│
                         └─────────────────────────┘
                                     │
                                     ▼
                         ┌─────────────────────────┐
                         │Terminating absorbance measurement│
                         └─────────────────────────┘
                                     │
                                     ▼
                         ┌─────────────────────────┐
                         │   Turning off light source  │
                         └─────────────────────────┘
                                     │
                                     ▼
                         ┌─────────────────────────┐
                         │ Identifying peaks from time- │
                         │absorbance plats.Calculating │
                         │specific analyte/concentration│
                         └─────────────────────────┘
                                     │
                                     ▼
                         ┌─────────────────────────┐
                         │      Displaying result      │
                         └─────────────────────────┘
                                     │
                                     ▼
                         ┌─────────────────────────┐
                         │  Cleaning measurement line  │
                         └─────────────────────────┘
```

# FIG.39

| | Step | Display Section | User Operation | Pump | Tank Switching Valve | Destination Switching Valve | Note) Flow in Channel [ ]: cartridge |
|---|---|---|---|---|---|---|---|
| 1 | injection of sample (①) | — | injection of given volume of sample using holder (before setting cartridge in analyzer) | — | — | — | injection port→filter→ (trapping analyte)→ discharge out of cartridge |
| 2 | setting of cartridge (②) | | setting of cartridge in holder operation of OK button | | | | |
| 3 | power ON | — | power ON | — | — | — | |
| 4 | internal cleaning | Initiation of startup operation | checkup of whether cleaning cartridge is inserted into holder section/operation of OK button | auto-zero | | B,C open / B,C close | pump→B→C (clean-up) |
| 5 | setup of cartridge (removal of gas bubbles) (③) | Under cartridge pretreatment | | suction 100 μl | F open / F close | | activating liquid →F→pump |
| | | | | discharge 100 μl | | A,C open / A,C close | pump→A→ [filter]→ waste liquid tank |
| 6 | setup of column (④) | Under column pretreatment | | suction 150 μl | G open / G close | | eluent liquid →G→pump |
| | | | | discharge 150 μl | | D open / D close | pump→D→ [column] |
| 7 | supplying eluent to column/analysis (⑤) | Under analysis | | suction 250 μl | G open / G close | | eluent liquid →G→pump |
| | | | | discharge 250 μl | | B,E open / B,E close | Pump→B→ [filter (eluting trapped analyte)]→E→ [column]→waste liquid tank |
| 8 | display of detection result (⑥) | display of detection result | | | | | |
| 9 | termination of analysis (⑦) | Under system cleaning | | suction 250 μl | H open / H close | | cleaning liquid tank →H→pump |
| | | | | discharge 50 μl | | A,C open / A,C close | pump→A→ [filter] →C→waste liquid tank |
| | | | | discharge 50 μl | | D open / D close | pump→D→ [column] →waste liquid tank |
| | | | | discharge 150 μl | | B,E open / B,E close | pump→B→ [filter]→E→ [column]→waste liquid tank |

EP 1 852 703 A1

# FIG.40

oxalic acid

succinic acid

3

12

Hold Time (min)

# FIG.41

(a) Competitive Immunoassay

I — sample antigen, labeled antibody (reagent), preliminary reaction
II — injection, waste liquid, accumulation element, immobilizing antigen, only unreacted labeled antibody is captured
III — supply of substrate, substrate, reaction product, detection at subsequent zone

(b) Competitive Immunoassay

I — sample antigen, labeled antibody (reagent), preliminary reaction
II — injection, waste liquid, accumulation element, reacted labeled antibody is captured
III — supply of substrate, substrate, reaction product, detection at subsequent zone

(c) Non-Competitive Immunoassay

I — sample antigen, accumulation element
II — immobilizing antibody, waste liquid, labeled antibody, waste liquid
III — supply of substrate, substrate, reaction product, detection at subsequent zone

(Note): ● antigen    Y antibody    ⊘ label

EP 1 852 703 A1

# FIG.42

# FIG.43

EP 1 852 703 A1

# FIG.44

| | Step | Display Section | User Operation | Pump | Tank Switching Valve | Destination Switching Valve | Processing Mechanism |
|---|---|---|---|---|---|---|---|
| 1 | power ON | | power ON | | | | |
| 2 | standby state until cartridge setting | Please set reagent tank and insert cartridge | insertion of cartridge operation of OK button | | | | |
| 3 | auto-zero setting for pump | Auto-zero setting | | pulse→ON(clean-up) termination detection (built-in sensor of pump) | | valve F open valve F close | |
| 3 | actual measurement for 1st line (Cd, Pb, Hg measurement) | Under measurement | | 1ml suction | valve E open valve E close | | |
| | | | | 1ml discharge | | valve L open valve L close | |
| | | result (example) : No Cd Pb 20 ppm | | | | | ·current measurement/record ·creation of potential- current curve ·calculation of base line ·identification of peaks ·calculation of peak height/ area ·calculation of concentration of target substance |
| 4 | save of measurement result | display of saving dialog box | designation of saving path/file name | | | | |
| 5 | cleaning | Under system cleaning | | 200 $\mu$l suction | valve A open valve A close | | |
| | | | | 200 $\mu$l discharge | | valve L open valve K open valve K close valve L close | |
| | | | | 200 $\mu$l suction | valve A open valve A close | | |
| | | | | 200 $\mu$l discharge | | valve F open valve F close | |

EP 1 852 703 A1

# FIG.45

| | Step | Display Section | User Operation | Pump | Tank Switching Valve | Destination Switching Valve | Note) Flow in Channel [ ]: cartridge |
|---|---|---|---|---|---|---|---|
| 1 | injection of sample(①) | – | injection of given volume of sample (before setting cartridge in analyzer) | – | – | – | injection port→filter (trapping analyte) →discharge out of cartridge |
| 2 | setting of cartridge (②) | | setting of cartridge in holder operation of OK button | | | | |
| 3 | power ON | – | power ON | – | – | – | |
| 4 | internal cleaning | Initiation of startup operation | checkup of whether cleaning cartridge is inserted into holder section/operation of OK button | auto-zero | | B, C open / B, C close | pump→B→C (clean-up) |
| 5 | setup of cartridge supply of cleaning buffer | Under cartridge pretreatment | | suction 2ml | F open / F close | | cleaning buffer→F→pump |
| | | | | discharge 2ml | | A, C open / A, C close | pump→A→[filter]→waste liquid tank |
| 6 | supply of eluent buffer | Under analysis | | suction 0.2ml | G open / G close | | eluent buffer→G→pump |
| | | | | discharge 0.2ml | | B, E open / B, E close | pump→B→[filter (elution of tapped analyte)]→E→cell |
| 7 | supply of color former | Under analysis | | suction 0.2ml | G open / G close | | eluent buffer→G→pump |
| | | | | discharge 0.2ml | | D open / D close | pump→D→[cell] |
| 8 | analysis of absorbance | Under analysis | | | | | |
| 9 | display of detection result(⑥) | display of detection result | | | | | |
| 10 | termination of analysis(⑦) | Under system cleaning | | suction 250 μl | H open / H close | | cleaning liquid tank→H→pump |
| | | | | discharge 50 μl | | A, C open / A, C close | pump→A→[filter]→C→ waste liquid tank |
| | | | | discharge 50 μl | | D open / D close | pump→D→[cell]→waste liquid tank |
| | | | | discharge 150 μl | | B, E open / B, E close | pump→B→[filter]→E→[cell] →waste liquid tank |

EP 1 852 703 A1

# FIG.46

1022
1016
1013
1000
1018
1019
1020
1021
1015b
1017
1012
1014
1014a
1014a
1014a
1016
1011
R
S
S
S
1015a

## INTERNATIONAL SEARCH REPORT

| International application No. |
| --- |
| PCT/JP2006/300136 |

A. CLASSIFICATION OF SUBJECT MATTER
*G01N35/08*(2006.01), *G01N27/49*(2006.01), *G01N33/24*(2006.01), *G01N37/00* (2006.01)

According to International Patent Classification (IPC) or to both national classification and IPC

B. FIELDS SEARCHED

Minimum documentation searched (classification system followed by classification symbols)
*G01N35/00-35/10*(2006.01), *G01N27/49*(2006.01), *G01N33/24*(2006.01), *G01N37/00* (2006.01)

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| Y | WO 2003/029804 A1 (Arkray, Inc.),<br>10 April, 2003 (10.04.03),<br>Full text; all drawings<br>& US 2004/0244151 A1 & EP 1431758 A1 | 13,25,26,30,<br>35,36 |
| Y | WO 2004/039500 A1 (HEWLETT-PACKARD DEVELOPMENT CO., L.P.),<br>13 May, 2004 (13.05.04),<br>Full text; all drawings<br>& US 2004/0086872 A1 & EP 1567267 A1<br>& JP 2006-504957 A | 13,25,26,35,<br>36 |
| Y | JP 2005-3637 A (Yokogawa Electric Corp.),<br>06 January, 2005 (06.01.05),<br>Full text; all drawings<br>& US 2004/0253143 A1 | 30 |

| ☒ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>09 May, 2006 (09.05.06) | Date of mailing of the international search report<br>16 May, 2006 (16.05.06) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/300136

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-125777 A (Toshiba Corp.), 22 April, 2004 (22.04.04), Full text; all drawings & WO 2004/011925 A1 & EP 1542000 A1 & US 2005/0158787 A1 | 1-63 |
| A | WO 2003/104772 A1 (CHEMPAQ A/S), 18 December, 2003 (18.12.03), Full text; all drawings & EP 1552276 A1 & US 2006/013725 A1 & JP 2005-534896 A | 1-63 |
| A | WO 2003/040413 A1 (INTEGRATED NANO-TECHNOLOGIES, L.L.C.), 15 May, 2003 (15.05.03), Full text; all drawings & US 2003/0109031 A1 & EP 1451373 A1 & JP 2005-508198 A | 1-63 |
| A | JP 2004-93434 A (Horiba, Ltd.), 25 March, 2004 (25.03.04), Full text; all drawings (Family: none) | 1-63 |

Form PCT/ISA/210 (continuation of second sheet) (April 2005)

## INTERNATIONAL SEARCH REPORT

International application No.

PCT/JP2006/300136

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

   The inventions in Claims 1 to 12 and 39 to 63 relate to the cartridge type detection device comprising the plurality of switchingly connected liquid flow passages.
   The inventions in Claims 13 to 28, 36, and 37 relate to the cartridge type concentration detection device having the concentration part for concentrating the detected substances.
   The invention in Claim 29 relates to the portable cartridge type concentration detection device comprising the plurality of acting electrode elements with different areas.
(continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the    payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.

PCT/JP2006/300136

Continuation of Box No.III of continuation of first sheet(2)

    The invention in Claim 30 relates to the portable cartridge type concentration detection device having the waste sump.
    The inventions in Claims 31 to 35 and 38 relate to the cartridge type concentration detection device formed of three sheets.

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10311829 A
- US 6110354 A
- JP 2000155122 A
- JP 2003987171 A

**Non-patent literature cited in the description**

- **MATSUURA et al.** *Analytical Chemistry,* 2005, vol. 77 (13), 4235-4240